(19) Europäisches Patentamt
European Patent Office
Office européen des brevets

(11) EP 3 986 902 B1

(12) EUROPEAN PATENT SPECIFICATION

(45) Date of publication and mention of the grant of the patent:
**24.01.2024 Bulletin 2024/04**

(21) Application number: **20734336.9**

(22) Date of filing: **18.06.2020**

(51) International Patent Classification (IPC):
***C07D 515/22*** (2006.01) ***A61P 35/00*** (2006.01)
***A61K 31/395*** (2006.01)

(52) Cooperative Patent Classification (CPC):
**C07D 515/22; A61P 35/00**

(86) International application number:
**PCT/EP2020/066890**

(87) International publication number:
**WO 2020/254471 (24.12.2020 Gazette 2020/52)**

# (54) MACROCYCLIC INHIBITORS OF MCL-1

MAKROZYCLISCHE INHIBITOREN OF MCL-1

INHIBITEURS MACROCYCLIQUES DE MCL-1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.06.2019 EP 19181605**

(43) Date of publication of application:
**27.04.2022 Bulletin 2022/17**

(73) Proprietor: **Janssen Pharmaceutica NV**
**2340 Beerse (BE)**

(72) Inventors:
- **ROMBOUTS, Frederik, Jan, Rita**
  **2340 Beerse (BE)**
- **PESCHIULLI, Aldo**
  **2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2017/182625 WO-A1-2018/178226**
**WO-A1-2019/035927**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 986 902 B1

## Description

FIELD OF THE INVENTION

The present invention relates to pharmaceutical agents useful for therapy and/or prophylaxis in a subject, pharmaceutical composition comprising such compounds, and their use as MCL-1 inhibitors, useful for treating or preventing diseases such as cancer.

BACKGROUND OF THE INVENTION

Cellular apoptosis or programmed cell death is critical to the development and homeostasis of many organs including the hematopoietic system. Apoptosis can be initiated via the extrinsic pathway, which is mediated by death receptors, or by the intrinsic pathway using the B cell lymphoma (BCL-2) family of proteins. Myeloid cell leukemia-1 (MCL-1) is a member of the BCL-2 family of cell survival regulators and is a critical mediator of the intrinsic apoptosis pathway. MCL-1 is one of five principal anti-apoptotic BCL-2 proteins (MCL-1, BCL-2, BCL-XL, BCL-w, and BFL1/A1) responsible for maintaining cell survival. MCL-1 continuously and directly represses the activity of the pro-apoptotic BCL-2 family proteins Bak and Bax and indirectly blocks apoptosis by sequestering BH3 only apoptotic sensitizer proteins such as Bim and Noxa. The activation of Bak/Bax following various types of cellular stress leads to aggregation on the mitochondrial outer membrane and this aggregation facilitates pore formation, loss of mitochondrial outer membrane potential, and subsequent release of cytochrome C into the cytosol. Cytosolic cytochrome C binds Apaf-1 and initiates recruitment of procaspase 9 to form apoptosome structures (Cheng et al. eLife 2016; 5: e17755). The assembly of apoptosomes activates the executioner cysteine proteases 3/7 and these effector caspases then cleave a variety of cytoplasmic and nuclear proteins to induce cell death (Julian et al. Cell Death and Differentiation 2017; 24, 1380-1389).

Avoiding apoptosis is an established hallmark of cancer development and facilitates the survival of tumor cells that would otherwise be eliminated due to oncogenic stresses, growth factor deprivation, or DNA damage (Hanahan and Weinberg. Cell 2011;1-44). Thus, unsurprisingly, MCL-1 is highly upregulated in many solid and hematologic cancers relative to normal non-transformed tissue counterparts. The overexpression of MCL-1 has been implicated in the pathogenesis of several cancers where it correlated with poor outcome, relapse, and aggressive disease. Additionally, overexpression of MCL-1 has been implicated in the pathogenesis of the following cancers: prostate, lung, pancreatic, breast, ovarian, cervical, melanoma, B-cell chronic lymphocytic leukemia (CLL), acute myeloid leukemia (AML), and acute lymphoblastic leukemia (ALL). The human MCL-1 genetic locus (1q21) is frequently amplified in tumors and quantitatively increases total MCL-1 protein levels (Beroukhim et al. Nature 2010;463 (7283) 899-905). MCL-1 also mediates resistance to conventional cancer therapeutics and is transcriptionally upregulated in response to inhibition of BCL-2 function (Yecies et al. Blood 2010;115 (16)3304-3313).

A small molecule BH3 inhibitor of BCL-2 has demonstrated clinical efficacy in patients with chronic lymphocytic leukemia and is FDA approved for patients with CLL or AML (Roberts et al. NEJM 2016;374:311-322). The clinical success of BCL-2 antagonism led to the development of several MCL-1 BH3 mimetics that show efficacy in preclinical models of both hematologic malignancies and solid tumors (Kotschy et al. Nature 2016;538 477-486, Merino et al. Sci. Transl. Med;2017 (9)).

MCL-1 regulates several cellular processes in addition to its canonical role in mediating cell survival including mitochondrial integrity and non-homologous end joining following DNA damage (Chen et al. JCI 2018;128(1):500-516). The genetic loss of MCL-1 shows a range of phenotypes depending on the developmental timing and tissue deletion. MCL-1 knockout models reveal there are multiple roles for MCL-1 and loss of function impacts a wide range of phenotypes. Global MCL-1-deficient mice display embryonic lethality and studies using conditional genetic deletion have reported mitochondrial dysfunction, impaired activation of autophagy, reductions in B and T lymphocytes, increased B and T cell apoptosis, and the development of heart failure/ cardiomyopathy (Wang et al. Genes and Dev 2013;27 1351-1364, Steimer et al. Blood 2009;(113) 2805-2815).

WO2018178226 discloses MCL-1 inhibitors and methods of use thereof.

WO2017182625 discloses macrocyclic MCL-1 inhibitors for treating cancer.

WO2018178227 discloses the synthesis of MCL-1 inhibitors.

WO2020063792 discloses indole macrocyclic derivatives.

CN110845520 and WO2020103864 disclose macrocyclic indoles as MCL-1 inhibitors.

CN20191114551 discloses MCL-1 inhibitors.

There remains a need for MCL-1 inhibitors, useful for the treatment or prevention of cancers such as prostate, lung, pancreatic, breast, ovarian, cervical, melanoma, B-cell chronic lymphocytic leukemia (CLL), acute myeloid leukemia (AML), and acute lymphoblastic leukemia (ALL).

SUMMARY OF THE INVENTION

The present invention concerns novel compounds of Formula (I):

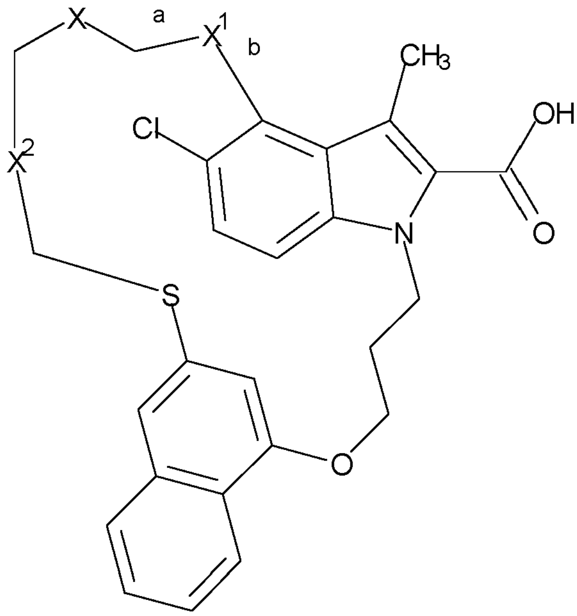

(I)

and the tautomers and the stereoisomeric forms thereof, wherein
$X^1$ represents

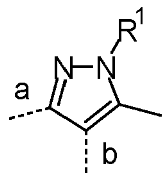

or

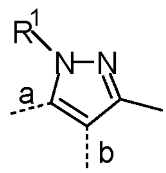

,

wherein 'a' and 'b' indicate how variable $X^1$ is attached to the remainder of the molecule;
$R^1$ represents hydrogen or $C_{1-6}$alkyl;
$X^2$ represents

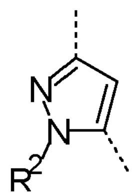

which can be attached to the remainder of the molecule in both directions;
$R^2$ represents hydrogen or $C_{1-6}$alkyl;
X represents -S- or -N($R^x$)-;
$R^x$ represents hydrogen, methyl, $C_{2-6}$alkyl, -C(=O)-$C_{1-6}$alkyl, -S(=O)$_2$-$C_{1-6}$alkyl, $C_{3-6}$cycloalkyl, -C(=O)-$C_{3-6}$cycloalkyl, or -S(=O)$_2$-$C_{3-6}$cycloalkyl; wherein $C_{2-6}$alkyl, -C(=O)-$C_{1-6}$alkyl, -S(=O)$_2$-$C_{1-6}$alkyl, $C_{3-6}$cycloalkyl, -C(=O)-$C_{3-6}$cycloalkyl, and -S(=O)$_2$-$C_{3-6}$cycloalkyl are optionally substituted with one, two or three substituents selected from the group consisting of halo, $C_{1-4}$alkyl and $C_{1-4}$alkyl substituted with one, two or three halo atoms;
and the pharmaceutically acceptable salts and the solvates thereof.

The present invention also relates to a pharmaceutical composition comprising a therapeutically effective amount of a compound of Formula (I), a pharmaceutically acceptable salt, or a solvate thereof, and a pharmaceutically acceptable carrier or excipient.

Additionally, the invention relates to a compound of Formula (I), a pharmaceutically acceptable salt, or a solvate thereof, for use as a medicament, and to a compound of Formula (I), a pharmaceutically acceptable salt, or a solvate thereof, for use in the treatment or in the prevention of cancer.

In a particular embodiment, the invention relates to a compound of Formula (I), a pharmaceutically acceptable salt, or a solvate thereof, for use in the treatment or in the prevention of cancer.

The invention also relates to the use of a compound of Formula (I), a pharmaceutically acceptable salt, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable salt, or a solvate thereof.

The invention also relates to a product comprising a compound of Formula (I), a pharmaceutically acceptable salt, or a solvate thereof, and an additional pharmaceutical agent, as a combined preparation for simultaneous, separate or sequential use in the treatment or prevention of cancer.

Additionally, the invention relates to a compound of Formula (I), a pharmaceutically acceptable salt, or a solvate thereof, as defined herein, or a pharmaceutical composition or combination as defined herein, for use in treating or preventing a cell proliferative disease.

DETAILED DESCRIPTION OF THE INVENTION

The term 'halo' or 'halogen' as used herein represents fluoro, chloro, bromo and iodo.

The prefix '$C_{x-y}$' (where x and y are integers) as used herein refers to the number of carbon atoms in a given group. Thus, a $C_{1-6}$alkyl group contains from 1 to 6 carbon atoms, and so on.

The term '$C_{1-4}$alkyl' as used herein as a group or part of a group represents a straight or branched chain fully saturated hydrocarbon radical having from 1 to 4 carbon atoms, such as methyl, ethyl, n-propyl, isopropyl, n-butyl, s-butyl, t-butyl and the like.

The term '$C_{1-6}$alkyl' as used herein as a group or part of a group represents a straight or branched chain fully saturated hydrocarbon radical having from 1 to 6 carbon atoms, such as methyl, ethyl, *n*-propyl, isopropyl, *n*-butyl, *s*-butyl, *t*-butyl, *n*-pentyl, *n*-hexyl and the like.

The term '$C_{2-6}$alkyl' as used herein as a group or part of a group represents a straight or branched chain fully saturated hydrocarbon radical having from 2 to 6 carbon atoms, such as ethyl, *n*-propyl, isopropyl, *n*-butyl, *s*-butyl, *t*-butyl, *n*-pentyl, *n*-hexyl and the like.

The term '$C_{3-6}$cycloalkyl' as used herein as a group or part of a group defines a fully saturated, cyclic hydrocarbon radical having from 3 to 6 carbon atoms, such as cyclopropyl, cyclobutyl, cyclopentyl and cyclohexyl.

It will be clear for the skilled person that $S(=O)_2$ or $SO_2$ represents a sulfonyl moiety.

It will be clear for the skilled person that CO or C(=O) represents a carbonyl moiety.

In general, whenever the term 'substituted' is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture.

Combinations of substituents and/or variables are permissible only if such combinations result in chemically stable compounds. 'Stable compound' is meant to indicate a compound that is sufficiently robust to survive isolation to a useful degree of purity from a reaction mixture.

The skilled person will understand that the term 'optionally substituted' means that the atom or radical indicated in the expression using 'optionally substituted' may or may not be substituted (this means substituted or unsubstituted respectively).

When two or more substituents are present on a moiety they may, where possible and unless otherwise indicated or clear from the context, replace hydrogens on the same atom or they may replace hydrogen atoms on different atoms in the moiety.

It will be clear for the skilled person that, unless otherwise is indicated or is clear from the context, a substituent on a heterocyclyl group may replace any hydrogen atom on a ring carbon atom or on a ring heteroatom (e.g. a hydrogen on a nitrogen atom may be replaced by a substituent).

It will be clear that a Compound of Formula (I) includes Compounds of Formula (I-a) and (I-b) (both directions of $X^2$ being

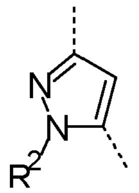

)

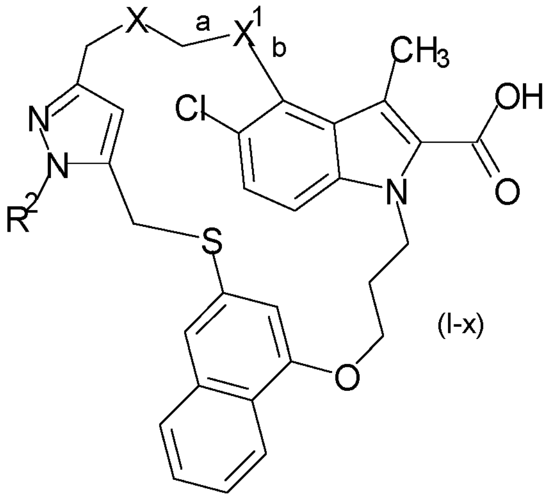

(I-x)

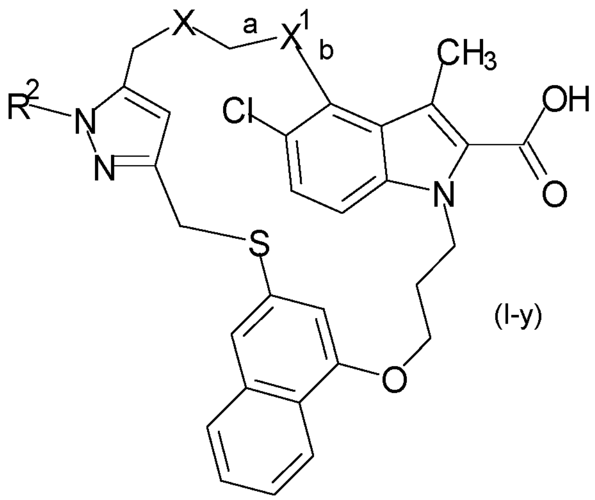

(I-y)

When any variable occurs more than one time in any constituent, each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, or subject (e.g., human) that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R*, *S*) around one or more atoms, contemplates each possible stereoisomer, or mixture of two or more stereoisomers.

Hereinbefore and hereinafter, the term "compound(s) of Formula (I)" is meant to include the tautomers thereof and the stereoisomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present invention.

In particular, the compounds disclosed herein possess axial chirality, by virtue of restricted rotation around a biaryl bond and as such may exist as mixtures of atropisomers. When a compound is a pure atropisomer, the stereochemistry at each chiral center may be specified by either $R_a$ or $S_a$. Such designations may also be used for mixtures

that are enriched in one atropisomer. Further description of atropisomerism and axial chirality and rules for assignment of configuration can be found in Eliel, E.L. & Wilen, S. H. 'Stereochemistry of Organic Compounds' John Wiley and Sons, Inc. 1994.

Diastereomers (or diastereoisomers) are stereoisomers that are not enantiomers, i.e. they are not related as mirror images. If a compound contains a double bond, the substituents may be in the E or the Z configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, atropisomers, diastereomers, racemates, E isomers, Z isomers, cis isomers, trans isomers and mixtures thereof, whenever chemically possible.

The meaning of all those terms, i.e. enantiomers, atropisomers, diastereomers, racemates, E isomers, Z isomers, cis isomers, trans isomers and mixtures thereof are known to the skilled person.

The absolute configuration is specified according to the Cahn-Ingold-Prelog system. The configuration at an asymmetric atom is specified by either R or S. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light. Optically active ($R_a$)- and ($S_a$)-atropisomers may be prepared using chiral synthons, chiral reagents or chiral catalysts, or resolved using conventional techniques well known in the art, such as chiral HPLC.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (*R*), this means that the compound is substantially free of the (*S*) isomer; when a compound of Formula (I) is for instance specified as *E*, this means that the compound is substantially free of the *Z* isomer; when a compound of Formula (I) is for instance specified as cis, this means that the compound is substantially free of the trans isomer; when a compound of Formula (I) is for instance specified as $R_a$, this means that the compound is substantially free of the $S_a$ atropisomer.

Pharmaceutically acceptable salts, in particular pharmaceutically acceptable additions salts, include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I), and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, N-oxides and solvates thereof, involves liquid chromatography using a chiral

stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$ , $^{13}N$, $^{15}O$, $^{17}O$, $^{18}O$, $^{32}P$, $^{33}P$, $^{35}S$, $^{18}F$, $^{36}Cl$, $^{122}I$, $^{123}I$, $^{125}I$, $^{131}I$, $^{75}Br$, $^{76}Br$, $^{77}Br$ and $^{82}Br$. Preferably, the radioactive isotope is selected from the group of $^{2}H$, $^{3}H$, $^{11}C$ and $^{18}F$. More preferably, the radioactive isotope is $^{2}H$. In particular, deuterated compounds are intended to be included within the scope of the present invention.

Certain isotopically-labeled compounds of the present invention (e.g., those labeled with $^{3}H$ and $^{14}C$) may be useful for example in substrate tissue distribution assays. Tritiated ($^{3}H$) and carbon-14 ($^{14}C$) isotopes are useful for their ease of preparation and detectability. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

The present invention relates in particular to compounds of Formula (I) as defined herein, and the tautomers and the stereoisomeric forms thereof, wherein

$X^1$ represents

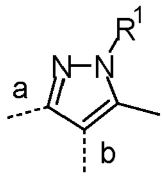
or
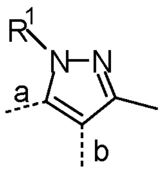
,

wherein 'a' and 'b' indicate how variable $X^1$ is attached to the remainder of the molecule;

$R^1$ represents hydrogen or $C_{1-6}$alkyl;

$X^2$ represents

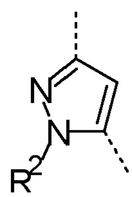

which can be attached to the remainder of the molecule in both directions;

$R^2$ represents hydrogen or $C_{1-6}$alkyl;

X represents -S- or $-N(R^x)-$;

$R^x$ represents hydrogen, methyl, $C_{2-6}$alkyl, $-C(=O)-C_{1-6}$alkyl, $-S(=O)_2-C_{1-6}$alkyl, $C_{3-6}$cycloalkyl, $-C(=O)-C_{3-6}$cycloalkyl, or $-S(=O)_2-C_{3-6}$cycloalkyl; wherein $C_{2-6}$alkyl, $-C(=O)-C_{1-6}$alkyl, $-S(=O)_2-C_{1-6}$alkyl, $C_{3-6}$cycloalkyl, $-C(=O)-C_{3-6}$cycloalkyl, and $-S(=O)_2-C_{3-6}$cycloalkyl are optionally substituted with one, two or three substituents selected from the group consisting of halo, $C_{1-4}$alkyl and $C_{1-4}$alkyl substituted with one, two or three halo atoms;

and the pharmaceutically acceptable salts and the solvates thereof; provided that

| | | |
|---|---|---|
| 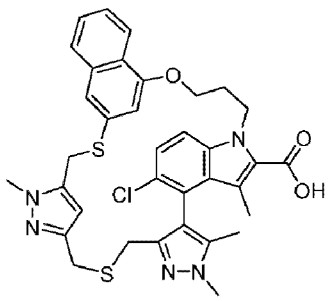 | 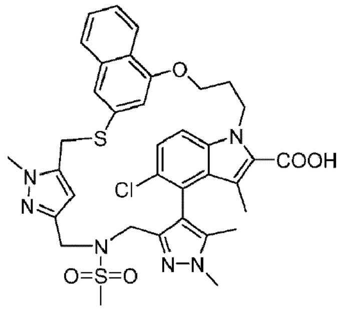 | 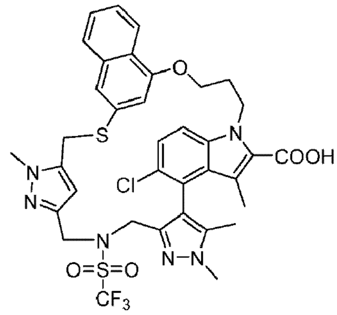 |
| 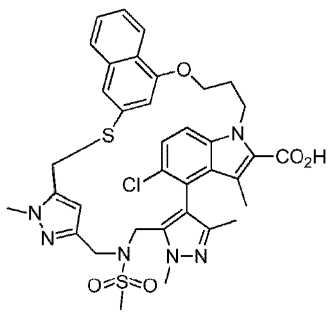 | 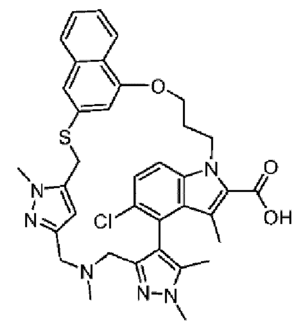 | |

and the tautomers and the stereoisomeric forms thereof are excluded.

The present invention relates in particular to compounds of Formula (I) as defined herein, and the tautomers and the stereoisomeric forms thereof, wherein $X^1$ represents

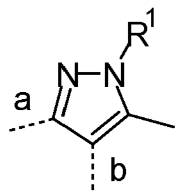

,

wherein 'a' and 'b' indicate how variable $X^1$ is attached to the remainder of the molecule;

$R^1$ represents hydrogen or $C_{1-6}$alkyl;

$X^2$ represents

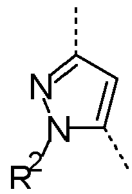

which can be attached to the remainder of the molecule in both directions;

$R^2$ represents hydrogen or $C_{1-6}$alkyl;

X represents -S- or -N($R^x$)-;

$R^x$ represents hydrogen, methyl or $C_{2-6}$alkyl;

and the pharmaceutically acceptable salts and the solvates thereof.

The present invention relates in particular to compounds of Formula (I) as defined herein, and the tautomers and the stereoisomeric forms thereof, wherein

$X^1$ represents

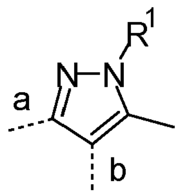

,

wherein ’a’ and ’b’ indicate how variable $X^1$ is attached to the remainder of the molecule;

$R^1$ represents $C_{1-6}$alkyl;

$X^2$ represents

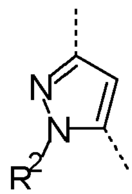

which can be attached to the remainder of the molecule in both directions;

$R^2$ represents $C_{1-6}$alkyl;

X represents -S- or -N($R^x$)-;

$R^x$ represents hydrogen or methyl;

and the pharmaceutically acceptable salts and the solvates thereof.

The present invention relates in particular to compounds of Formula (I) as defined herein, and the tautomers and the stereoisomeric forms thereof, wherein

$X^1$ represents

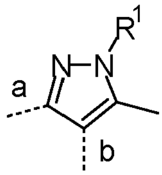

,

wherein 'a' and 'b' indicate how variable $X^1$ is attached to the remainder of the molecule;
$R^1$ represents methyl;
$X^2$ represents

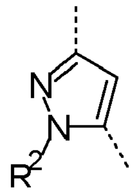

which can be attached to the remainder of the molecule in both directions;
$R^2$ represents methyl;
X represents -S- or -N($R^x$)-;
$R^x$ represents hydrogen or methyl;
and the pharmaceutically acceptable salts and the solvates thereof.

In an embodiment, the present invention relates to those compounds of Formula (I) and the pharmaceutically acceptable salts, and the solvates thereof, or any subgroup thereof as mentioned in any of the other embodiments, wherein X represent-N($R^x$)-.

In an embodiment, the present invention relates to those compounds of Formula (I) and the pharmaceutically acceptable salts, and the solvates thereof, or any subgroup thereof as mentioned in any of the other embodiments, wherein X represent-N($R^x$)-; and $R^x$ represents hydrogen.

In an embodiment, the present invention relates to those compounds of Formula (I) and the pharmaceutically acceptable salts, and the solvates thereof, or any subgroup thereof as mentioned in any of the other embodiments, wherein X represent-N($R^x$)-; and $R^x$ represents methyl.

In an embodiment, the present invention relates to those compounds of Formula (I) and the pharmaceutically acceptable salts, and the solvates thereof, or any subgroup thereof as mentioned in any of the other embodiments, wherein $X^1$ represents

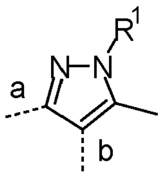

.

In an embodiment, the present invention relates to those compounds of Formula (I) and the pharmaceutically acceptable salts, and the solvates thereof, or any subgroup thereof as mentioned in any of the other embodiments, wherein $X^1$ represents

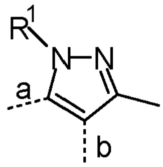

.

In an embodiment, the present invention relates to those compounds of Formula (I) and the pharmaceutically acceptable salts, and the solvates thereof, or any subgroup thereof as mentioned in any of the other embodiments, wherein $R^1$ represents hydrogen.

In an embodiment, the present invention relates to those compounds of Formula (I) and the pharmaceutically acceptable salts, and the solvates thereof, or any subgroup thereof as mentioned in any of the other embodiments, wherein $R^1$ represents $C_{1-6}$alkyl.

In an embodiment, the present invention relates to those compounds of Formula (I) and the pharmaceutically acceptable salts, and the solvates thereof, or any subgroup thereof as mentioned in any of the other embodiments, wherein $R^1$ represents $C_{2-6}$alkyl.

In an embodiment, the present invention relates to those compounds of Formula (I) and the pharmaceutically acceptable salts, and the solvates thereof, or any subgroup thereof as mentioned in any of the other embodiments, wherein $R^1$ represents methyl.

In an embodiment, the present invention relates to those compounds of Formula (I) and the pharmaceutically acceptable salts, and the solvates thereof, or any subgroup thereof as mentioned in any of the other embodiments, wherein $R^2$ represents hydrogen.

In an embodiment, the present invention relates to those compounds of Formula (I) and the pharmaceutically acceptable salts, and the solvates thereof, or any subgroup thereof as mentioned in any of the other embodiments, wherein at least one of $R^1$ and $R^2$ represents hydrogen or $C_{2-6}$alkyl.

In an embodiment, the present invention relates to those compounds of Formula (I) and the pharmaceutically acceptable salts, and the solvates thereof, or any subgroup thereof as mentioned in any of the other embodiments, wherein at least one of $R^1$ and $R^2$ is other than methyl.

In an embodiment, the present invention relates to those compounds of Formula (I) and the pharmaceutically acceptable salts, and the solvates thereof, or any subgroup thereof as mentioned in any of the other embodiments, wherein $R^2$ represents $C_{1-6}$alkyl.

In an embodiment, the present invention relates to those compounds of Formula (I) and the pharmaceutically acceptable salts, and the solvates thereof, or any subgroup thereof as mentioned in any of the other embodiments, wherein $R^2$ represents $C_{2-6}$alkyl.

In an embodiment, the present invention relates to those compounds of Formula (I) and the pharmaceutically acceptable salts, and the solvates thereof, or any subgroup thereof as mentioned in any of the other embodiments, wherein $R^2$ represents hydrogen or $C_{2-6}$alkyl.In an embodiment, the present invention relates to those compounds of Formula (I) and the pharmaceutically acceptable salts, and the solvates thereof, or any subgroup thereof as mentioned in any of the other embodiments, wherein $R^2$ represents methyl.

In an embodiment, the present invention relates to those compounds of Formula (I) and the pharmaceutically acceptable salts, and the solvates thereof, or any subgroup thereof as mentioned in any of the other embodiments, wherein

i) at least one of $R^1$ and $R^2$ is other than methyl; or
ii) $R^x$ is other than methyl or $-S(=O)_2-C_{1-6}$alkyl.

In an embodiment, the present invention relates to those compounds of Formula (I) and the pharmaceutically acceptable salts, and the solvates thereof, or any subgroup thereof as mentioned in any of the other embodiments, wherein

$R^x$ represents hydrogen, $C_{2-6}$alkyl, $-C(=O)-C_{1-6}$alkyl, $C_{3-6}$cycloalkyl, $-C(=O)-C_{3-6}$cycloalkyl, or $-S(=O)_2-C_{3-6}$cycloalkyl; wherein $C_{2-6}$alkyl, $-C(=O)-C_{1-6}$alkyl, $C_{3-6}$cycloalkyl, $-C(=O)-C_{3-6}$cycloalkyl, and $-S(=O)_2-C_{3-6}$cycloalkyl are optionally substituted with one, two or three substituents selected from the group consisting of halo, $C_{1-4}$alkyl and $C_{1-4}$alkyl substituted with one, two or three halo atoms.

In an embodiment, the present invention relates to those compounds of Formula (I) and the pharmaceutically acceptable salts, and the solvates thereof, or any subgroup thereof as mentioned in any of the other embodiments, wherein $R^x$ represents hydrogen or $C_{2-6}$alkyl.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-x):

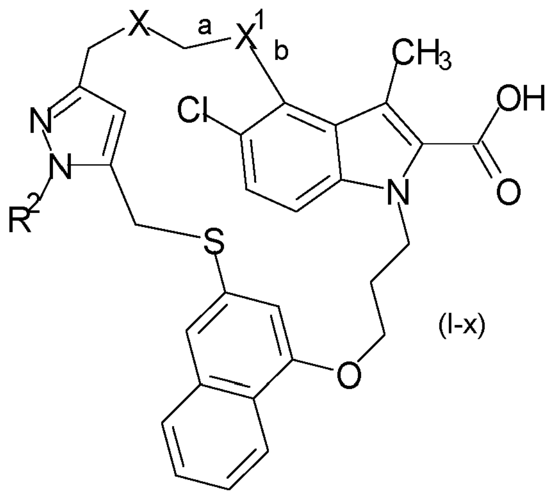

(I-x)

.

It will be clear that all variables in the structure of Formula (I-x), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-y):

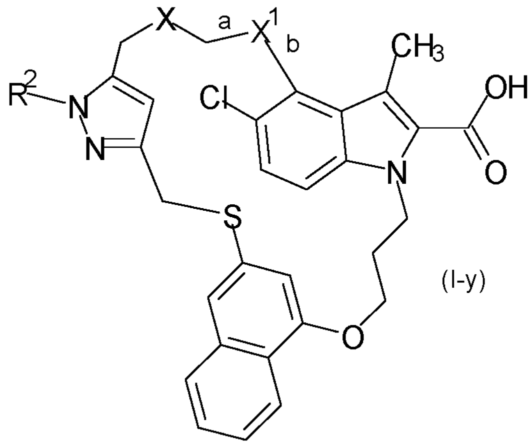

(I-y)

.

It will be clear that all variables in the structure of Formula (I-y), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the following compounds

| | | |
|---|---|---|
| 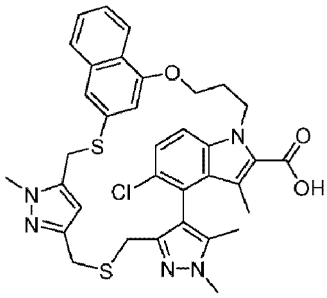 | 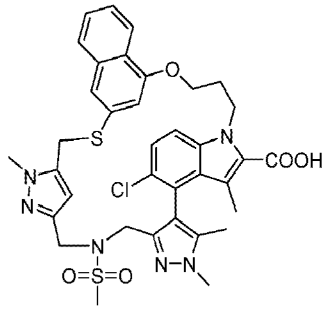 | 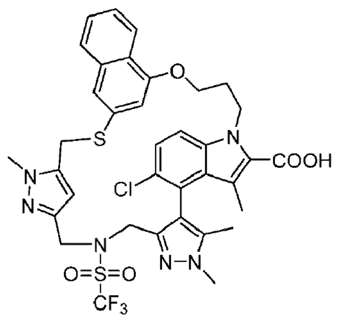 |
| 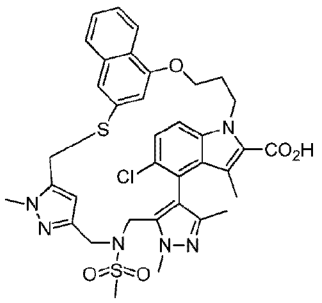 | 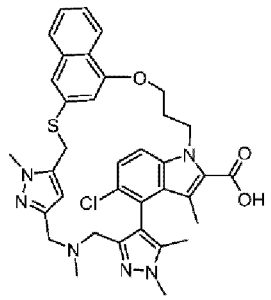 | |

and the tautomers and the stereoisomeric forms thereof are excluded. In an embodiment, the scope of the present invention does not include said excluded compounds, and the pharmaceutically acceptable salts thereof. In an embodiment, the scope of the present invention does not include said excluded compounds, and the pharmaceutically acceptable salts and the solvates thereof.

In an embodiment, the present invention relates to a subgroup of Formula (I) as defined in the general reaction schemes.

In an embodiment the compound of Formula (I) is selected from the group consisting of any of the exemplified compounds,

tautomers and stereoisomeric forms thereof,
any pharmaceutically acceptable salts, and the solvates thereof.

All possible combinations of the above indicated embodiments are considered to be embraced within the scope of the invention.

METHODS FOR THE PREPARATION OF COMPOUNDS

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or prepared by standard synthetic processes commonly used by those skilled in the art of organic chemistry. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under $N_2$-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.

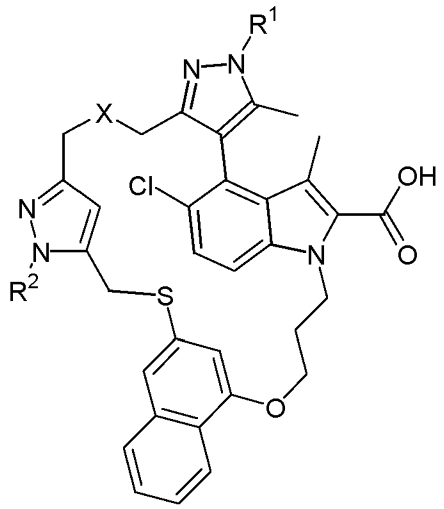

(I-a)

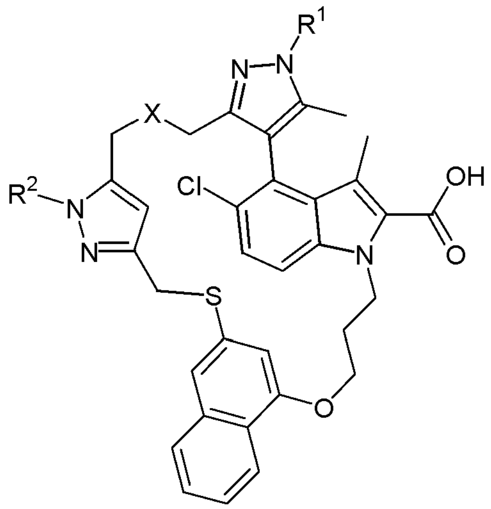

(I-b)

In Compounds of Formula (I-a) and (I-b) all variables are as defined for Formula (I) in the scope of this invention.

A skilled person will understand that analogous reaction protocols can also be used to prepare Compounds of the invention wherein $X^1$ represents

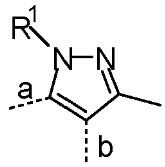

.

To obtain such compounds, during the conversion step of an intermediate of Formula (XVIII) to an intermediate of Formula (XVII), an alternative pyrazole-boronate should be used. The following reaction steps of the synthesis are analogous as described for Compounds (I-a) and (I-b).

In the schemes below, Me means methyl, Et means ethyl, and Ac means acetyl.

Compounds of Formula (I-a) can be prepared by reacting an intermediate of Formula (III),

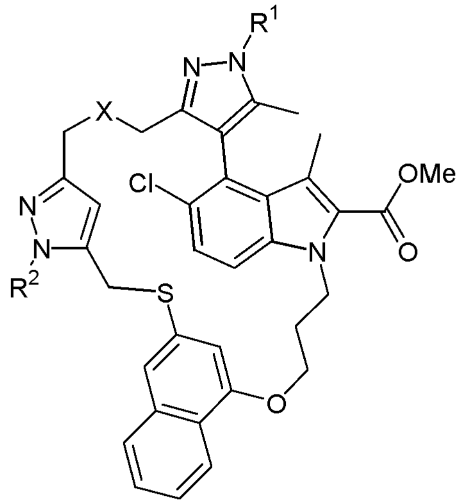

(III)

wherein X, $R^1$ and $R^2$ are as defined in Formula (I-a), in a suitable solvent, such as water or a mixture of water and a suitable organic solvent such as dioxane or THF, or a mixture of MeOH and THF, in the presence of a suitable base, such as, for example, LiOH or NaOH, at a suitable temperature, such as room temperature or 60 °C.

Similarly, Compounds of Formula (I-b) can be prepared by reacting a compound of Formula (IV),

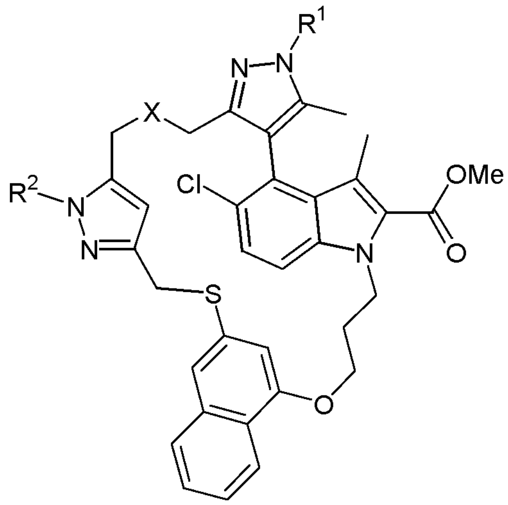

(IV)

where X, $R^1$ and $R^2$ are as defined in Formula (I-b), in a suitable solvent, such as water or a mixture of water and a suitable organic solvent such as dioxane or THF, or a mixture of MeOH and THF, in the presence of a suitable base, such as, for example, LiOH or NaOH, at a suitable temperature, such as room temperature or 60 °C.

Intermediates of Formula (III) and Intermediates of Formula (IV), can be prepared by reacting an intermediate of Formula (V),

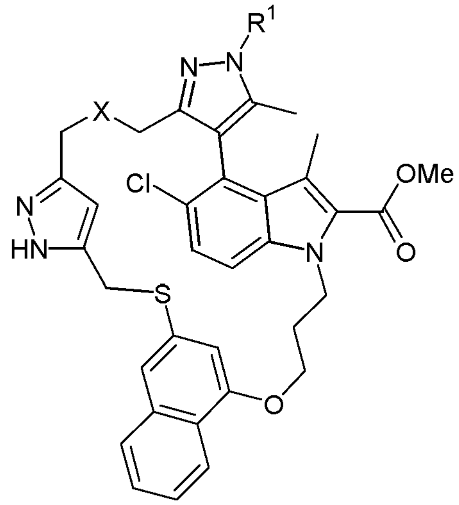

(V)

where X and $R^1$ are as defined in Formula (I-a) or Formula (I-b), with a suitable alkylating agent, such as, for example, an alkyl halide, in a suitable solvent, such as, for example, DMF, or acetonitrile, in the presence of a suitable base, such as, for example, trietylamine ($Et_3N$), N,N-Diisopropylethylamine ($iPr_2EtN$), or 1,8-Diazabicyclo[5.4.0]undec-7-*ene* (DBU), at a suitable temperature, such as, for example, room temperature or 60 °C, followed by a suitable separation of the isomers (III) and (IV), such as, for example, a chromatographic separation.

When X is defined as S (sulfur), intermediates of Formula (V) can be prepared by reacting an intermediate of Formula (VI),

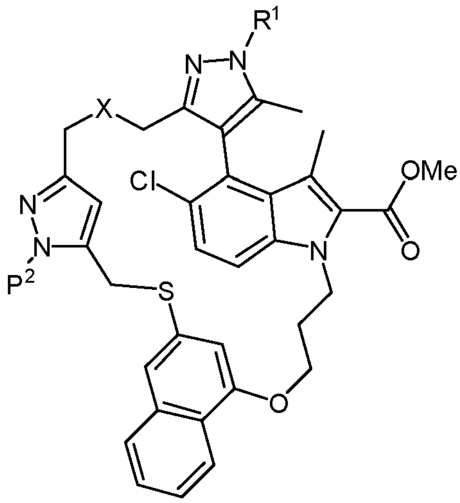

(VI)

where $P^2$ is defined as a suitable protecting group, such as, for example, paramethoxybenzyl (PMB), dimethoxylbenzyl (DMB), or tetrahydropyranyl (THP), and X is defined as S (sulfur) in this chemical protocol, with a suitable deprotecting agent, such as, for example, HCl or trifluoroacetic acid (TFA), or 2,3-Dichloro-5,6-dicyano-1,4-benzoquinone (DDQ) in a suitable solvent, such as, for example, 1,4-dioxane, $CH_2Cl_2$, or toluene, at a suitable temperature, such as, for example, room temperature or 80 °C.

When X is defined as $NR^x$, with $R^x$ as defined in Formula (I), intermediates of Formula (V) can be prepared by reacting a compound of Formula (VII),

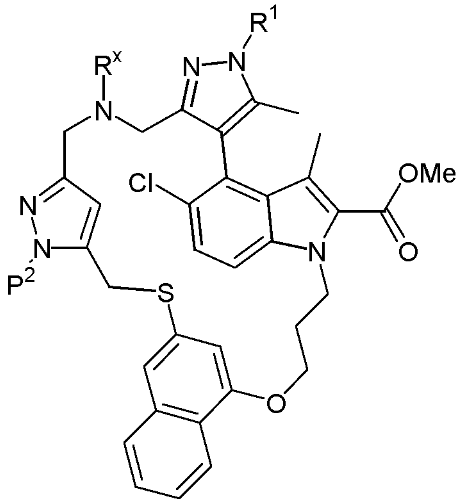

(VII)

where $P^2$ is a suitable protecting group, such as, for example, paramethoxybenzyl (PMB), dimethoxylbenzyl (DMB), or tetrahydropyranyl (THP), with a suitable deprotecting agent, such as, for example, HCl or TFA, or DDQ in a suitable solvent, such as, for example, 1,4-dioxane, $CH_2Cl_2$, or toluene, at a suitable temperature, such as, for example, room temperature or 80 °C.

Intermediates of Formula (VII) can be prepared by reacting an intermediate of Formula (VIII),

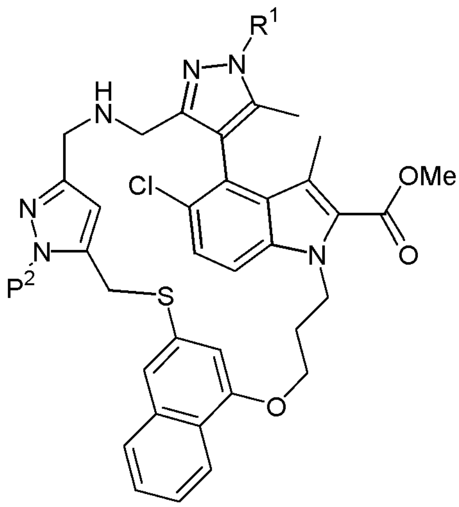

(VIII)

where $P^2$ is as defined in Formula (VII), with a suitable alkylating agent, such as, for example, an alkyl halide, in a suitable solvent, such as, for example, DMF, or acetonitrile, in the presence of a suitable base, such as, for example, $Et_3N$, $iPr_2EtN$, or DBU, at a suitable temperature, such as, for example, room temperature or 60 °C.

Alternatively, intermediates of Formula (VII) can be prepared by reacting an intermediate of Formula (VIII), with a suitable aldehyde, such as, for example, formaldehyde, and a suitable reducing agent, such as, for example, $NaBH_3CN$, in a suitable solvent, such as, for example, $CH_2Cl_2$, at a suitable temperature, such as, for example, room temperature.

Intermediates of Formula (VIII) can be prepared by reacting an intermediate of Formula (VI), where $P^2$ is as defined in Formula (VII), and X is defined as nitrogen protected by a protecting group such as for example 2-nitrophenylsulfonyl, with a suitable deprotecting agent, such as, for example, thiophenol, in the presence of a suitable base, such as, for example, $K_2CO_3$, in a suitable solvent, such as, for example, acetonitrile or DMF, at a suitable temperature, such as, for example, room temperature or 80 °C.

Intermediates of Formula (VI) can be prepared by reacting an intermediate of Formula (IX),

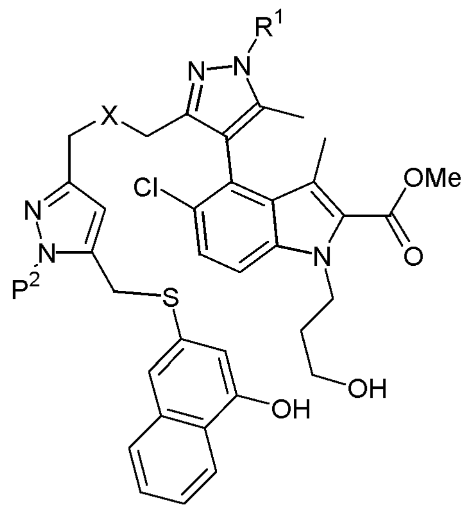

(IX)

where X is as defined in Formula (I-a), and $P^2$ is a suitable protecting group, such as, for example, paramethoxybenzyl (PMB), dimethoxylbenzyl (DMB), or tetrahydropyranyl (THP), or can also be a suitable alkyl substituent, such as, for example, methyl, with a suitable reagent, such as, for example, Diethyl azodicarboxylate (DEAD) or Di-tert-butyl azodicarboxylate (DBAD), in the presence of a suitable phosphine, such as, for example, $PPh_3$, in a suitable solvent, such as, for example, THF, toluene, or a mixture thereof, at a suitable temperature, such as, for example, room temperature or 60 °C.

Intermediates of Formula (IX) can be prepared by reacting an intermediate of Formula (X),

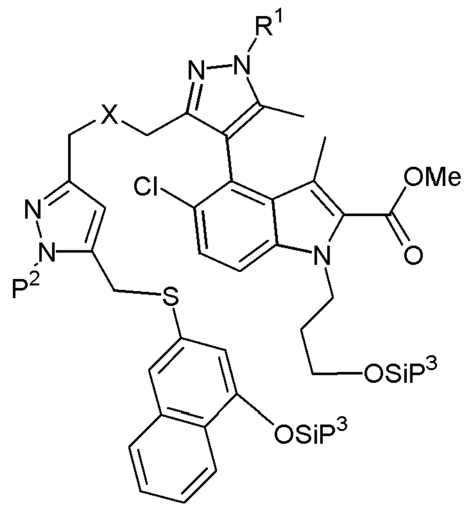

(X)

where X and $P^2$ are as defined in Formula (IX), and $SiP^3$ is a suitable protecting group, such as, for example, tert-butyldimethylsilyl (TBDMS) or tert-butyldiphenylsilyl (TBDPS), with a deprotecting reagent, such as, for example, tetrabutylammonium fluoride (TBAF), in a suitable solvent, such as, for example, THF, at a suitable temperature, such as, for example, room temperature or 60 °C.

When X is defined as S (sulfur), intermediates of Formula (X) can be prepared by reacting an intermediate of Formula (XI),

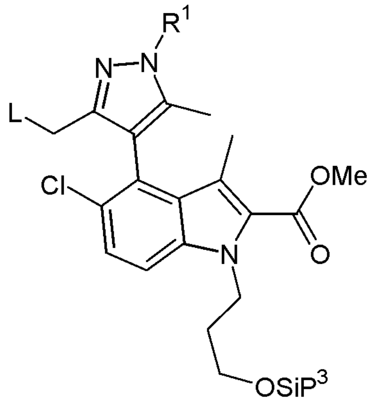

(XI)

where $SiP^3$ is as defined in Formula (X), and L is a suitable leaving group, such as, for example, an halogen or a alkyl sulfonate, with an intermediate of Formula (XII),

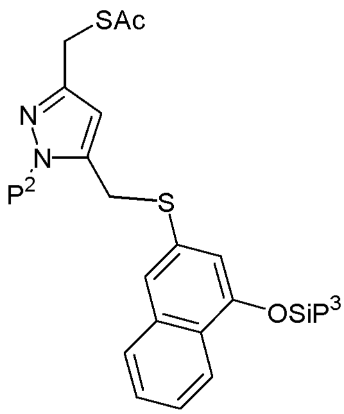

(XII)

where $SiP^3$ and $P^2$ are as defined in Formula (X),
in the presence of a suitable base, such as, for example, $K_2CO_3$, in a suitable solvent, such as, for example, MeOH, at a suitable temperature, such as, for example, room temperature or 60 °C.

When X is defined as nitrogen protected by a protecting group such as for example 2-nitrophenylsulfonyl, intermediates of Formula (X) can be prepared by reacting an intermediate of Formula (XIII),

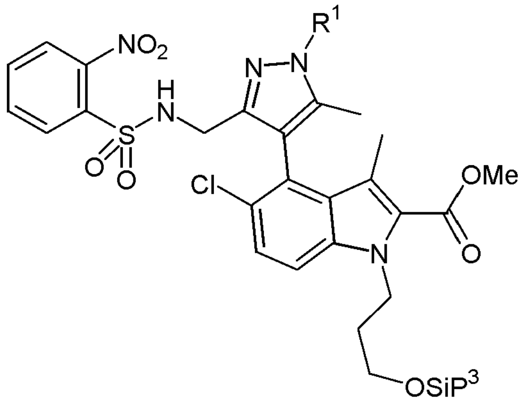

(XIII)

where $SiP^3$ is as defined in Formula (X), with intermediates of Formula (XIV),

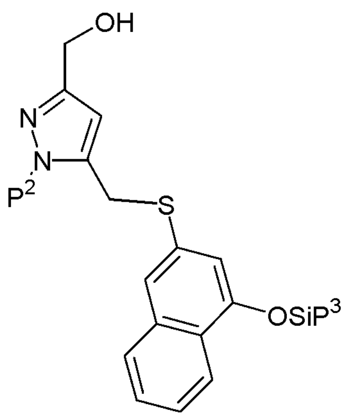

(XIV)

where $SiP^3$ and $P^2$ are as defined in Formula (X), with a suitable reagent, such as, for example, DEAD or DBAD, in the presence of a suitable phosphine, such as, for example, triphenylphosphine ($PPh_3$), in a suitable solvent, such as, for example, THF, toluene, or a mixture thereof, at a suitable temperature, such as, for example, room temperature or 60 °C.

Intermediates of Formula (XIII) can be prepared by reacting an intermediate of Formula (XV),

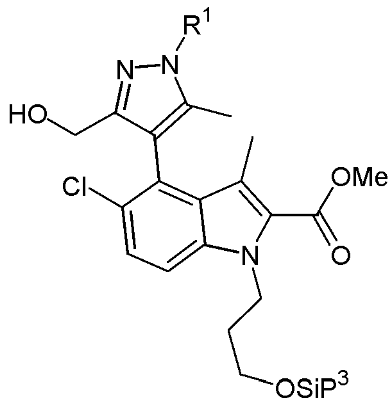

(XV)

where $SiP^3$ is as defined in Formula (X), with a suitable protected nitrogen, such as, for example, 2-nitrophenylsulfonamide, in the presence of a suitable reagent, such as, for example, DEAD or DBAD, in the presence of a suitable phosphine, such as, for example, $PPh_3$, in a suitable solvent, such as, for example, THF, toluene, or a mixture thereof, at a suitable temperature, such as, for example, room temperature or 60 °C.

Intermediates of Formula (XI) can be prepared by reacting an intermediate of Formula (XV), with a suitable alkylsulfonyl chloride, such as, for example, mesyl chloride, in the presence of a suitable base, such as, for example, triethylamine, in a suitable solvent, such as, for example, $CH_2Cl_2$, at a suitable temperature, such as, for example, room temperature.

Alternatively, intermediates of Formula (XI) can be prepared in two steps, by reacting an intermediate of Formula (XV), with a suitable alkylsulfonyl chloride, such as, for example, mesyl chloride, in the presence of a suitable base, such as, for example, triethylamine, in a suitable solvent, such as, for example, $CH_2Cl_2$, at a suitable temperature, such as, for example, room temperature; followed by reaction with a suitable metal halide, such as, for example, potassium iodide, in a suitable solvent, such as, for example, acetonitrile, at a suitable temperature, such as, for example, room temperature or 60 °C.

Intermediates of Formula (XV) can be prepared by reacting an intermediate of Formula (XVI),

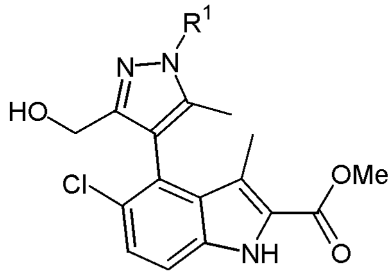

(XVI)

with a suitable O-protected propyl halide or alkyl sulfonate, such as, for example, (3-bromopropoxy)(tert-butyl)dimethylsilane, in the presence of a suitable base, such as, for example, $Cs_2CO_3$, in a suitable solvent, such as, for example, DMF, at a suitable temperature, such as, for example, room temperature.

An intermediate of Formula (XVI) can be prepared by reacting an intermediate of Formula (XVII),

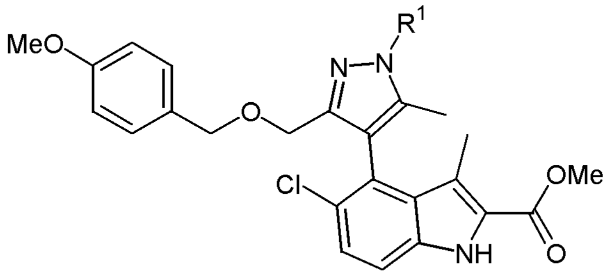

(XVII)

with a suitable deprotecting agent, such as, for example, trifluoromethanesulfonic acid or TFA, in a suitable solvent, such as, for example, $CH_2Cl_2$, at a suitable temperature, such as, for example, room temperature.

An intermediate of Formula (XVII) can be prepared by reacting an intermediate of Formula (XVIII),

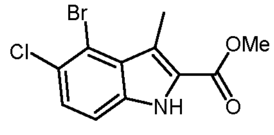

(XVIII)

with a suitable substituted pyrazole derivative, such as, for example, 3-(((4-methoxybenzyl)oxy)methyl)-1,5-dimethyl-4-(4,4,5,5-tetramethyl-1,3,2-dioxaborolan-2-yl)-1H-pyrazole , in the presence of a suitable catalyst, such as, for example, $Pd_2(dba)_3$, in the presence of a suitable phosphine ligand, such as, for example, S-Phos, in the presence of a suitable base, such as, for example, sodium bicarbonate, in a suitable solvent, such as, for example, dioxane, water, or a mixture thereof, at a suitable temperature, such as, for example, 100 °C.

An intermediate of Formula (XVIII) can be prepared by reacting an intermediate of Formula (XIX),

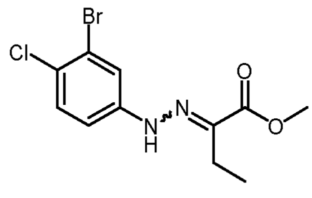

(mixture of (E) and (Z) isomers)

(XIX)

with a suitable acid, such as, for example, sulfuric acid, in a suitable solvent, such as, for example, acetic acid, at a suitable temperature, such as, for example, 70 °C.

An intermediate of Formula (XIX) can be prepared by reacting (3-bromo-4-chlorophenyl)hydrazine with methyl 2-oxobutanoate, in the presence of a suitable acid, such as, for example, hydrochloric acid, in a suitable solvent, such as, for example, methanol, at a suitable temperature, such as, for example, 65 °C.

Intermediates of Formula (XII) can be prepared by reacting an intermediate of Formula (XX),

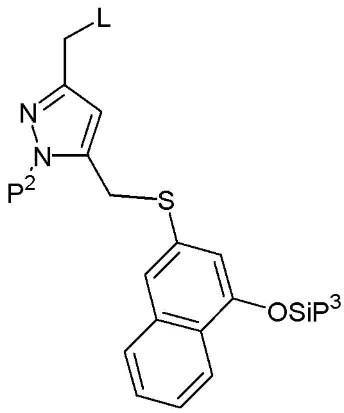

(XX)

where $SiP^3$ and $P^2$ are as defined in Formula (X) and L is a suitable leaving group, with potassium thioacetate, in a suitable solvent, such as, for example, DMF, at a suitable temperature, such as, for example, room temperature.

Intermediates of Formula (XX) can be prepared by reacting an intermediate of Formula (XIV), with a suitable reagent such as, for example, mesyl chloride or thionyl chloride, if necessary in the presence of a suitable base, such as, for example, triethylamine, in a suitable solvent, such as, for example, $CH_2Cl_2$, at a suitable temperature, such as, for example, 0 °C or room temperature.

Intermediates of Formula (XIV) can be prepared by reacting an intermediate of Formula (XXII),

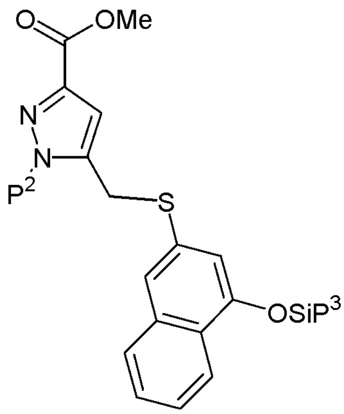

(XXII)

where $SiP^3$ and $P^2$ are as defined in Formula (X), with a suitable reducing agent such as, for example, DIBALH, in a suitable solvent, such as, for example, THF, at a suitable temperature, such as, for example, 0 °C or room temperature.

Intermediates of Formula (XXII) can be prepared by reacting an intermediate of Formula (XXIII),

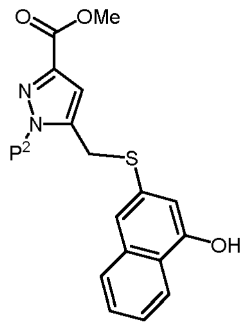

(XXIII)

where $P^2$ is as defined in Formula (X), with a suitable trisubstituted silyl chloride such as, for example, TBDMSCl (tert-butyldimethylsilyl chloride) or TBDPSCl (tert-butyldiphenylsilyl chloride), in the presence of a suitable base, such as, for example, imidazole, in a suitable solvent, such as, for example, DMF, at a suitable temperature, such as, for example, room temperature.

Intermediates of Formula (XXIII) can be prepared by reacting an intermediate of Formula (XXIV),

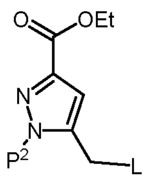

(XXIV)

where $P^2$ is as defined in Formula (X) and L is a suitable leaving group, such as, for example, chloride or mesylate, with 3-(acetylthio)naphthalen-1-yl acetate , in the presence of a suitable base, such as, for example, $K_2CO_3$, in a suitable solvent, such as, for example, methanol, at a suitable temperature, such as, for example, room temperature.

Intermediates of Formula (XXIV) can be prepared by reacting an intermediate of Formula (XXV),

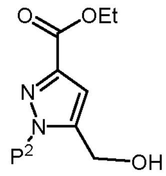

(XXV)

where $P^2$ is as defined in Formula (X), with a suitable reagent such as, for example, mesyl chloride or thionyl chloride, if necessary in the presence of a suitable base, such as, for example, triethylamine, in a suitable solvent, such as, for example, $CH_2Cl_2$, at a suitable temperature, such as, for example, 0 °C or room temperature.

Intermediates of Formula (XXV) can be prepared by reacting an intermediate of Formula (XXVI),

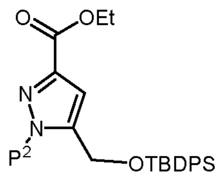

(XXVI)

where $P^2$ is as defined in Formula (X), with a deprotecting agent, such as, for example, TBAF, in a suitable solvent, such as, for example THF, at a suitable temperature, such as, for example, room temperature.

Intermediates of Formula (XXVI) can be prepared by reacting ethyl 5-(((tert-butyldiphenylsilyl)oxy)methyl)-1H-pyrazole-3-carboxylate, with a suitable protecting group precursor, such as, for example, paramethoxybenzyl chloride, dimethoxylbenzyl chloride, or also a suitable alkyl halide, such as, for example, methyl iodide, in the presence of suitable base, such as, for example, Sodium bis(trimethylsilyl)amide, in a suitable solvent, such as, for example THF, at a suitable temperature, such as, for example, 0 °C or room temperature.

Alternatively, intermediates of Formula (XXVI) can be prepared by reacting ethyl 5-(((tert-butyldiphenylsilyl)oxy)methyl)-1H-pyrazole-3-carboxylate, with a suitable protecting group precursor, such as, for example, 3,4-dihydro-2H-pyran, in the presence of suitable catalyst, such as, for example, p-toluenesulfonic acid (PTSA), in a suitable solvent, such as, for example tetrahydrofuran (THF) or $CH_2Cl_2$, at a suitable temperature, such as, for example, 0 °C or room temperature.

Alternatively, Intermediates of Formula (VI) can be prepared by reacting an intermediate of Formula (XXVII),

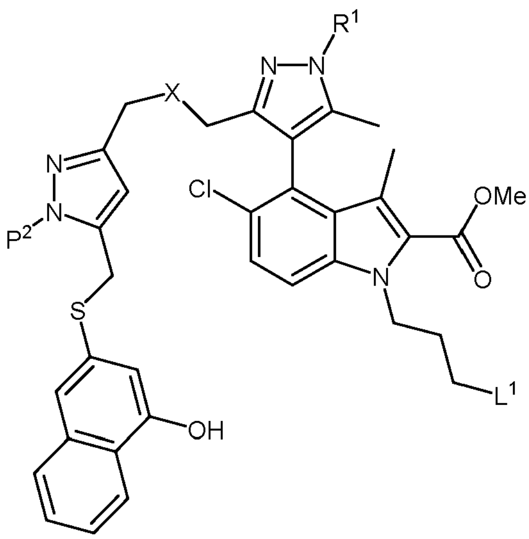

(XXVII)

Where X is as defined in Formula (I-a), and $P^2$ is a suitable protecting group such as, for example, tetrahydropyranyl (THP), or can also be a suitable alkyl substituent such as, for example, methyl, and $L^1$ is a suitable leaving group such as, for example, mesylate, in the presence of a suitable base such as, for example, $K_2CO_3$, in a suitable solvent such as, for example, acetonitrile, at a suitable temperature such as, for example, 80 °C.

Intermediates of Formula (XXVII) can be prepared by reacting an intermediate of Formula (XXVIII),

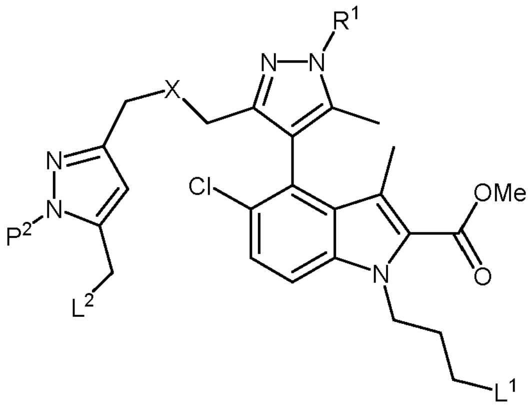

(XXVIII)

Where $L^2$ is a suitable leaving group such as, for example, iodide , with 3-(acetylthio)naphthalen-1-yl acetate , in the presence of a suitable base such as, for example, $K_2CO_3$, in the presence of a suitable catalyst such as, for example, $PPh_3$, in a suitable solvent such as, for example, methanol or THF, or a mixture thereof, at a suitable temperature such as, for example, 0 °C or room temperature.

Intermediates of Formula (XXVIII) can be prepared by reacting an intermediate of Formula (XXIX),

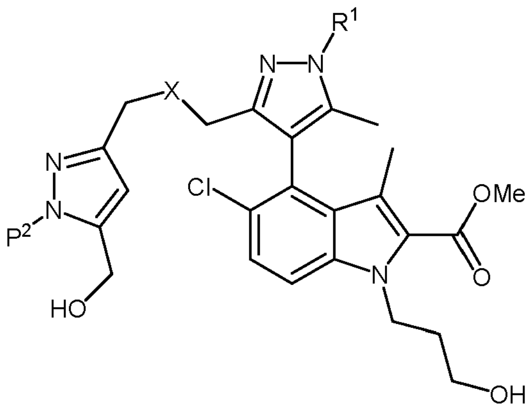

(XXIX)

First with a suitable activating agent such as, for example, mesyl anhydride, in the presence of a suitable base such as, for example, DIPEA, in a suitable solvent such as, for example, THF, at a suitable temperature such as, for example, 0 °C or room temperature; then by reacting the obtained intermediate bis-mesylate with a suitable halide such as, for example, sodium iodide, in a suitable solvent such as, for example, THF, at a suitable temperature such as, for example, room temperature.

Intermediates of Formula (XXIX) can be prepared by reacting an intermediate of Formula (XXX),

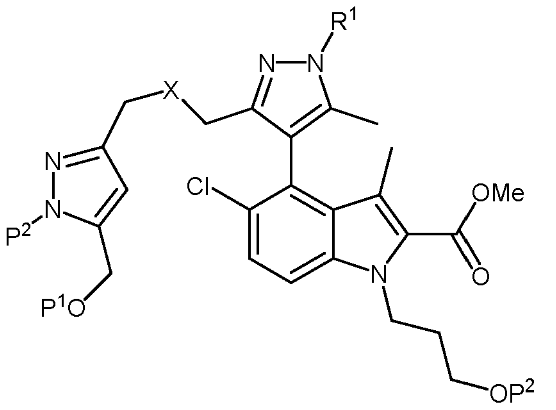

(XXX)

Where $P^1$ and $P^2$ are suitable protecting groups such as, for example, TBDMS or TBDPS, with a suitable deprotecting agent such as, for example, TBAF, in a suitable solvent such as, for example, THF, at a suitable temperature such as, for example, room temperature.

Intermediates of Formula (XXX) can be prepared by reacting an intermediate of Formula (XXXI),

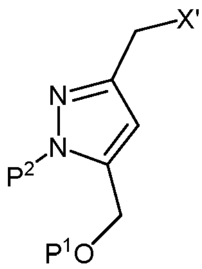

(XXXI)

Where X' is a suitable activated/protected form of X such as, for example, thioacetate, with an intermediate of Formula (XI), in the presence of a suitable base such as, for example, $K_2CO_3$, in a suitable solvent such as, for example, MeOH, at a suitable temperature such as, for example, room temperature.

Alternatively, Intermediates of Formula (XXX) can be prepared by reacting an intermediate of Formula (XXXI), where X' is a suitable activated/protected form of X such as, for example, 2-nitrophenylsulfonamide, with an intermediate of Formula (XV), in the presence of a suitable reagent such as, for example, DEAD or DBAD, and in the presence of a suitable phosphine such as, for example, triphenylphosphine ($PPh_3$), in a suitable solvent such as, for example, THF, toluene, or a mixture thereof, at a suitable temperature such as, for example, room temperature or 60 °C.

Intermediates of Formula (XXXI), where X' is a precursor of S (sulfur), can be prepared by reacting an intermediate of Formula (XXXII),

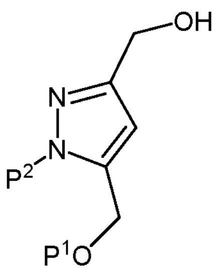

(XXXII)

First with a suitable activating agent such as, for example, MsCl, in the presence of a suitable base such as,

for example, $Et_3N$, in a suitable solvent such as, for example, THF, at a suitable temperature such as, for example, room temperature; then by reacting the obtained activated intermediate with a suitable source of X' such as, for example, potassium thioacetate, in a suitable solvent such as, for example, DMF or THF, or a mixture thereof, at a suitable temperature such as, for example, room temperature.

Alternatively, Intermediates of Formula (XXXI), where X' is a precursor of $NR^x$, can be prepared by reacting an intermediate of Formula (XXXII) with a suitable X' precursor such as, for example, 2-nitrophenylsulfonamide, in the presence of a suitable reagent such as, for example, DEAD or DBAD, and in the presence of a suitable phosphine such as, for example, triphenylphosphine ($PPh_3$), in a suitable solvent such as, for example, THF, toluene, or a mixture thereof, at a suitable temperature such as, for example, room temperature or 60 °C.

Intermediates of Formula (XXXII) can be prepared by reacting an intermediate of Formula (XXVI), with a suitable reducing agent such as, for example, DIBAL-H, in a suitable solvent such as, for example, THF, at a suitable temperature such as, for example, 0 °C.

It will be apparent for the skilled person that intermediates of Formula (XXVII) can also bear the protecting group $P_2$ on the other pyrazole nitrogen. In that case, an analogous synthetic pathway can be used, starting from the corresponding isomer of Intermediate (XXVI).

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

## PHARMACOLOGY OF COMPOUNDS

It has been found that the compounds of the present invention inhibit one of more MCL-1 activities, such as MCL-1 antiapoptotic activity.

An MCL-1 inhibitor is a compound that blocks one or more MCL-1 functions, such as the ability to bind and repress proapoptotic effectors Bak and Bax or BH3 only sensitizers such as Bim, Noxa or Puma.

The compounds of the present invention can inhibit the MCL-1 pro-survival functions. Therefore, the compounds of the present invention may be useful in treating and / or preventing, in particular treating, diseases that are susceptible to the effects of the immune system such as cancer.

In another embodiment of the present invention, the compounds of the present invention exhibit anti-tumoral properties, for example, through immune modulation.

In an embodiment, the present invention is directed to a therapeutically effective amount of a compound of Formula (I), or pharmaceutically acceptable salt, or a solvate thereof, for use in treating and / or preventing a cancer, wherein the cancer is selected from those described herein.

In an embodiment, the present invention is directed to a therapeutically effective amount of a compound of Formula (I), or a pharmaceutically acceptable salt, or a solvate thereof, for use in treating and / or preventing cancer wherein the cancer is selected from the group consisting of acute lymphoblastic leukemia (ALL), acute myeloid leukemia (AML), B cells acute lymphoblastic leukemia, B-cell chronic lymphocytic leukemia (CLL), bladder cancer, breast cancer, chronic lymphocytic leukemia, chronic myeloid leukemia, colon adenocarcinoma, diffuse large B cell lymphoma, esophageal cancer, follicular lymphoma, gastric cancer, head and neck cancer (including, but not limited to head and neck squamous cell carcinoma), hematopoietic cancer, hepatocellular carcinoma, Hodgkin lymphoma, liver cancer, lung cancer (including but not limited to lung adenocarcinoma), lymphoma, medulloblastoma, melanoma, monoclonal gammopathy of undetermined significance, multiple myeloma, myelodysplastic syndromes, myelofibrosis, myeloproliferative neoplasms, ovarian cancer, ovarian clear cell carcinoma, ovarian serous cystadenoma, pancreatic cancer, polycythemia vera, prostate cancer, rectum adenocarcinoma, renal cell carcinoma, smoldering multiple myeloma, T cell acute lymphoblastic leukemia, T cell lymphoma, and Waldenstroms macroglobulinemia.

In another embodiment, the present invention is directed to a therapeutically effective amount of a compound of Formula (I), or a pharmaceutically acceptable salt, or a solvate thereof, for use in treating and / or preventing cancer, wherein the cancer is preferably selected from the group consisting of acute lymphoblastic leukemia (ALL), acute myeloid leukemia (AML), B cells acute lymphoblastic leukemia, B-cell chronic lymphocytic leukemia (CLL), breast cancer, chronic lymphocytic leukemia, chronic myeloid leukemia, diffuse large B cell lymphoma, follicular lymphoma, hematopoietic cancer, Hodgkin lymphoma, lung cancer (including, but not limited to lung adenocarcinoma) lymphoma, monoclonal gammopathy of undetermined significance, multiple myeloma, myelodysplastic syndromes, myelofibrosis, myeloproliferative neoplasms, smoldering multiple myeloma, T cell acute lymphoblastic leukemia, T cell lymphoma and Waldenstroms macroglobulinemia.

In another embodiment, the present invention is directed to a therapeutically effective amount of a compound of Formula (I), or a pharmaceutically acceptable salt, or a solvate thereof, for use in treating and / or preventing cancer, wherein the cancer is selected from the group consisting of adenocarcinoma, benign monoclonal gammopathy, biliary cancer (including, but not limited to, cholangiocarcinoma), bladder cancer, breast cancer (including, but not limited to, adenocarcinoma of the breast, papillary carcinoma of the breast, mammary cancer, medullary carcinoma of the breast), brain cancer (including, but not limited to, meningioma), glioma (including, but not limited to, astrocytoma, oligodendroglioma; medulloblastoma), bronchus cancer, cervical cancer (including, but not limited to, cervical adenocarcinoma), chordoma, choriocarcinoma, colorectal cancer (including, but not limited to, colon cancer, rectal cancer, colorectal adenocarcinoma), epithelial carcinoma, endothelial sarcoma (including, but not limited to, Kaposi’s sarcoma, multiple idiopathic hemorrhagic sarcoma), endometrial cancer (including, but not limited to, uterine cancer, uterine sarcoma), esophageal cancer (including, but not limited to, adenocarcinoma of the esophagus, Barrett’ s adenocarinoma), Ewing sarcoma, gastric cancer (including, but not limited to, stomach adenocarcinoma), gastrointestinal stromal tumor (GIST), head and neck cancer (including, but not limited to, head and neck squamous cell carcinoma), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma. splenic marginal zone B-cell lymphoma), primary mediastinal B-cell lymphoma, Burkitt lymphoma, lymphoplasmacytic lymphoma (including, but not limited to, Waldenstrom’s macro globulinemia), immunoblastic large cell lymphoma, hairy cell leukemia (HCL), precursor B - lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, kidney cancer (including, but not limited to, nephroblastoma a.k.a. Wilms’ tumor, renal cell carcinoma), liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, *Lewis lung carcinoma,* lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES), ovarian cancer (including, but not limited to, cystadenocarcinoma, ovarian embryonal carcinoma, ovarian adenocarcinoma), pancreatic cancer (including, but not limited to, pancreatic andenocarcinoma, intraductal papillary mucinous neoplasm (IPMN), Islet cell tumors), prostate cancer (including, but not limited to, prostate adenocarcinoma), skin cancer (including, but not limited to, squamous cell carcinoma (SCC), keratoacanthoma (KA), melanoma, basal cell carcinoma (BCC)) and soft tissue sarcoma (e.g. malignant fibrous histiocytoma (MFH), liposarcoma, malignant peripheral nerve sheath tumor (MPNST), chondrosarcoma, fibrosarcoma, myxosarcoma).

In another embodiment, the present invention is directed to a therapeutically effective amount of a compound of Formula (I), or a pharmaceutically acceptable salt, or a solvate thereof, for use in treating and / or preventing cancer, wherein the cancer is selected from the group consisting of benign monoclonal gammopathy, breast cancer (including,

but not limited to, adenocarcinoma of the breast, papillary carcinoma of the breast, mammary cancer, medullary carcinoma of the breast), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T-cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma. splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, Lewis lung carcinoma, lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), and prostate cancer (including, but not limited to, prostate adenocarcinoma).

In another embodiment, the present invention is directed to a therapeutically effective amount of a compound of Formula (I), or a pharmaceutically acceptable salt, or a solvate thereof, for treating and / or preventing cancer, wherein the cancer is selected from the group consisting of prostate, lung, pancreatic, breast, ovarian, cervical, melanoma, B-cell chronic lymphocytic leukemia (CLL), acute myeloid leukemia (AML), and acute lymphoblastic leukemia (ALL).

In another embodiment, the present invention is directed to a therapeutically effective amount of a compound of Formula (I), or a pharmaceutically acceptable salt, or a solvate thereof, for treating and / or preventing cancer, wherein the cancer is multiple myeloma.

The compounds according to the present invention or pharmaceutical compositions comprising said compounds, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1 and or CTLA-4 or engineered chimeric antigen receptor T cells (CART) targeting tumor associated antigens.

The compounds according to the present invention or pharmaceutical compositions comprising said compounds, may also be combined with radiotherapy or chemotherapeutic agents (including, but not limited to, anti-cancer agents) or any other pharmaceutical agent which is administered to a subject having cancer for the treatment of said subject's cancer or for the treatment or prevention of side effects associated with the treatment of said subject's cancer.

The compounds according to the present invention or pharmaceutical compositions comprising said compounds, may also be combined with other agents that stimulate or enhance the immune response, such as vaccines.

In an embodiment, the present invention is directed to a therapeutically effective amount of co-therapy or combination therapy for use in treating and / or preventing a cancer (wherein the cancer is selected from those described herein); wherein the co-therapy or combination therapy comprises a compound of Formula (I) of the present invention and one or more anti-cancer agent(s) selected from the group consisting of (a) immune modulatory agent (such as inhibitors of the PD1/PDL1 immune checkpoint axis, for example antibodies (or peptides) that bind to and/or inhibit the activity of PD-1 or the activity of PD-L1 and or CTLA-4); (b) engineered chimeric antigen receptor T cells (CART) targeting tumor associated antigens; (c) radiotherapy; (d) chemotherapy; and (e) agents that stimulate or enhance the immune response, such as vaccines.

The present invention is directed to compounds of Formula (I) and pharmaceutically acceptable salts, and solvates thereof, for use as a medicament.

The present invention is directed to compounds of Formula (I) and pharmaceutically acceptable salts, and solvates thereof, for use in the inhibition of MCL-1 activity.

As used herein, unless otherwise noted, the term "anti-cancer agents" shall encompass "anti-tumor cell growth agents" and "anti-neoplastic agents".

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, MCL-1 mediated diseases or conditions, preferably cancer, more preferably a cancer as herein described (for example, multiple myeloma).

The present invention is directed to compounds of Formula (I) and pharmaceutically acceptable salts, and solvates thereof, for use in treating and / or preventing, in particular for use in treating, MCL-1 mediated diseases or conditions, preferably cancer, more preferably a cancer as herein described (for example, multiple myeloma).

The present invention relates to compounds of Formula (I) and pharmaceutically acceptable salts, and solvates thereof, for the manufacture of a medicament.

The present invention relates to compounds of Formula (I) and pharmaceutically acceptable salts, and solvates thereof, for the manufacture of a medicament for the inhibition of MCL-1.

The present invention relates to compounds of Formula (I) and pharmaceutically acceptable salts, and solvates thereof, for the manufacture of a medicament for treating and / or preventing, in particular for treating, a cancer, preferably a cancer as herein described. More particularly, the cancer is a cancer which responds to inhibition of MCL-1 (for example, multiple myeloma).

The present invention is directed to compounds of Formula (I) and pharmaceutically acceptable salts, and solvates thereof, for the manufacture of a medicament for treating and / or preventing, in particular for treating, any one of the disease conditions mentioned hereinbefore.

The present invention is directed to compounds of Formula (I) and pharmaceutically acceptable salts, and solvates thereof, for the manufacture of a medicament for treating and / or preventing any one of the disease conditions mentioned hereinbefore.

The compounds of Formula (I) and pharmaceutically acceptable salts, and solvates thereof, can be administered to subjects, preferably humans, for use in treating and / or preventing of any one of the diseases mentioned hereinbefore.

An effective amount of a compound of Formula (I) or a pharmaceutically acceptable salt, or a solvate thereof, can be administered to subjects such as humans, i.e. systemic or topical administration, preferably oral or intravenous administration, more preferably oral administration.

One skilled in the art will recognize that a therapeutically effective amount of the compounds of the present invention is the amount sufficient to have therapeutic activity and that this amount varies *inter alias,* depending on the type of disease, the concentration of the compound in the therapeutic formulation, and the condition of the patient. In an embodiment, a therapeutically effective daily amount may be from about 0.005 mg/kg to 100 mg/kg.

The amount of a compound according to the present invention, also referred to herein as the active ingredient, which is required to achieve a therapeutic effect may vary on case-by-case basis, for example with the specific compound, the route of administration, the age and condition of the recipient, and the particular disorder or disease being treated. The methods of the present invention may also include administering the active ingredient on a regimen of between one and four intakes per day. In these methods of the present invention, the compounds according to the invention are preferably formulated prior to administration.

The present invention also provides compositions for treating and / or preventing the disorders (preferably a cancer as described herein) referred to herein. Said compositions comprise a therapeutically effective amount of a compound of Formula (I), or a pharmaceutically acceptable salt, or a solvate thereof, and a pharmaceutically acceptable carrier or diluent.

While it is possible for the active ingredient (e.g. a compound of the present invention) to be administered alone, it is preferable to administer it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of the present invention may be prepared by any methods well known in the art of pharmacy, for example, using methods such as those described in, for example, Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own

separate pharmaceutical dosage formulation.

Therefore, in an embodiment, the present invention is directed to a product comprising, as a first active ingredient a compound according to the invention and as further, as an additional active ingredient one or more anti-cancer agent(s), as a combined preparation for simultaneous, separate or sequential use in the treatment of patients suffering from cancer.

The one or more other anti-cancer agents and the compound according to the present invention may be administered simultaneously (e.g. in separate or unitary compositions) or sequentially, in either order. In an embodiment, the two or more compounds are administered within a period and / or in an amount and / or a manner that is sufficient to ensure that an advantageous or synergistic effect is achieved. It will be appreciated that the preferred method and order of administration and the respective dosage amounts and regimes for each component of the combination will depend on the particular other anti-cancer agent and the compound of the present invention being administered, their route of administration, the particular condition, in particular tumor, being treated and the particular host being treated.

The following examples further illustrate the present invention.

EXAMPLES

Several methods for preparing the Compounds of this invention are illustrated in the following examples. Unless otherwise noted, all starting materials were obtained from commercial suppliers and used without further purification, or alternatively can be synthesized by a skilled person by using well-known methods.

| Abbreviation | Meaning |
|---|---|
| **2-Me-THF** | 2-methyltetrahydrofuran |
| **CAN or ACN** | acetonitrile |
| **BuLi** | n-butyllithium |
| **DTBAD** | di-tert-butyl azodicarboxylate |
| **DCM** | dichloromethane |
| **DIBAL-H** | di-isobutylaluminiumhydride |
| **S-Phos** | 2-Dicyclohexylphosphino-2',6'-dimethoxybiphenyl |
| **min** | minute(s) |
| **DMF** | *N,N*-dimethylformamide |
| **Me** | methyl |
| **$Pd_2(dba)_3$** | tris(dibenzylideneacetone)dipalladium |
| **$Pd(amphos)Cl_2$** | bis(di-tert-butyl(4-dimethylaminophenyl)phosphine)dichloropalladium(II) |
| **$PPh_3$** | triphenylphosphine |
| **EtOAc** | ethyl acetate |
| **eq.** | equivalent(s) |
| **EtOH** | ethanol |
| **quant.** | quantitative |
| **h** | hour(s) |
| **DCE** | 1,2-dichloroethane |
| **DIBALH** | di-isobutylaluminiumhydride |
| **DIPEA** | N,N-diisopropylethylamine |
| **DMAP** | 4-dimethylaminopyridine |
| **HPLC** | high performance liquid chromatography |
| **MeOH** | methanol |
| **MsCl** | methanesulfonyl chloride |
| **$Ms_2O$** | methanesulfonic anhydride |

(continued)

| Abbreviation | Meaning |
|---|---|
| **$NaBH(OAc)_3$** | sodium triacetoxyborohydride |
| **SFC** | super critical fluid chromatography |
| **THF** | tetrahydrofuran |
| **TLC** | thin layer chromatography |
| **Celite®** | diatomaceous earth |
| **RP** | reversed phase |
| **$i\text{-}PrNH_2$** | isopropylamine |
| **TBAF** | tetrabutylammonium fluoride |
| **TBDMS** | tert-butyldimethylsilyl |
| **TBDPS** | tert-butyldiphenylsilyl |

As understood by a person skilled in the art, Compounds synthesized using the protocols as indicated may contain residual solvent or minor impurities.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

**Preparation of intermediates**

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

**Methyl (E)-2-(2-(3-bromo-4-chlorophenyl)hydrazono)butanoate (Intermediate 1)**

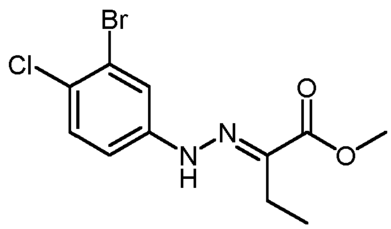

A solution of (3-bromo-4-chlorophenyl)hydrazine (4.655 g, 18.047 mmol) and methyl 2-oxobutanoate (1.02 eq) in HCl (93 mL, 1.25 M in MeOH) was refluxed for 90 min. The reaction was cooled to room temperature and volatiles were removed under reduced pressure to give 5.768 g of Intermediate 1 as a brown oily residue that solidified within minutes. The crude was used as such in the following step.

**Methyl 4-bromo-5-chloro-3-methyl-1H-indole-2-carboxylate (Intermediate** 2)

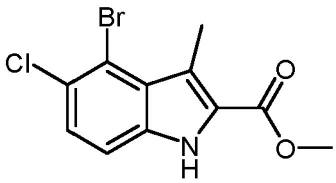

A suspension of Intermediate 1 (5.768 g, crude) in acetic acid (37 mL) was heated to 70 °C. Sulfuric acid (4.81 mL, 5 eq.) was added dropwise over 10 min (exotherm developed and a precipitate formed). After 15 additional min, the reaction was cooled to room temperature and then to 0 °C by adding ice. The solid precipitate was filtered and washed with water until the filtrate was of neutral pH. The solid was triturated with cold heptane/diisopropylether (8/2, 50 mL) to give an off-white solid. This solid was purified by preparative SFC (Stationary phase: Chiralpak Daicel IG 20 x 250 mm, Mobile phase: $CO_2$, EtOH + 0.4 % i-$PrNH_2$) to give Intermediate 2 (1.745 g, 32 %).

**Methyl 5-chloro-4-(3-(((4-methoxybenzyl)oxy)methyl)-1,5-dimethyl-1H-pyrazol-4-yl)-3-methyl-1H-indole-2-carboxylate (Intermediate 3)**

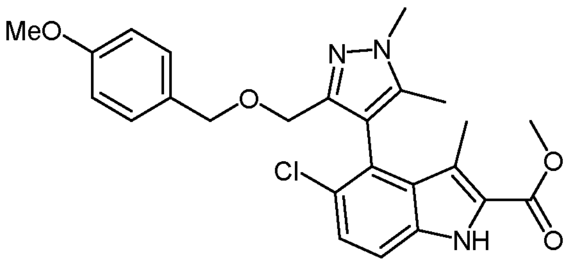

Intermediate 2 (500 mg), 3-(((4-methoxybenzyl)oxy)methyl)-1,5-dimethyl-4-(4,4,5,5-tetramethyl-1,3,2-dioxaborolan-2-yl)-1H-pyrazole (800 mg, 1.3 eq.), $Pd_2(dba)_3$ (76 mg, 0.05 eq.), and S-Phos (68 mg, 0.1 eq.) were weighed in a pressure tube under $N_2$. Dioxane (10.5 mL) and a saturated aqueous $NaHCO_3$ solution (4.5 mL) were added and the mixture was heated at 100 °C for 2 h. The reaction was cooled to room temperature, diluted with EtOAc (40 mL) and water (40 mL). The organic layer was separated and the aqueous one was extracted with EtOAc (40 mL). The combined organic layer was dried over $MgSO_4$, filtered and evaporated. The crude mixture was purified by flash chromatography on silica gel (40 g, gradient: from heptane 100 % up to heptane/EtOAc 4/6). Intermediate 3 (790 mg, 89 %) was obtained as a yellowish oil that solidified on standing. Intermediate 3 was used as such in the next reaction step.

**Methyl 5-chloro-4-(3-(hydroxymethyl)-1,5-dimethyl-1H-pyrazol-4-yl)-3-methyl-1H-indole-2-carboxylate (Intermediate 4)**

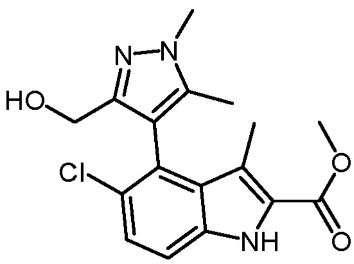

Trifluoromethanesulfonic acid (0.888 mL, 5 eq.) was added to a solution of Intermediate 3 (1080 mg) in DCM (25 mL). The reaction was stirred at room temperature for 1 h. The reaction was diluted with DCM (100 mL) and treated with $NaHCO_3$ sat (30 mL). The organic phase was separated and the aqueous one was extracted with DCM (50 mL x 3). The combined organic layer was dried over $MgSO_4$, filtered and evaporated. Intermediate 4 (625 mg, 89 %) was obtained as a yellowish solid and used as such in the following step.

**Methyl 1-(3-((tert-butyldimethylsilyl)oxy)propyl)-5-chloro-4-(3-(hydroxymethyl)-1,5-dimethyl-1H-pyrazol-4-yl)-3-methyl-1H-indole-2-carboxylate (Intermediate 5)**

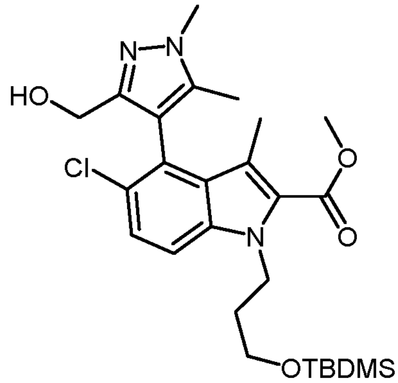

Cesium carbonate (732 mg, 1.25 eq.) was added to a solution of Intermediate 4 (625 mg) in DMF (10 mL) under nitrogen atmosphere. (3-Bromopropoxy)(tert-butyl)dimethylsilane (0.458 mL, 1.1 eq.) was added dropwise and the reaction was stirred at room temperature overnight. The reaction was diluted with EtOAc (100 mL) and water (50 mL). The organic layer was separated and washed with brine (2 x 30 mL). The combined aqueous layers were extracted with EtOAc (50 mL). The combined organic layer was then dried over $MgSO_4$, filtered and evaporated. The crude mixture was purified by flash chromatography on silica gel (40 g, gradient: from heptane 100 % up to EtOAc 100 %) to afford Intermediate 5 (360 mg, 38 %) as a white solid.

**Methyl 1-(3-((tert-butyldimethylsilyl)oxy)propyl)-5-chloro-4-(1,5-dimethyl-3-(((methylsulfonyl)oxy)methyl)-1H-pyrazol-4-yl)-3-methyl-1H-indole-2-carboxylate (Intermediate 6)**

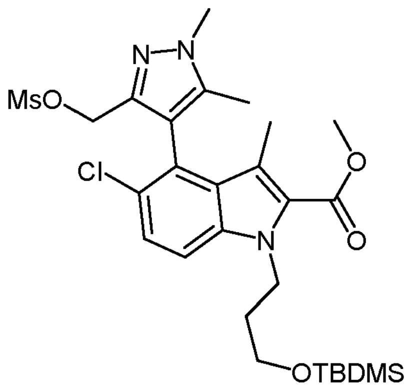

Mesyl chloride (0.12 mL, 2.5 eq.) was added dropwise to a solution of Intermediate 5 (320 mg) and triethylamine (0.256 mL, 3 eq.) in DCM (10 mL) stirring at 0 °C under nitrogen. The reaction was then allowed to warm up to room temperature and was stirred at room temperature. Additional triethylamine (3 eq.) and mesyl chloride (2.5 eq.) were added and stirring was continued at room temperature for 1 h. The reaction mixture was diluted with DCM (10 mL) and treated with saturated aqueous $NaHCO_3$ (5 mL). The organic layer was separated and the aqueous one was extracted with DCM (10 mL). The combined organic layer was dried over $MgSO_4$, filtered and evaporated to give Intermediate 6 (368 mg, quantitative), used as such in the following step.

**Methyl 1-(3-((tert-butyldimethylsilyl)oxy)propyl)-5-chloro-4-(3-(iodomethyl)-1,5-dimethyl-1H-pyrazol-4-yl)-3-methyl-1H-indole-2-carboxylate (Intermediate** 7)

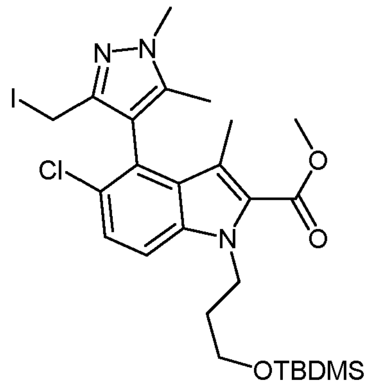

Potassium iodide (1.021 g, 10 eq.) was added to a solution of Intermediate 6 (368 mg) in acetonitrile (5 mL). The reaction was stirred at room temperature overnight. The reaction mixture was diluted with EtOAc (50 mL) and filtered over Dicalite®. Water (25 mL) was added to the filtrate and, after some stirring, the organic layer was separated. The aqueous layer was back-extracted with EtOAc (25 mL). The combined organic layer was dried over $MgSO_4$, filtered and evaporated to give Intermediate 7, used as such in the following step.

**Methyl 5-(((4-((tert-butyldimethylsilyl)oxy)naphthalen-2-yl)thio)methyl)-1-methyl-1H-pyrazole-3-carboxylate (Intermediate 8)**

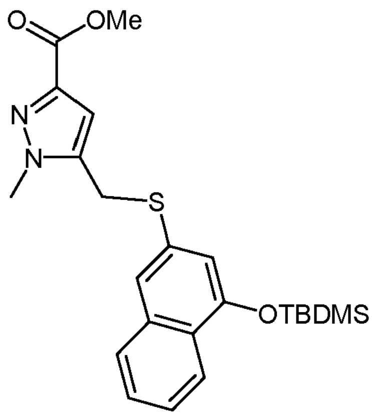

Imidazole (258 mg, 1.4 eq.) was added to a solution of methyl 5-(((4-hydroxynaphthalen-2-yl)thio)methyl)-1-methyl-1H-pyrazole-3-carboxylate (890 mg) and TBDMSCl (511 mg, 1.25 eq.) in DMF (17.8 mL). The reaction was stirred at room temperature for 48 h. The reaction mixture was diluted with EtOAc (100 mL) and water (50 mL). The organic layer was separated and washed with brine (2 × 50 mL). The combined aqueous layers were extracted with EtOAc (50 mL). The combined organic layers were dried over $MgSO_4$, filtered and evaporated. The crude mixture was purified by flash chromatography on silica gel (40 g, gradient: from heptane 100 % up to heptane/EtOAc 6/4) to obtain Intermediate 8 (1.24 g, quant.).

**(5-(((4-((tert-butyldimethylsilyl)oxy)naphthalen-2-yl)thio)methyl)-1-methyl-1H-pyrazol-3-yl)methanol (Intermediate 9)**

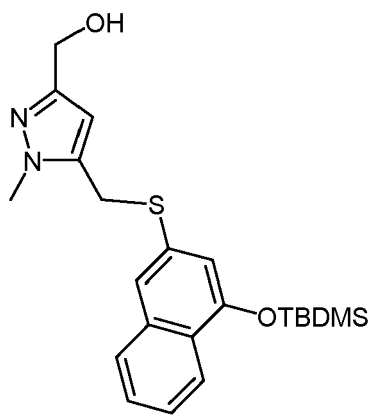

DIBALH (1M in heptane, 5.82 mL, 2.5 eq.) was added dropwise to a solution of Intermediate 8 in THF (40 mL) at 0 °C under nitrogen atmosphere and the reaction was stirred at 0 °C for 30 min. Additional DIBALH (1 eq.) was added and the reaction mixture was further stirred for 10 min. The reaction was treated with wet THF (40 mL) and, after a few min stirring, with water (10 mL, initial dropwise addition). The mixture was allowed to warm up to room temperature and then Celite® was added. After 5 min stirring, the mixture was filtered, washing with EtOAc. The filtrate was treated with $MgSO_4$, filtered and evaporated to give crude Intermediate 9 (892 mg, 92 %) as a colorless paste that solidified upon standing. Intermediate 9 was used as such in the following step.

**5-(((4-((tert-butyldimethylsilyl)oxy)naphthalen-2-yl)thio)methyl)-3-(chloromethyl)-1-methyl-1H-pyrazole (Intermediate 10)**

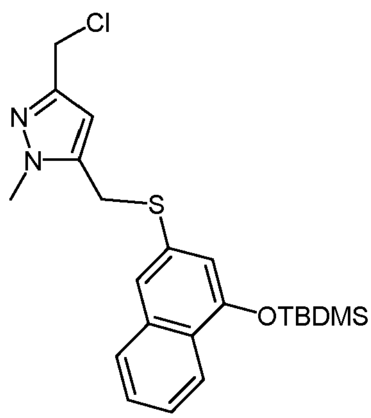

Thionyl chloride (0.187 mL, 1.2 eq.) was added dropwise to a solution of Intermediate 9 (892 mg) in DCM (20 mL) stirring at 0 °C under nitrogen atmosphere. The reaction was then allowed to warm up to room temperature and stirred for 1 h. The reaction mixture was cooled to 0 °C, diluted with DCM (20 mL) and quenched with a saturated aqueous solution of $NaHCO_3$ (20 mL). The organic layer was separated and the aqueous one was extracted with DCM (20 mL). The combined organic layer was dried over $MgSO_4$, filtered and evaporated. Intermediate 10 (931 mg, quant.) was obtained as a colorless oil and used without further purification.

**S-((5-(((4-((tert-butyldimethylsilyl)oxy)naphthalen-2-yl)thio)methyl)-1-methyl-1H-pyrazol-3-yl)methyl) ethanethioate (Intermediate 11)**

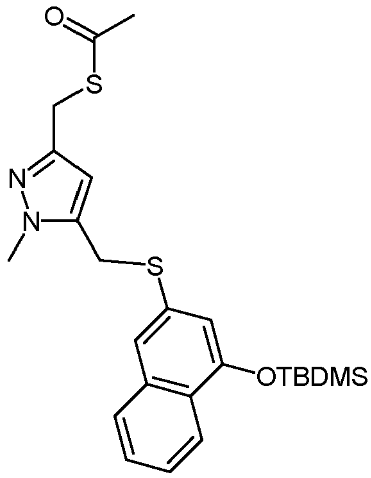

Potassium thioacetate (295 mg, 1.2 eq.) was added to a solution of Intermediate 10 (931 mg) in DMF (10 mL) under nitrogen atmosphere. The reaction was stirred at room temperature for 1 h. The reaction mixture was diluted with EtOAc (50 mL) and water (30 mL). The aqueous layer was separated and the organic one was washed with brine (2 × 30 mL). The combined aqueous layers were back-extracted with EtOAc (50 mL). The combined organic layers were dried over $MgSO_4$, filtered and evaporated. The crude mixture was purified by flash chromatography on silica gel (40 g, gradient: from heptane 100 % up to heptane/EtOAc 6/4) to give Intermediate 11 (900 mg, 88 % over 2 steps) as a colorless paste.

**Methyl 1-(3-((tert-butyldimethylsilyl)oxy)propyl)-5-chloro-4-(3-((((5-(((4-hydroxy-naphthalen-2-yl)thio)methyl)-1-methyl-1H-pyrazol-3-yl)methyl)thio)methyl)-1,5-dimethyl-1H-pyrazol-4-yl)-3-methyl-1H-indole-2-carboxylate (Intermediate 12)**

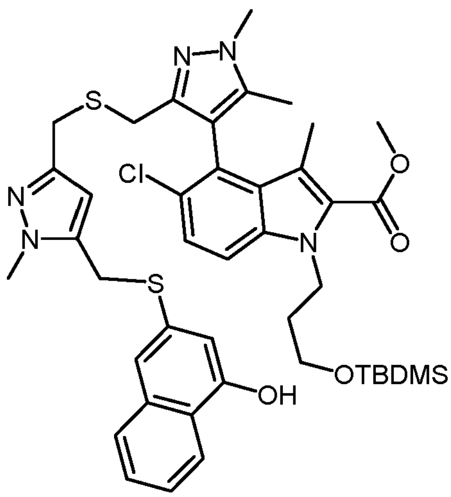

Potassium carbonate (170 mg, 2 eq.) was added to a solution of Intermediate 7 (387 mg, crude) and Intermediate 11 (407 mg, 1.4 eq.) in nitrogen-degassed MeOH (7 mL). The reaction mixture was stirred at room temperature for 2 h. Volatiles were removed under reduced pressure and the residue was partitioned between EtOAc (20 mL) and water (10 mL). The organic layer was separated and the aqueous one was extracted with EtOAc (10 mL). The combined organic layers were dried over $MgSO_4$, filtered and evaporated. The crude mixture was purified by flash chromatography on silica gel (12 g, gradient: from heptane 100 % up to EtOAc 100 %) to give two batches of Intermediate 12 (290 mg, contains impurities; and 135 mg pure).

**Methyl 5-chloro-4-(3-((((5-(((4-hydroxynaphthalen-2-yl)thio)methyl)-1-methyl-1H-pyrazol-3-yl)methyl)thio)methyl)-1,5-dimethyl-1H-pyrazol-4-yl)-1-(3-hydroxypropyl)-3-methyl-1H-indole-2-carboxylate (Intermediate 13)**

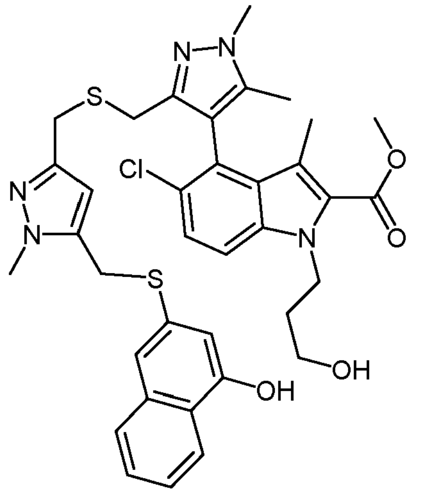

TBAF (1 M in THF, 0.247 mL, 1.5 eq.) was added dropwise to a solution of Intermediate 12 (135 mg) in THF (5 mL) under nitrogen atmosphere. The reaction was stirred at room temperature for 16 h. Volatiles were removed under reduced pressure. The residue was dissolved in EtOAc (20 mL), washed with water (10 mL) and brine (10 mL), dried over $MgSO_4$, filtered and evaporated. The crude mixture was purified by flash chromatography on silica gel (12 g, gradient: from DCM 100 % up to DCM/MeOH 95/5) to give Intermediate 13 (96 mg, 83 % yield) as a white foamy solid.

**Methyl $1^5$-chloro-$1^3$,$2^1$,$2^5$,$6^1$-tetramethyl-$1^1H$,$2^1H$,$6^1H$-10-oxa-4,8-dithia-1(4,1)-indola-2(4,3),6(3,5)-dipyrazola-9(3,1)-naphthalenacyclotridecaphane-$1^2$-carboxylate**

**(Intermediate 14: $S_a$ or $R_a$; one atropisomer but absolute stereochemistry undetermined)**

**(Intermediate 15: $R_a$ or $S_a$; one atropisomer but absolute stereochemistry undetermined)**

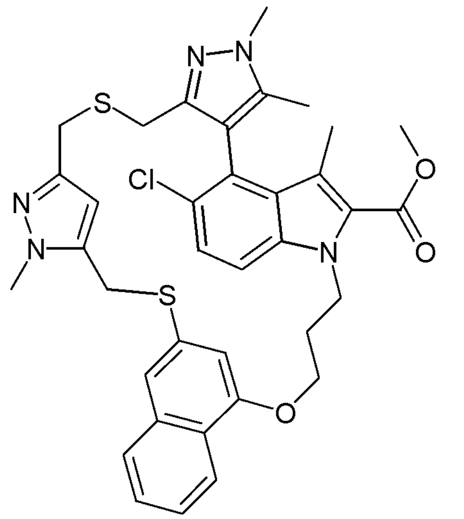

**Intermediate 14: $S_a$ or $R_a$; one atropisomer but absolute stereochemistry undetermined**
**Intermediate 15: $R_a$ or $S_a$; one atropisomer but absolute stereochemistry undetermined**

A solution of Intermediate 13 (100 mg) and di-tert-butyl azodicarboxylate (65 mg, 2 eq.) in toluene (6 mL) and THF (0.4 mL) was added with a syringe pump (0.075 ml/min) to a solution of triphenylphosphine (75 mg, 2 eq.) in toluene (4 mL). The reaction mixture was stirred at room temperature overnight. Volatiles were removed under reduced pressure. The residue was dissolved in EtOAc (40 mL) and washed with water (20 mL) and brine (20 mL). The organic layer was dried over $MgSO_4$, filtered and evaporated. The crude product was purified by flash chromatography on silica gel (40 g, gradient: from DCM 100 % up to DCM/MeOH 95/5). As the product was still contaminated with triphenylphosphine oxide and in order to separate atropisomers, it was purified by preparative SFC (Stationary phase: Chiralcel Diacel OJ 20 × 250 mm, Mobile phase: $CO_2$, EtOH + 0.4 % i-$PrNH_2$), yielding Intermediate 14 ($S_a$ or $R_a$ atropisomer, 27 mg, 28 %) and Intermediate 15 ($R_a$ or $S_a$ atropisomer, 29 mg, 30 %).

**Methyl 1-(3-((tert-butyldimethylsilyl)oxy)propyl)-5-chloro-4-(1,5-dimethyl-3-(((2-nitrophenyl)sulfonamido)methyl)-1H-pyrazol-4-yl)-3-methyl-1H-indole-2-carboxylate (Intermediate 16)**

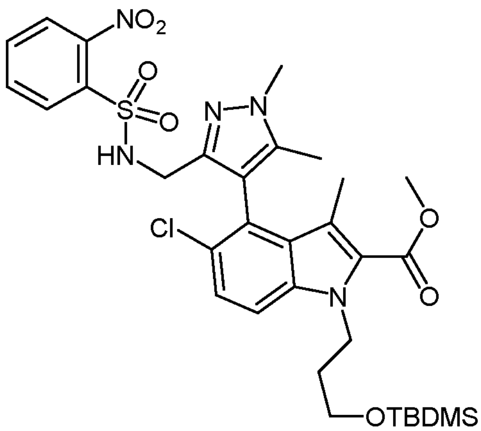

A solution of di-tert-butyl azodicarboxylate (78 mg, 2 eq.) in DCM (1 mL) was added dropwise to a suspension of Intermediate 5 (88 mg), 2-nitrobenzenesulfonamide (38 mg, 1.1 eq.), and triphenylphospine (89 mg, 2 eq.) in DCM (2.5 mL) stirring at room temperature under nitrogen atmosphere. After 15 min, the reaction mixture was directly loaded onto a silica gel column (12 g) and the product was purified eluting with a gradient from heptane 100 % up to heptane/EtOAc 1/1. Intermediate 16 (120 mg, quantitative) was obtained as a yellow solid.

**3-(((tert-butyldiphenylsilyl)oxy)methyl)-5-(chloromethyl)-1-methyl-1H-pyrazole (Intermediate 17)**

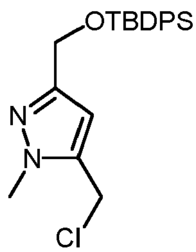

Thionyl chloride (270 μL, 1.2 eq.) was added dropwise to a solution of (3-(((tert-butyldiphenylsilyl)oxy)methyl)-1-methyl-1H-pyrazol-5-yl)methanol (1.18 g, 3.1 mmol) in DCM stirring at 0 °C under nitrogen atmosphere. The reaction was then allowed to warm up to room temperature and stirred for 45 min. The reaction mixture was cooled to 0 °C, diluted with DCM (30 mL) and quenched with saturated aqueous $NaHCO_3$ (20 mL). The organic layer was separated and the aqueous one was extracted with DCM (30 mL). The combined organic layer was dried over $MgSO_4$, filtered and evaporated to afford Intermediate 17 (1.17 g) as a colorless oil, used without further purification.

**3-(((3-(((tert-butyldiphenylsilyl)oxy)methyl)-1-methyl-1H-pyrazol-5-yl)methyl)-thio)naphthalen-1-ol (Intermediate 18)**

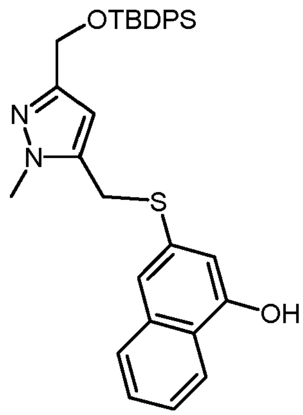

$K_2CO_3$ was added to a solution of Intermediate 17 (820 mg, 2.05 mmol) and 3-(acetylthio)naphthalen-1-yl acetate (588 mg, 2.26 mmol) in MeOH. The reaction mixture was stirred at room temperature for 1 h. The mixture was concentrated in vacuo. The residue was dissolved in EtOAc (40 mL) and water (25 mL). The organic layer was separated and washed with water (15 mL) and brine (15 mL). The combined aqueous layer was back-extracted with EtOAc (30 mL). The combined organic layer was dried over $MgSO_4$, filtered and evaporated. The residue was purified by flash chromatography on silica gel (24 g, gradient from heptane 100 % up to heptane/EtOAc 6/4). Yield: Intermediate 18 (825 mg, 74 % yield) as a pink solid.

**3-(((tert-butyldiphenylsilyl)oxy)methyl)-5-(((4-((4-methoxybenzyl)oxy)naphthalen-2-yl)thio)methyl)-1-methyl-1H-pyrazole (Intermediate 19)**

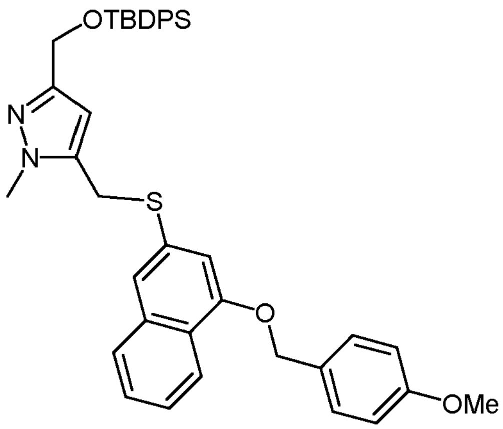

$K_2CO_3$ (423 mg, 2 eq.) was added portionwise to a solution of Intermediate 18 (825 mg, 1.53 mmol) and 4-methoxybenzyl chloride (260 μL, 1.25 eq.) in DMF (8 mL) under nitrogen atmosphere. The reaction was stirred at room temperature for 3 h. Then, more 4-methoxybenzyl chloride (207 μL, 1 eq.) was added and stirring was continued for 2 h at room temperature. The reaction was diluted with EtOAc (50 mL) and water (20 mL). The organic layer was separated and washed with brine (2 × 20 mL). The combined aqueous layer was back-extracted with EtOAc (30 mL). The combined organic layer was dried over $MgSO_4$, filtered and evaporated. The residue was purified by flash column chromatography on silica gel (40 g, gradient: from heptane 100 % up to heptane/EtOAc 6/4). Intermediate 19 (950 mg) was obtained as a brownish paste, used without further purification.

**5-(((4-((4-methoxybenzyl)oxy)naphthalen-2-yl)thio)methyl)-1-methyl-1H-pyrazol-3-yl)methanol (Intermediate 20)**

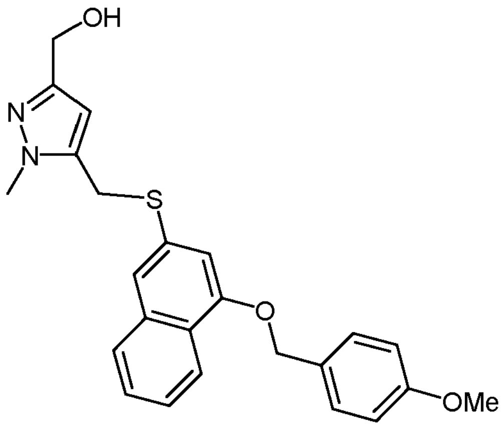

TBAF (2.16 mL, 1.5 eq., 1 M in THF) was added dropwise to a solution of Intermediate 19 (950 mg) in dry THF under nitrogen atmosphere. The reaction was stirred at room temperature for 3 h. The reaction mixture was concentrated under reduced pressure. The residue was partitioned between EtOAc (60 mL) and saturated aqueous $NH_4Cl$ (30 mL). The organic layer was separated and the aqueous one was extracted with EtOAc (30 mL). The combined organic layer was dried over $MgSO_4$, filtered and evaporated. The residue was purified by flash chromatography on silica gel (40 g, gradient from heptane 100 % up to EtOAc 100 %). A second purification by flash chromatography was performed on silica gel (120 g, gradient: from DCM 100% up to DCM/MeOH 96/4) to afford Intermediate 20 (362 mg, 60 %) as a white solid.

**Methyl 1-(3-((tert-butyldimethylsilyl)oxy)propyl)-5-chloro-4-(3-(((N-((5-(((4-((4-methoxybenzyl)oxy)naphthalen-2-yl)thio)methyl)-1-methyl-1H-pyrazol-3-yl)methyl)-2-nitrophenyl)sulfonamido)methyl)-1,5-dimethyl-1H-pyrazol-4-yl)-3-methyl-1H-indole-2-carboxylate (Intermediate 21)**

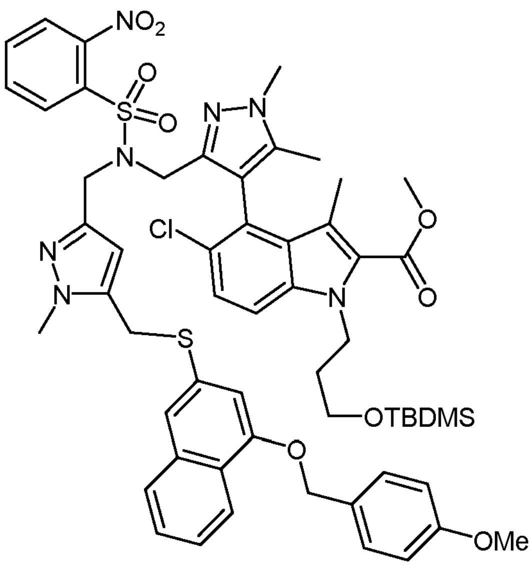

A solution of di-tert-butyl azodicarboxylate (81 mg, 1.5 eq.) in DCM (2 mL) was added dropwise over a period of 15 minutes to a suspension of Intermediate 16 (165 mg), Intermediate 20 (108 mg, 1.1 eq.), and triphenylphosphine (92 mg, 1.5 eq.) in DCM (2 mL) stirring at room temperature under nitrogen atmosphere. After 15 minutes, additional triphenylphospine (18 mg, 0.3 eq.) and di-tert-butyl azodicarboxylate (16 mg, 0.3 eq) in DCM (0.5 mL) were added

dropwise. The solvent was evaporated and the residue was purified by flash chromatography on silica gel (40 g, gradient from heptane 100 % up to heptane/EtOAc 2/8), affording Intermediate 21. After combination with another batch, in total 480 mg of intermediate 21 was obtained, used as such in the next reaction step.

**Methyl 5-chloro-1-(3-hydroxypropyl)-4-(3-(((N-((5-(((4-((4-methoxybenzyl)oxy)naphthalen-2-yl)thio)methyl)-1-methyl-1H-pyrazol-3-yl)-methyl)-2-nitrophenyl)sulfonamido)methyl)-1,5-dimethyl-1H-pyrazol-4-yl)-3-methyl-1H-indole-2-carboxylate (Intermediate 22)**

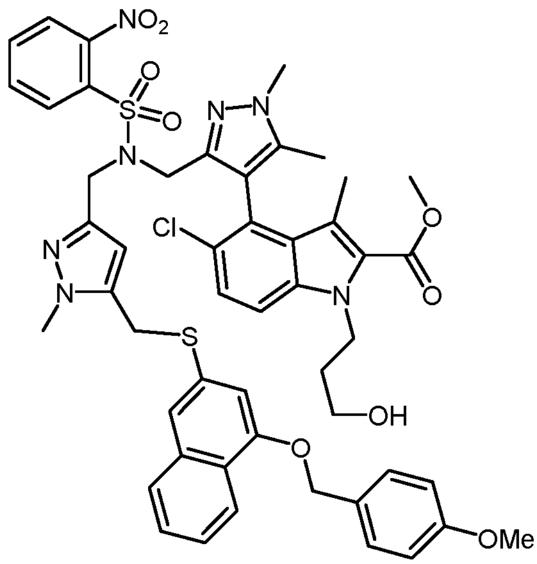

TBAF (452 μL, 1.1 eq., 1 M in THF) was added dropwise to a solution of Intermediate 21 (455 mg) in dry THF at room temperature under nitrogen atmosphere. After stirring for 15 min, the reaction mixture was concentrated under reduced pressure. The residue was dissolved in EtOAc (50 mL) and washed with water (25 mL) and brine (25 mL). The organic layer was dried over $MgsO_4$, filtered and evaporated. The residue was purified by flash column chromatography on silica gel (24 g, gradient from DCM 100 % up to DCM/MeOH 95/5), affording Intermediate 22 (418 mg, quantitative) as a foamy solid.

**Methyl 5-chloro-4-(3-(((N-((5-(((4-hydroxynaphthalen-2-yl)thio)methyl)-1-methyl-1H-pyrazol-3-yl)methyl)-2-nitrophenyl)sulfonamido)methyl)-1,5-dimethyl-1H-pyrazol-4-yl)-1-(3-hydroxypropyl)-3-methyl-1H-indole-2-carboxylate (Intermediate 23)**

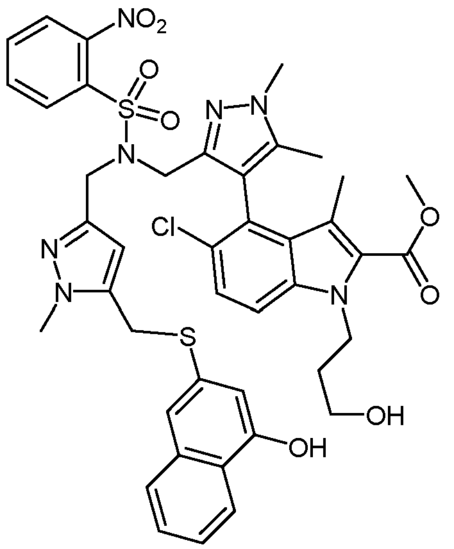

Trifluoromethanesulfonic acid (156 μL, 5 eq.) was added dropwise to a solution of Intermediate 22 (350 mg) in anisole (10 mL), stirring at 0 °C. The reaction was stirred for 15 min at 0 °C. The reaction mixture was diluted with DCM (30 mL) and treated with saturated aqueous $NaHCO_3$ (20 mL). The organic layer was separated and the aqueous one was extracted with DCM (10 mL). The combined organic layer was dried over $MgSO_4$, filtered and evaporated. The residue was purified by flash column chromatography on silica gel (40 g, gradient from DCM 100 % up to DCM/MeOH 95/5) to afford Intermediate 23 (175 mg) as a foamy white solid, .

**Methyl 15-chloro-13,21,25,61-tetramethyl-4-((2-nitrophenyl)sulfonyl)-11H,21H,61H-10-oxa-8-thia-4-aza-1(4,1)-indola-2(4,3),6(3,5)-dipyrazola-9(3,1)-naphthalenacyclotridecaphane-12-carboxylate (Intermediate 24)**

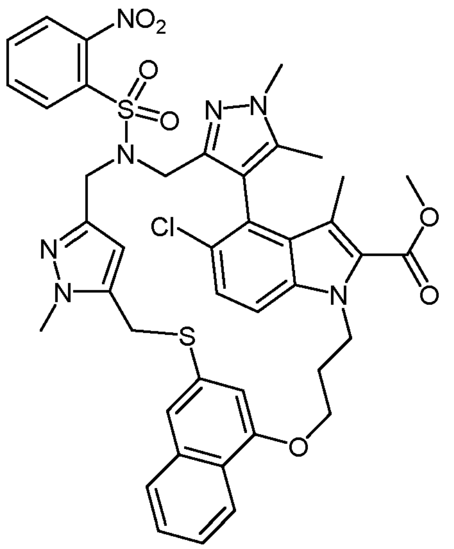

A solution of Intermediate 23 and di-tert-butyl azodicarboxylate (185 mg, 4 eq.) in toluene (6 mL) and THF (0.6 mL) was added with a syringe pump (0.1 mL/min) to a solution of triphenylphospine (210 mg, 4 eq.) in toluene (6 mL), stirring at 70 °C. Once the addition was complete, the reaction mixture was concentrated under reduced pressure and the residue was purified by flash column chromatography on silica gel (40 g, gradient from DCM 100 % up to DCM/MeOH 98/2), affording Intermediate 24 (95 mg, 55 %).

**Methyl 15-chloro-13,21,25,61-tetramethyl-11H,21H,61H-10-oxa-8-thia-4-aza-1(4,1)-indola-2(4,3),6(3,5)-dipyrazola-9(3,1)-naphthalenacyclotridecaphane-12-carboxylate (Intermediate 25)**

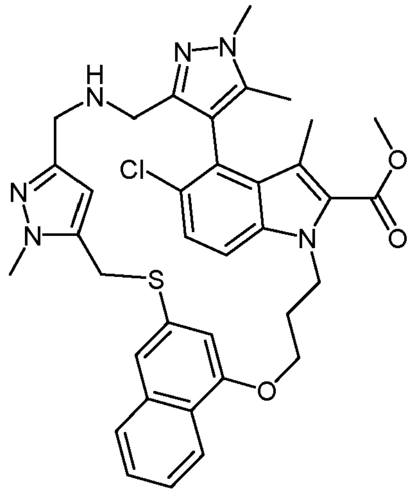

Thiophenol (57 µL, 5 eq.) was added dropwise to a suspension of Intermediate 24 and $K_2CO_3$ (77 mg, 5 eq.) in acetonitrile under nitrogen atmosphere. The reaction was stirred at room temperature overnight. More thiophenol (57 µL, 5 eq.) and $K_2CO_3$ (77 mg, 5 eq.) were added to the reaction mixture and it was stirred at room temperature for 30 minutes. After further addition of thiophenol (28 µL, 2.5 eq.) and $K_2CO_3$ (39 mg, 2.5 eq.), the reaction mixture was stirred for 30 minutes at room temperature. The reaction mixture was diluted with DCM (20 mL) and Celite® was added. The mixture was filtered over Celite® and the filtrate was concentrated under reduced pressure. The residue was purified by flash column chromatography on silica gel (24 g, gradient from DCM 100 % up to DCM/MeOH 90/10), affording Intermediate 25 (48 mg, 64 %).

**Methyl 15-chloro-13,21,25,61,4-pentamethyl-11H,21H,61H-10-oxa-8-thia-4-aza-1(4,1)-indola-2(4,3),6(3,5)-dipyrazola-9(3,1)-naphthalenacyclotridecaphane-12-carboxylate**

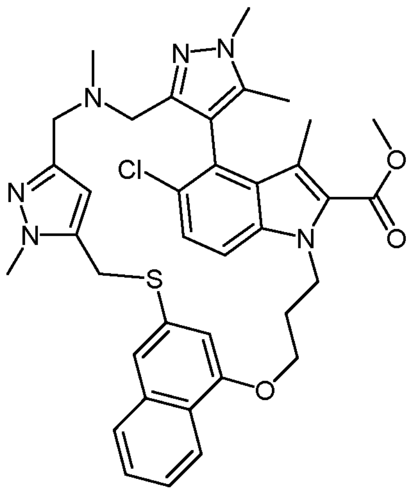

Intermediate 26 - mixture of atropisomers
Intermediate 27 (Ra or Sa, one atropisomer but absolute stereochemistry undetermined)
Intermediate 28 (Sa or Ra, one atropisomer but absolute stereochemistry undetermined)

Formaldehyde (16 µL, 3 eq., 37 % in water) was added to a solution of Intermediate 25 (48 mg) and acetic

acid (12 µL, 3 eq.) in DCM (0.7 mL) at room temperature. Then, sodium triacetoxyborohydride (45 mg, 3 eq.) was added and the reaction mixture was stirred at room temperature for 1 h. The reaction was quenched with saturated aqueous $NaHCO_3$ (2.5 mL) and diluted with water (2.5 mL) and DCM (10 mL). The organic layer was separated and the aqueous one was extracted with DCM (2 × 10 mL). The combined organic layer was dried over $MgSO_4$, filtered and evaporated. The residue was purified by flash column chromatography on silica gel (24 g, gradient from DCM 100 % up to DCM/MeOH 93/7), affording Intermediate 26 (mixture of atropisomers) as a white solid (30 mg, 61% yield).

Another batch of Intermediate 26 was prepared in the same way, and was subsequently separated into its atropisomers by preparative SFC (Stationary phase: Chiralpak Daicel IG 20 × 250 mm, Mobile phase: $CO_2$, iPrOH + 0.4 % i-$PrNH_2$) to afford Intermediate 27 ($R_a$ or $S_a$ atropisomer) and Intermediate 28 ($S_a$ or $R_a$ atropisomer).

## Intermediate 29

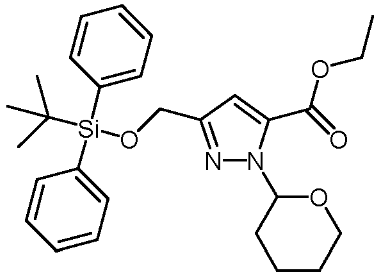

Intermediate 29

3-(hydroxymethyl)-1-(tetrahydro-2H-pyran-2-yl)-1H-Pyrazole-5-carboxylic acid ethyl ester [847139-28-0] (5.2 g, 20.45 mmol) was dissolved in dry DMF (60 mL) under nitrogen atmosphere. Imidazole (2.088 g, 1.5 eq.) and DMAP (0.25 g, 0.1 eq.) were added. TBDPSCl (6.91 mL, 1.3 eq.) was added slowly and the reaction mixture was stirred at room temperature overnight. The reaction mixture was diluted with EtOAc (250 mL) and water (200 mL). The organic layer was separated and washed with brine (3 × 100 mL). The combined aqueous layer was extracted with EtOAc (150 mL). The combined organic layer was dried over $MgSO_4$, filtered, and evaporated. The crude product was purified by flash chromatography on silica gel (120 g, gradient: from heptane 100 % to heptane/EtOAc 8/2) to afford Intermediate 29 (11.56 g, yield: 97 %) as a colorless paste.

## Intermediate 30

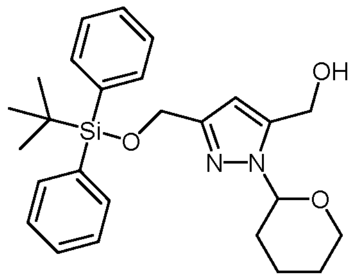

Intermediate 30

$LiAlH_4$ (2 M in THF, 10. 97 mL, 1.1 eq.) was added dropwise to a solution of Intermediate 29 (9.826 g, 19.94 mmol) in THF (80 mL) stirring at 0 °C under nitrogen atmosphere. After 15 min, the reaction mixture was treated with wet THF (25 mL), then with water (5 mL, added dropwise) and then was allowed to warm up to room temperature. Celite was added, followed by $MgSO_4$ and EtOAc. After 5 min stirring, the suspension was filtered, washing with EtOAc. The filtrate was concentrated under reduced pressure. The crude product was purified by flash chromatography on silica gel (120 g, gradient: from heptane 100 % to heptane/EtOAc 1/1) to give Intermediate 30 (8.54 g, yield: 95 %) as a colorless paste.

## Intermediate 31

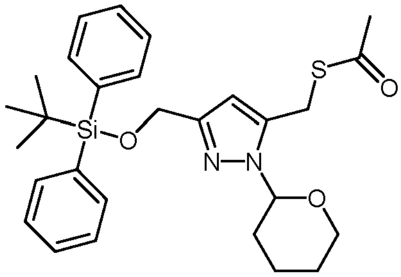

Intermediate 31

MsCl (1.84 mL, 1.25 eq.) was added dropwise to a solution of Intermediate 30 (8.54 g, 18.95 mmol) and $Et_3N$ (3.95 mL, 1.5 eq.) in THF (85 mL) stirring at 0 °C under nitrogen atmosphere. The reaction mixture was then allowed to warm up to room temperature and was stirred at room temperature for 1 h. A solution of KSAc (3.246 g, 1.5 eq.) in DMF (85 mL) was added and stirring was continued at room temperature for 3 h. The reaction mixture was diluted with EtOAc (250 mL) and water (200 mL). The aqueous layer was separated and the organic one was washed with brine (3 × 150 mL). The combined aqueous layers were back-extracted with EtOAc (200 mL). The combined organic layer was dried over $MgSO_4$, filtered, and evaporated. The crude product was purified by flash chromatography on silica gel (120 g, gradient: from heptane 100 % to heptane/EtOAc 8/2) to afford Intermediate 31 (8.78 g, 91 %) as a yellow paste.

## Intermediate 32

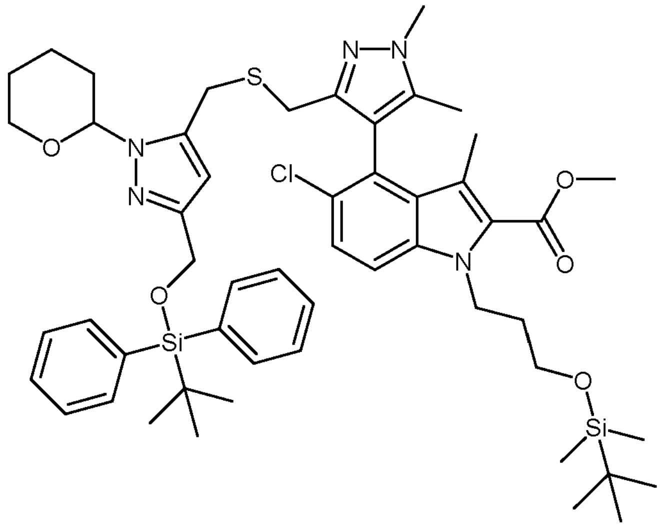

Intermediate 32

A solution of Intermediate 7 (5.34 g, 1.2 eq.) and Intermediate 31 (3.593 g, 7.06 mmol) in dry MeOH (86 mL) was placed in a round-bottom flask. The solution was degassed and re-filled with nitrogen three times. The reaction mixture was then cooled to 0 °C and $K_2CO_3$ (1.952 g, 2 eq.) was added. The resulting mixture was degassed and re-filled with nitrogen twice, then was allowed to warm to room temperature and was stirred for 3 h under nitrogen flow. The reaction mixture was concentrated in vacuo. EtOAc and water were added to the resulting residue. The layers were separated and the aqueous layer was extracted twice with EtOAc. The combined organic layer was washed with brine, dried over $MgSO_4$, filtered, and evaporated to afford Intermediate 32 (7.47 g, 98 %) as an orange sticky foam, used without further purification.

## Intermediate 33

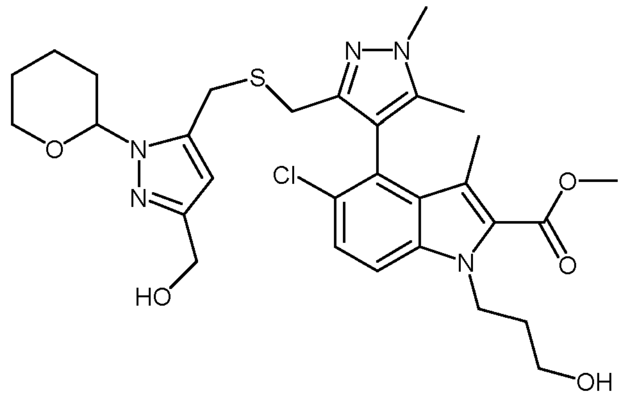

Intermediate 33

TBAF (1 M in THF, 11.1 mL, 1.6 eq.) was added to a stirred solution of Intermediate 32 (7.47 g, 6.94 mmol) in dry THF (130 mL) under nitrogen atmosphere. The reaction mixture was stirred at room temperature for 2.5 h before additional TBAF (1 M in THF, 8 mL, 1.15 eq.) was added. After stirring for 1 h, the solvent was evaporated. The residue was dissolved in EtOAc and washed with water followed by brine, dried over $MgSO_4$, filtered, and evaporated. The residue was purified by flash column chromatography (silica; MeOH in DCM 0/100 to 5/95) to afford Intermediate 33 (3.51 g, yield: 82 %) as a light yellow solid.

## Intermediate 34

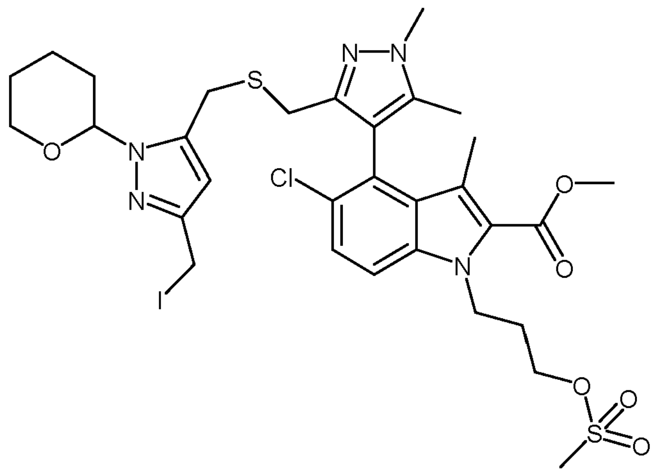

Intermediate 34

DIPEA (282 μL, 4 eq.) followed by $Ms_2O$ (283 mg, 4 eq.) was added to a solution of Intermediate 33 (250 mg, 0.406 mmol) in THF (14 mL), cooled to 0 °C. Once the addition was complete, the reaction mixture was stirred at room temperature for 1 h. The reaction mixture was then cooled to 0 °C before addition of NaI (304 mg, 5 eq.). After 30 min, the reaction was allowed to warm to room temperature and was stirred at this temperature for 4 h. The reaction mixture was diluted with EtOAc (30 mL) and washed with saturated aqueous sodium thiosulfate (25 mL), saturated $NH_4Cl$ (25 mL) and brine (25 mL), dried over $MgSO_4$, filtered, and concentrated under reduced pressure (bath temperature: 30 °C, to prevent decomposition) to give Intermediate 34 (380 mg, yield: 66 %) as a colourless oil, used without further purification.

**Intermediate 35**

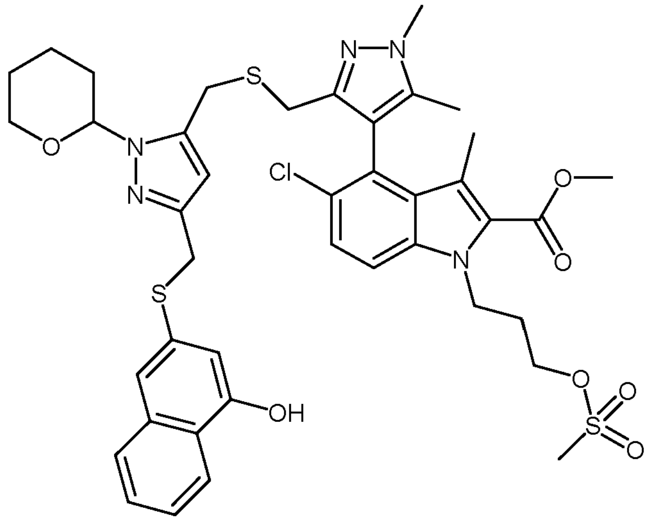

Intermediate 35

Intermediate 34 (380 mg, 0.378 mmol) was dissolved in a mixture of MeOH (17 mL) and THF (5 mL). The reaction mixture was degassed and re-filled with nitrogen twice. Ethanethioic acid, S-[4-(acetyloxy)-2-naphthalenyl] ester [2143010-96-0] (98 mg, 1 eq.) and $PPh_3$ (10 mg, 0.1 eq.) were added to the mixture which was degassed and re-filled with nitrogen twice. Once all the reagents were in solution, the reaction mixture was cooled to 0 °C before addition of $K_2CO_3$ (130 mg, 2.5 eq.). The reaction mixture was then degassed and re-filled with nitrogen twice. The reaction mixture was stirred at 0 °C for 1 h. The reaction mixture was diluted with DCM (30 mL) and water (20 mL). The layers were separated and the aqueous layer was extracted with DCM (30 mL). The combined organic layer was washed with saturated aqueous $NH_4Cl$ (20 mL), dried over $MgSO_4$, filtered, and concentrated under reduced pressure. The crude product was purified by flash column chromatography on silica gel (heptane:EtOAc - 3:1 to 1:3) to give Intermediate 35 (160 mg, yield: 44 %) as a yellow foam.

**Intermediate 36**

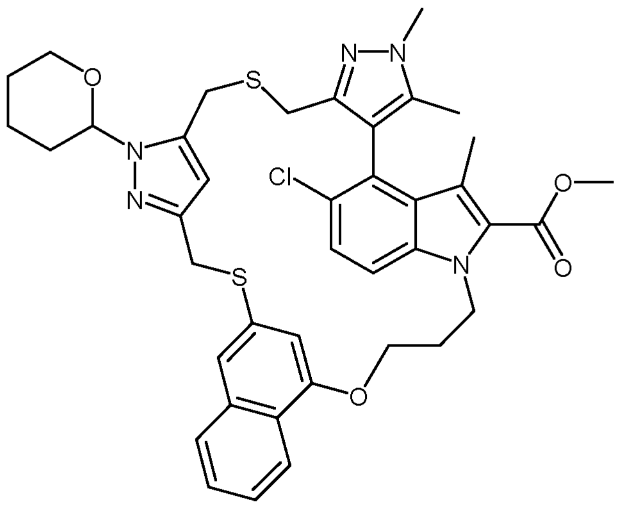

Intermediate 36

Intermediate 35 (156 mg, 0.164 mmol) was dissolved in ACN (3 mL). The resulting solution was added via syringe pump (0.02 mL/min) to a solution of $K_2CO_3$ (45 mg, 2 eq.) in ACN (3 mL) at 82 °C. After the addition was complete, the reaction mixture was concentrated under reduced pressure to give a yellow oil. This oil was purified by flash column chromatography on silica gel (heptane:EtOAc - 1:0 to 1:1) to give Intermediate 36 (107 mg, yield: 82 %) as a clear oil which solidified on standing.

## Intermediate 37 and Intermediate 38

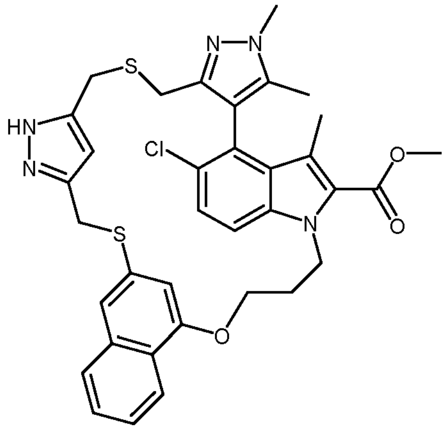

Intermediate 37: $S_a$ or $R_a$; one atropisomer but absolute stereochemistry undetermined
Intermediate 38: $R_a$ or $S_a$; one atropisomer but absolute stereochemistry undetermined

HCl in MeOH (1.25 M, 5 mL, 50 eq.) was added to a solution of Intermediate 36 (100 mg, 0.126 mmol) in THF (5 mL). The reaction mixture was stirred at room temperature for 4 h. The reaction mixture was concentrated under reduced pressure to give a white solid. The white solid was purified by preparative SFC (Stationary phase: Chiralpak Diacel AD 20 × 250 mm, Mobile phase: $CO_2$, EtOH + 0.4 % i-PrNH$_2$) to afford Intermediate 37 (35 mg, yield: 39 %) and Intermediate 38 (34 mg, yield: 38 %) as clear oils which solidified on standing.

## Intermediate 39

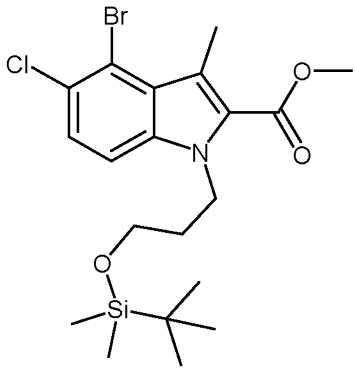

Intermediate 39

A mixture of Intermediate 2 (37.4 g, 123.6 mmol), (3-bromopropoxy)-tert-butyldimethylsilane [89031-84-5] (37.56 g, 1.2 eq.), and $K_2CO_3$ (51.25 g, 3 eq.) in ACN (300 mL) was stirred at 80 °C overnight. The reaction mixture was cooled to room temperature and was filtered. The filter cake was washed with EtOAc (100 mL). The filtrate was concentrated and the residue was purified by column chromatography over silica gel (eluent: petroleum ether/EtOAc from 100/0 to 10/90) to afford Intermediate 39 (42 g, 71 %) as a red gum.

## Intermediate 40

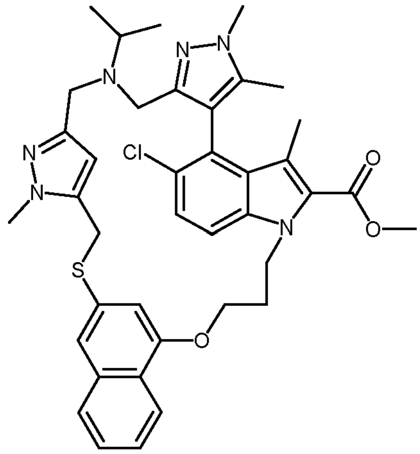

Intermediate 40

Acetone (37 µL, 4 eq.) and AcOH (14 µL, 2 eq.) were added to a solution of Intermediate 25 (83 mg, 0.124 mmol) in DCE (2 mL) in a sealed tube. The reaction mixture was stirred at room temperature for 20 min. $NaBH(OAc)_3$ (53 mg, 2 eq.) was added and the reaction mixture was stirred at room temperature for 16 h. The reaction mixture was diluted with DCM and washed with a saturated aqueous solution of $Na_2CO_3$. The organic layer was dried over $MgSO_4$, filtered, and evaporated to yield Intermediate 40 (61 mg, yield: 69 %) as an oil.

## Intermediate 41 and Intermediate 42

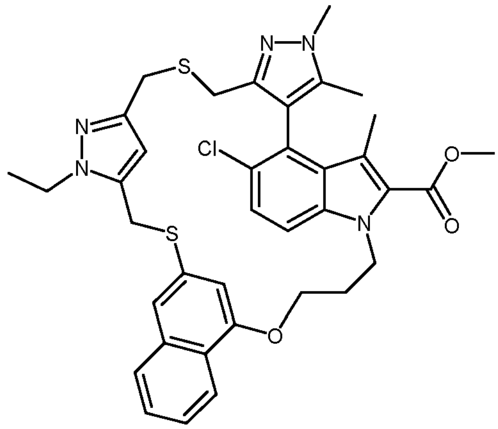

Intermediate 41: $R_a$ or $S_a$; one atropisomer but absolute stereochemistry undetermined

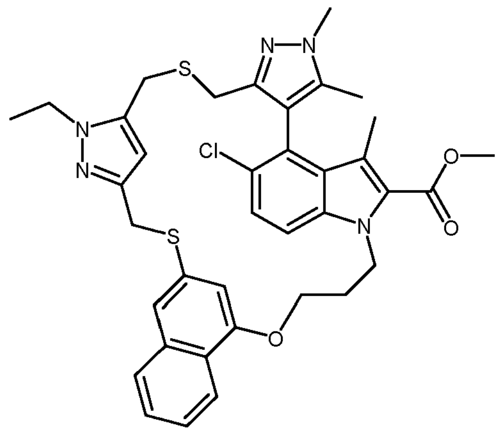

Intermediate 42: Ra or Sa; one atropisomer but absolute stereochemistry undetermined

Iodoethane (40 µL, 2.51 eq.) was added to a suspension of Intermediate 38 (140 mg, 0.197 mmol) and $Cs_2CO_3$ (193.3 mg, 3 eq.) in DMF (3 mL) in a sealed tube, under nitrogen atmosphere. The reaction mixture was stirred at room temperature for 4.5 h. Water was added (10 mL) and the mixture was extracted with EtOAc (20 mL). The layers were separated and the aqueous layer was extracted with more EtOAc (10 mL). The combined organic layer was washed with brine, dried over $MgSO_4$, filtered, and evaporated. The residue was purified by flash column chromatography on silica gel (DCM: MeOH - 100/0 to 95/5). The mixture obtained was further purified by preparative SFC (Stationary phase: Chiralcel Diacel OJ 20 × 250 mm, Mobile phase: $CO_2$, EtOH + 0.4 % i-$PrNH_2$) to afford Intermediate 41 (49.1 mg, yield: 35 %) and Intermediate 42 (62 mg, yield: 45 %).

## Intermediate 43 and Intermediate 44

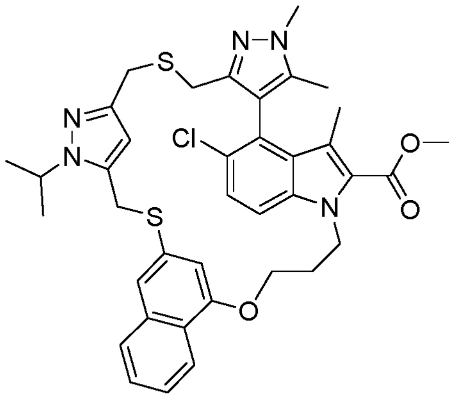

Intermediate 43: $R_a$ or $S_a$; one atropisomer but absolute stereochemistry undetermined

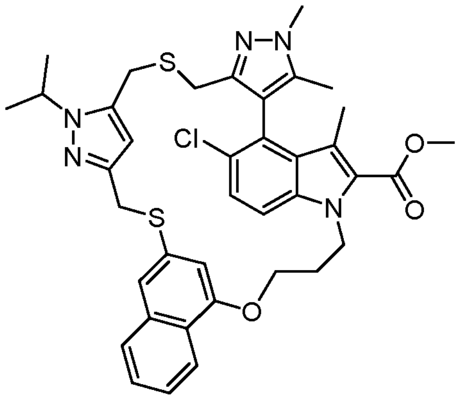

Intermediate 44: $R_a$ or $S_a$; one atropisomer but absolute stereochemistry undetermined

2-Bromopropane (67 μL, 2.63 eq.) was added to a suspension of Intermediate 38 (200 mg, 0.28 mmol) and $Cs_2CO_3$ (278.7 mg, 3 eq.) in DMF (4.4 mL) in a sealed tube, under nitrogen atmosphere. The reaction mixture was stirred at room temperature for 16 h. Water was added (15 mL) and the mixture was extracted with EtOAc (30 mL). The layers were separated and the aqueous layer was extracted with more EtOAc (15 mL). The combined organic layer was washed with brine, dried over $MgSO_4$, filtered, and evaporated. The residue was purified by flash column chromatography on silica gel (DCM: MeOH - 100/0 to 95/5) to afford Intermediate 43 (93 mg, yield: 46 %) and Intermediate 44 (116 mg, yield: 58 %), both as colorless oils.

## Intermediate 45

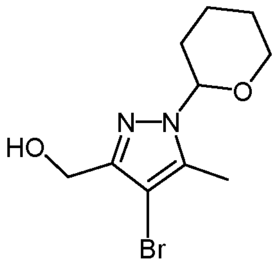

Lithium borohydride (32.2 g, 4 eq.) was added slowly to a solution of 1H-pyrazole-3-carboxylic acid, 4-bromo-5-methyl-1-(tetrahydro-2H-pyran-2-yl)-, ethyl ester [2246368-58-9] (130 g, 369.7 mmol) in 2-Me-THF (1 L) at 0 °C. The reaction mixture was allowed to warm to room temperature and was left stirring at room temperature overnight. The reaction was quenched by addition of water (800 mL). The mixture was extracted with EtOAc (800 mL × 2). The combined organic layer was washed with brine (500 mL), dried with $Na_2SO_4$, filtered, and evaporated to afford Intermediate 45 (105 g, yield: 94 %) as a white solid.

## Intermediate 46

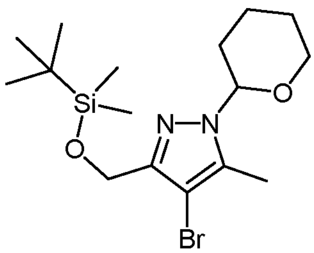

DMAP (16.28 g, 0.4 eq.) and $Et_3N$ (92.38 mL, 2 eq.) were added to a solution of Intermediate 45 (100 g, 333.2

mmol) in THF (1 L). TBDMSCl (75.3 g, 1.5 eq.) was added at room temperature and the reaction mixture was stirred for 16 h. The reaction was quenched by addition of saturated aqueous $NaHCO_3$ (800 mL) and the mixture was extracted with EtOAc (1 L × 2). The combined organic layer was washed with brine (800 mL), dried with $Na_2SO_4$, filtered, and evaporated. The residue was purified by column chromatography over silica gel (petroleum ether/ EtOAc 100/0 to 30/70) to afford Intermediate 46 (130 g, yield: 94 %) as a colorless oil.

## Intermediate 47

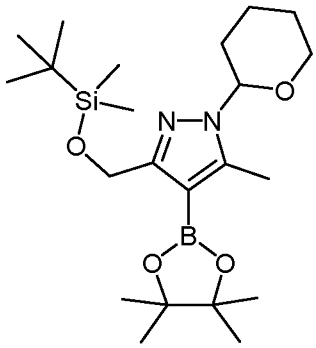

BuLi (104.55 mL, 1 eq.) was slowly added to a solution of Intermediate 46 (108 g, 261.4 mmol) in THF (1 L) at -78 °C, under nitrogen atmosphere, and the reaction mixture was stirred at -78 °C for 1 h. Then, 2-isopropoxy-4,4,5,5-tetramethyl-1,3,2-dioxaborolane (97.2 g, 2 eq.) was added slowly and the reaction mixture was stirred at room temperature for 2 h. Saturated aqueous $NH_4Cl$ (800 mL) was added slowly to quench the reaction. The mixture was extracted with EtOAc (1 L × 2). The combined organic layer was washed with brine (800 mL), dried with $Na_2SO_4$, filtered, and evaporated to afford Intermediate 47 (140 g, assumed quantitative) as a yellow oil.

## Intermediate 48

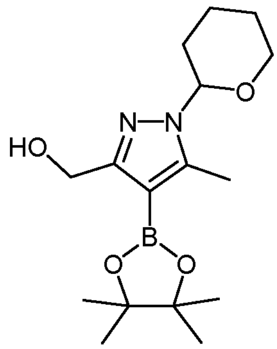

TBAF (1 M in THF, 192.4 mL, 1.2 eq.) was added dropwise to a solution of Intermediate 47 (70 g, 160 mmol) in DCM (700 mL) at room temperature under nitrogen atmosphere. The reaction mixture was stirred overnight at room temperature. The reaction mixture was added to a stirring solution of saturated aqueous $NaHCO_3$ (500 mL) and this mixture was extracted with EtOAc (700 mL × 2). The combined organic layer was washed with brine (500 mL), dried with $Na_2SO_4$, filtered, and evaporated. The residue was purified by column chromatography over silica gel (petroleum ether/ EtOAc 100/0 to 50/50) to afford Intermediate 48 (35 g, yield: 62 %) as a white solid.

## Intermediate 49

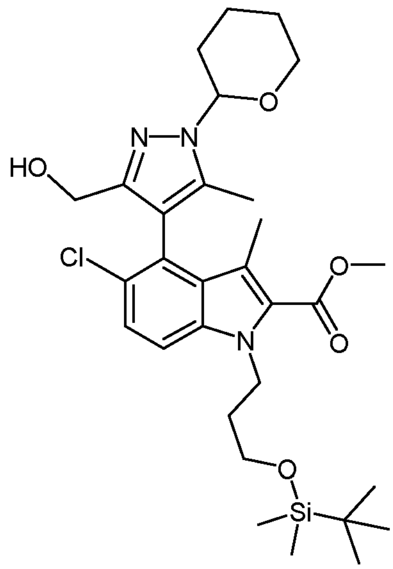

$K_2CO_3$ (6.9 g, 2 eq.) was added to a solution of Intermediate 39 (12 g, 24.9 mmol) and Intermediate 48 (9.6 g, 1.2 eq.) in water (40 mL) and dioxane (200 mL). Pd(amphos)$Cl_2$ [887919-35-9] (0.8 g, 0.05 eq.) was added under nitrogen atmosphere and the reaction mixture was stirred at 60 °C for 2 h. Water (40 mL) was added to the mixture and it was extracted with EtOAc (60 mL × 2). The combined organic layer was washed with brine, dried with $Na_2SO_4$, filtered, and evaporated. The residue was purified by flash column chromatography over silica gel (petroleum ether/EtOAc 100/0 to 60/40) to afford Intermediate 49 (15 g, yield: 99 %) as a yellow solid.

## Intermediate 50

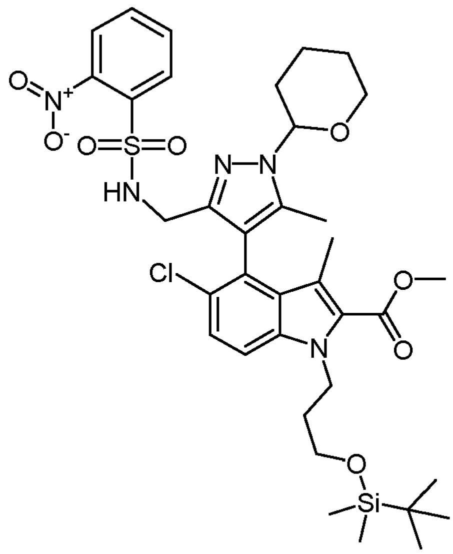

A solution of Intermediate 49 (23 g, 37.8 mmol) and 2-nitrobenzenesulfonamide (11.48 g, 1.5 eq.) was prepared in DCM (150 mL) under nitrogen atmosphere and cooled to 0 °C. To this solution, DTBAD (13.1 g, 1.5 eq.) and $PPh_3$ (14.9 g, 1.5 eq.) were added. The reaction mixture was stirred at room temperature for 3 h. The solvent was evaporated and the residue was purified by flash column chromatography over silica gel (petroleum ether/EtOAc 100/0 to 50/50) to afford Intermediate 50 (24.6 g, yield: 83 %) as a yellow liquid.

## Intermediate 51

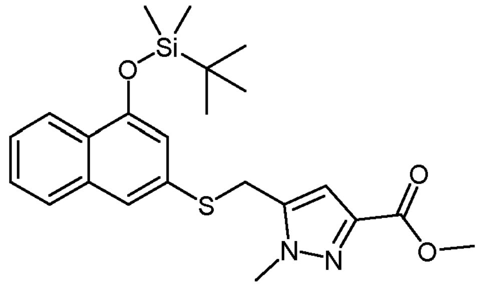

Imidazole (6.64 g, 3 eq.) was added to a solution of 5-[[(4-hydroxy-2-naphthalenyl)thio]methyl]-1-methyl-1H-pyrazole-3-carboxylic acid methyl ester [2245716-34-9] (11 g, 32.5 mmol) and TBDMSCl (9.8 g, 2 eq.) in DCM (75 mL). The reaction mixture was stirred at room temperature for 16 h. The reaction was quenched by addition of saturated aqueous $NH_4Cl$ (20 mL) and the mixture was extracted with DCM (30 mL × 2). The combined organic layer was dried with $Na_2SO_4$, filtered, and evaporated. The crude product was purified by flash column chromatography over silica gel (petroleum ether/EtOAc 100/0 to 30/70) to afford Intermediate 51 (14 g, yield: 97 %) as a white solid.

## Intermediate 52

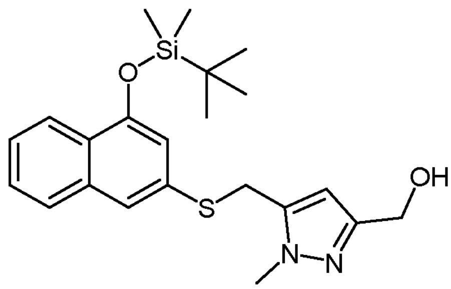

DIBAL-H (1 M solution in toluene, 28.5 mL, 1.8 eq.) was added slowly to a solution of Intermediate 51 (7 g, 15.8 mmol) in THF (100 mL). The reaction mixture was stirred at room temperature for 2 h. The reaction was quenched by addition of water (10 mL) at 0 °C, followed by 10 % aqueous NaOH (10 mL). $MgSO_4$ (30 g) was added and the mixture was stirred at room temperature for 15 min. The solid was filtered and was washed with EtOAc (100 mL). The filtrate was evaporated. The residue was purified by flash column chromatography over silica gel (petroleum ether/EtOAc 100/0 to 20/80) to afford Intermediate 52 (5.8 g, yield: 88 %) as a white solid.

## Intermediate 53

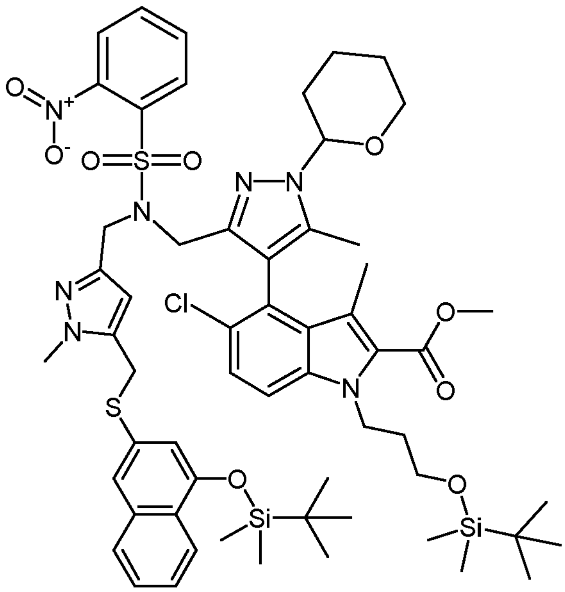

A solution of Intermediate 50 (24.6 g, 31.5 mmol) and Intermediate 52 (15.6 g, 1.2 eq.) in DCM (250 mL) was prepared under nitrogen atmosphere and cooled down at 0 °C.

Then, DTBAD (10.8 g, 1.5 eq.) and $PPh_3$ (12.4 g, 1.5 eq.) were added and the reaction mixture was stirred at room temperature for 16 h. The solvent was evaporated and the residue was purified by flash column chromatography over silica gel (petroleum ether/EtOAc 100/0 to 60/40) to afford Intermediate 53 (32 g, yield: 76 %) as a yellow liquid.

## Intermediate 54

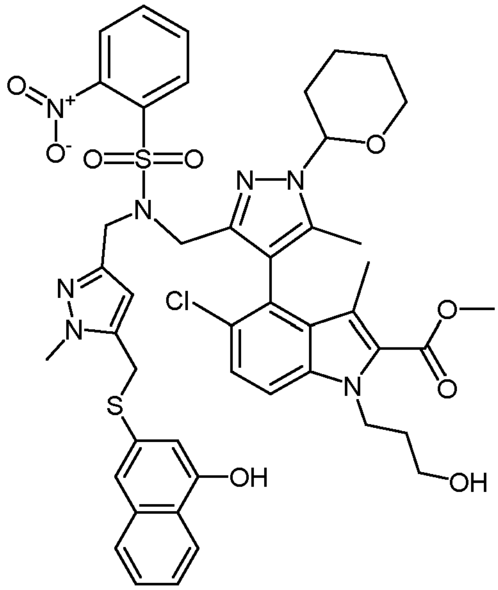

TBAF (1 M in THF, 27.1 mL, 3 eq.) was added to a solution of Intermediate 53 (12 g, 9 mmol) in THF (80 mL). The reaction mixture was stirred at room temperature for 16 h. EtOAc (100 mL) was added and the mixture was washed with water (50 mL × 2). The organic layer was washed with brine (50 mL), dried with $Na_2SO_4$, filtered, and evaporated. The residue was purified by flash column chromatography over silica gel (petroleum ether/EtOAc 100/0 to 0/100) to afford Intermediate 54 (11.7 g, yield: 87 %) as a yellow liquid.

## Intermediate 55

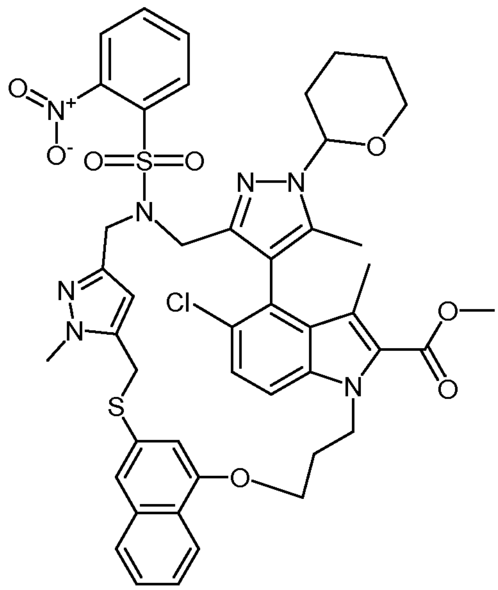

DTBAD (0.9 g, 3 eq.) was added to a solution of Intermediate 54 (2 g, 1.35 mmol) and $PPh_3$ (1 g, 3 eq.) in DCM (80 mL) under nitrogen atmosphere at 0 °C. The reaction mixture was stirred at room temperature for 16 h. The solvent was evaporated and the residue was purified by flash column chromatography over silica gel (petroleum ether/EtOAc 100/0 to 20/80) to afford Intermediate 55 (2 g, yield: 66 %) as a yellow liquid.

**Intermediate 56**

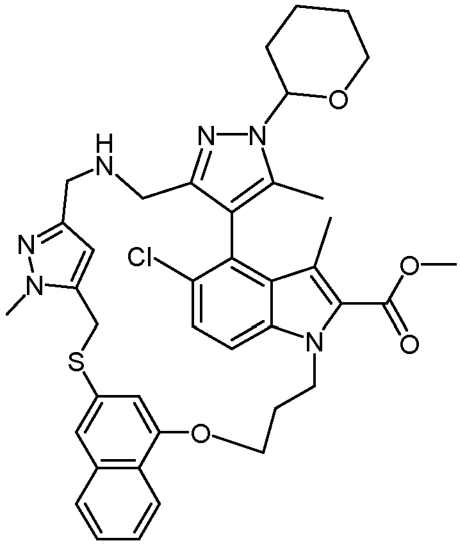

Thiophenol (10 g. 6.7 eq.) was added to a solution of Intermediate 55 (20.7 g, 13.6 mmol) and $K_2CO_3$ (5.6 g, 3 eq.) in ACN (150 mL). The reaction mixture was stirred at room temperature for 24 h. More thiophenol (6.5 g, 4.4 eq.) was added and the reaction mixture was stirred at room temperature for another 48 h. Water (40 mL) was added and the mixture was extracted with EtOAc (80 × 2 mL). The combined organic layer was dried with $Na_2SO_4$, filtered, and evaporated. The residue was purified by flash column chromatography over silica gel (DCM/ MeOH 100/0 to 90/10) to afford Intermediate 56 (5.4 g, yield: 44 %) as a white solid.

**Intermediate 57**

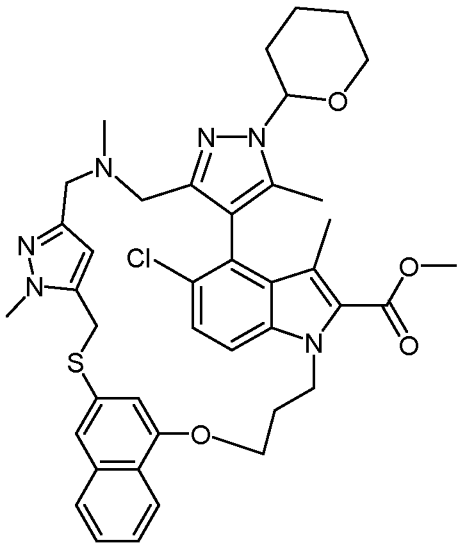

$NaBH_3CN$ (100 mg, 3 eq.) was added to a solution of Intermediate 56 (470 mg, 0.528 mmol) and paraformaldehyde (238 mg, 5 eq.) in MeOH (8 mL). The reaction mixture was stirred at room temperature for 3 h. The reaction was quenched by addition of water (20 mL) and the mixture was extracted with EtOAc (20 mL × 2). The combined organic layer was washed with brine, dried with $Na_2SO_4$, filtered, and evaporated. The residue was purified by flash column chromatography over silica gel (DCM/MeOH 100/0 to DCM/MeOH 90/10) to afford Intermediate 57 (400 mg, yield: 80 %) as a yellow solid.

**Intermediate 58 and Intermediate 59**

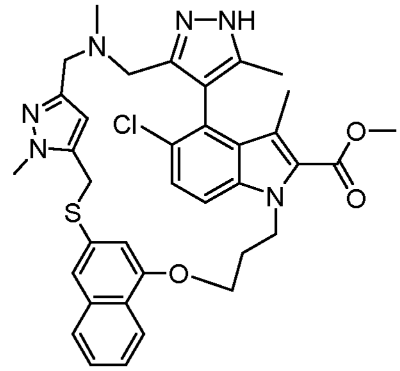

Intermediate 58: $R_a$ or $S_a$; one atropisomer but absolute stereochemistry undetermined
Intermediate 59: $S_a$ or $R_a$; one atropisomer but absolute stereochemistry undetermined

HCl (4 M in dioxane, 935 µL, 10 eq.) was added to a solution of Intermediate 57 (350 mg, 0.374 mmol) in dioxane (3 mL). The reaction mixture was stirred at room temperature for 3 h. The solvent was evaporated and the residue was purified by preparative SFC (Column: Daicel Chiralpak AD (250 mm × 30 mm, 10 um); Mobile phase: A: $CO_2$, B: (0.1 % $NH_3.H_2O$) iPrOH, A:B = 60:40) to afford Intermediate 58 (85 mg, yield: 34 %) and Intermediate 59 (80 mg, yield: 32 %).

**Intermediate 60**

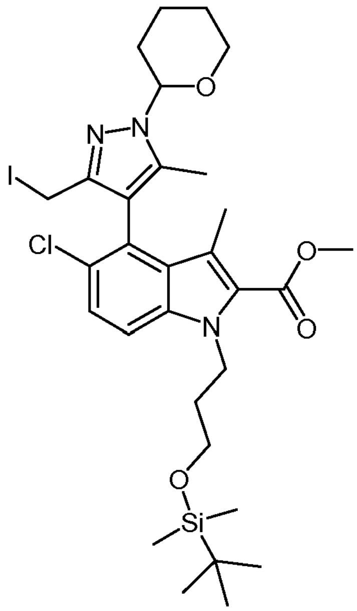

$Ms_2O$ (2.775 g, 2 eq.) was added slowly to a solution of Intermediate 49 (4.8 g, 7.966 mmol) and DIPEA (3 g, 3 eq.) in THF (80 mL) at 0 °C. The reaction mixture was stirred at room temperature for 24 h. LiI (3.2 g, 3 eq.) was then added to the reaction mixture at 0 °C, and stirring was continued at room temperature for 24 h. The reaction was quenched by addition of water (40 mL) and the mixture was extracted with DCM (80 mL × 2). The combined organic layer was evaporated to give Intermediate 60 (5.7 g, yield: 73 %), used without further purification.

## Intermediate 61

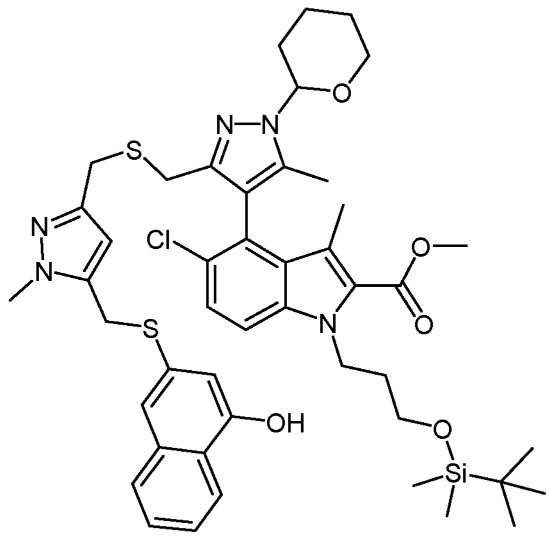

$K_2CO_3$ (2.435 g, 3 eq.) and $PPh_3$ (154 mg, 0.1 eq.) were added to a solution of Intermediate 60 (5.7 g, 5.873 mmol) and ethanethioic acid, S-[[5-[[[4-(acetyloxy)-2-naphthalenyl]thio]methyl]-1-methyl-1H-pyrazol-3-yl]methyl] ester [2245716-36-1] (3.835 g, 1.5 eq.) in MeOH (100 mL). The reaction mixture was stirred at room temperature for 16 h. The solvent was evaporated and the residue was partitioned between water (40 mL) and EtOAc. The layers were separated and the aqueous layer was extracted with EtOAc (50 mL × 2). The combined organic layer was dried with $Na_2SO_4$, filtered, and evaporated to give Intermediate 61 (8 g, yield: 63 %), used without further purification.

## Intermediate 62

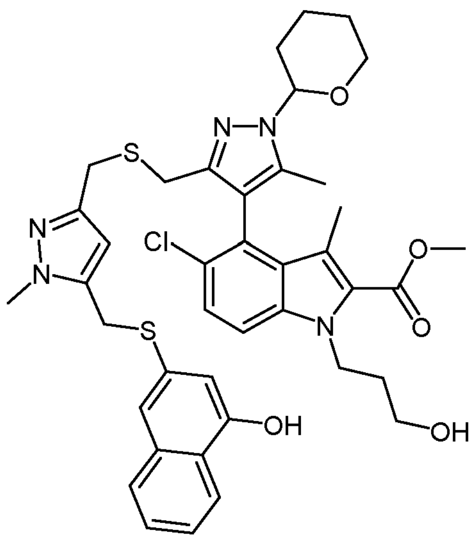

TBAF (1 M in THF, 11.2 mL, 3 eq.) was added to a solution of Intermediate 61 (8 g, 41 % pure, 3.731 mmol) in THF (50 mL) and the reaction mixture was stirred at room temperature for 16 h. EtOAc (60 mL) was added to the mixture and the solution was washed with water (40 mL × 2) and brine (20 mL), dried with $Na_2SO_4$, filtered, and evaporated. The residue was purified by flash column chromatography over silica gel (petroleum ether/EtOAc 100/0 to 0/100). The isolated product was purified again by preparative HPLC (Column: YMC-Triart Prep C18 150 × 40 mm × 7 um; Gradient: water (0.04 % $NH_3.H_2O$ + 10 mM $NH_4.HCO_3$)/ACN 40/60 to 30/70) to give Intermediate 62 (2.5 g, yield: 84 %) as a white solid.

## Intermediate 63

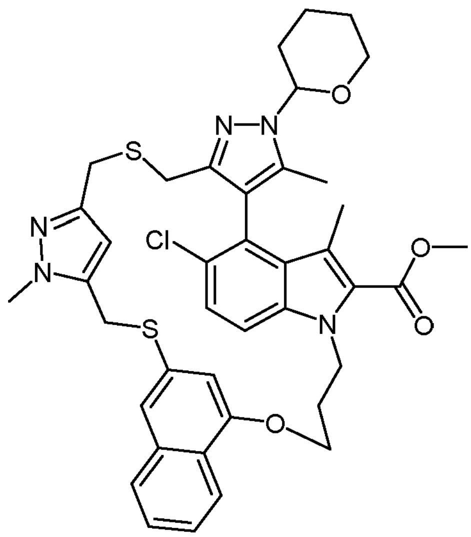

DTBAD (2.42 g, 3 eq.) was added to a solution of Intermediate 62 (2.5 g, 3.196 mmol) and $PBu_3$ (2.37 mL, 3 eq.) in DCM (150 mL) at 0 °C under nitrogen atmosphere. The reaction mixture was stirred at room temperature for 16 h. The solvent was evaporated and the residue was purified by flash column chromatography over silica gel (petroleum ether/EtOAc 100/0 to 30/70) to afford Intermediate 63 (2.4 g, yield: 79 %) as a white solid.

## Intermediate 64 and Intermediate 65

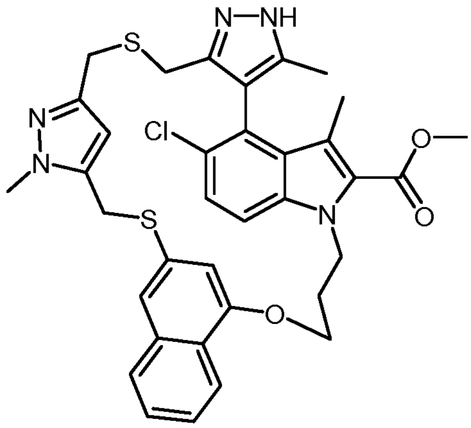

Intermediate 64: $R_a$ or $S_a$; one atropisomer but absolute stereochemistry undetermined
Intermediate 65: $S_a$ or $R_a$; one atropisomer but absolute stereochemistry undetermined

HCl (4 M in dioxane, 6.33 mL, 10 eq.) was added to a solution of Intermediate 63 (2.4 g, 2.534 mmol) in dioxane (30 mL) and the reaction mixture was stirred at room temperature for 16 h. The reaction mixture was adjusted to pH 8 by progressively adding saturated aqueous $NaHCO_3$ and was then extracted with DCM (30 mL × 2). The combined organic layer was washed with brine, dried with $Na_2SO_4$, filtered, and evaporated. The residue was purified by flash column chromatography over silica gel (petroleum ether/EtOAc 100/0 to 0/100) to give the racemic mixture of Intermediate 64 and Intermediate 65. This mixture was separated into its atropisomers by 1.8 g separated by SFC (Column: Daicel Chiralpak AD (250 mm × 30 mm, 10 um); Mobile phase: A: $CO_2$, B: 0.1 %$NH_3.H_2O$ in EtOH, A:B = 55:45) to afford Intermediate 64 (630 mg, yield: 48 %) and Intermediate 65 (620 mg, yield: 47 %).

**Preparation of Compounds**

**$1^5$-chloro-$1^3$,$2^1$,$2^5$,$6^1$-tetramethyl-$1^1H$,$2^1H$,$6^1H$-10-oxa-4,8-dithia-1(4,1)-indola-2(4,3),6(3,5)-dipyrazola-9(3,1)-naphthalenacyclotridecaphane-$1^2$-carboxylic acid, $S_a$ or $R_a$ atropisomer (one atropisomer but absolute stereochemistry undetermined) (Compound 1)**

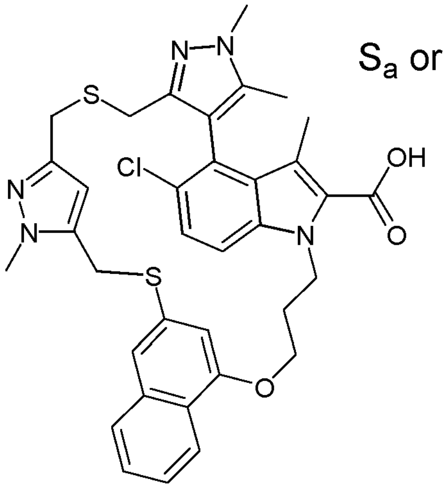

$S_a$ or $R_a$ atropisomer

$S_a$ or $R_a$ atropisomer (one atropisomer but absolute stereochemistry undetermined)

A solution of lithium hydroxide (15 mg, 10 eq.) in water (0.6 mL) was added to a solution of Intermediate 14 (42 mg) in THF (1.8 mL)/MeOH(1.8 mL). The reaction mixture was heated at 60 °C for 2 h. The reaction mixture was cooled to room temperature, HCl (1 mL, 1 M in water) was added and volatiles were removed in vacuo. The residue was purified by preparative HPLC (Stationary phase: RP XBridge Prep C18 OBD-10μm,30×150mm, Mobile phase: 0.25% $NH_4HCO_3$ solution in water, $CH_3CN$). The product was then triturated with DIPE, filtered, and dried in vacuo at 60 °C, to give Compound 1 (30 mg, 73 %).

LC-MS m/z 672 [M + H]+ (LCMS Method Code 2)

$^1H$ NMR (400 MHz, DMSO-$d_6$, 27°C) $\delta$ ppm 1.90 (s, 3 H) 1.98 (s, 3 H) 2.30 - 2.43 (m, 2 H) 2.91 (d, J=14.0 Hz, 1 H) 3.10 (d, J=14.0 Hz, 1 H) 3.15 (d, J=13.2 Hz, 1 H) 3.25 - 3.28 (m, 1 H) 3.68 (s, 3 H) 3.74 (s, 3 H) 3.79 - 3.99 (m, 2 H) 4.18 (d, J=15.5 Hz, 1 H) 4.30 (d, J=15.5 Hz, 1 H) 4.51 - 4.63 (m, 1 H) 4.89 (s, 1 H) 5.00 - 5.10 (m, 1 H) 6.64 (s, 1 H) 7.19 (d, J=8.9 Hz, 1 H) 7.38 (s, 1 H) 7.43 - 7.53 (m, 2 H) 7.66 (d, J=9.0 Hz, 1 H) 7.69 - 7.75 (m, 1 H) 8.10 - 8.17 (m, 1 H).

**$1^5$-chloro-$1^3$,$2^1$,$2^5$,$6^1$-tetramethyl-$1^1H$,$2^1H$,$6^1H$-10-oxa-4,8-dithia-1(4,1)-indola-2(4,3),6(3,5)-dipyrazola-9(3,1)-naphthalenacyclotridecaphane-$1^2$-carboxylic acid, $R_a$ or $S_a$ atropisomer (one atropisomer but absolute stereochemistry undetermined) (Compound 2)**

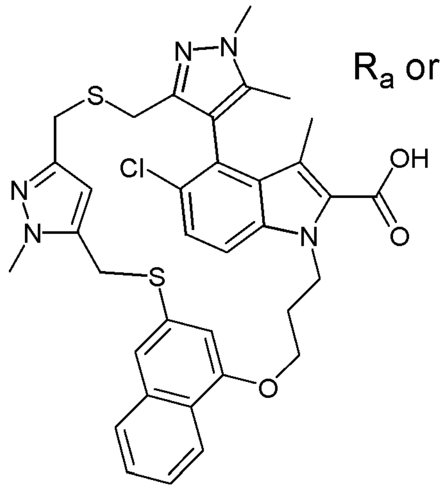

$R_a$ or $S_a$ atropisomer

$R_a$ or $S_a$ atropisomer (one atropisomer but absolute stereochemistry undetermined)

A solution of lithium hydroxide (15 mg, 10 eq.) in water (0.6 mL) was added to a solution of Intermediate 15 (43 mg) in THF (1.8 mL)/MeOH(1.8 mL). The reaction mixture was heated at 60 °C for 2 h. The reaction mixture was cooled to room temperature, HCl (1 mL, 1 M in water) was added and volatiles were removed in vacuo. The residue was purified by preparative HPLC (Stationary phase: RP XBridge Prep C18 OBD-10μm,30×150mm, Mobile phase: 0.25% $NH_4HCO_3$ solution in water, $CH_3CN$). The product was then triturated with DIPE, filtered, and dried in vacuo at 60 °C, to give Compound 2 (30 mg, 71 %).

LC-MS m/z 672 [M + H]+ (LCMS Method Code 2)

$^1$H NMR (400 MHz, DMSO-$d_6$, 27°C) δ ppm 1.90 (s, 3 H) 1.98 (s, 3 H) 2.30 - 2.42 (m, 2 H) 2.91 (d, J=14.0 Hz, 1 H) 3.10 (d, J=14.0 Hz, 1 H) 3.15 (d, J=13.2 Hz, 1 H) 3.25 - 3.28 (m, 1 H) 3.68 (s, 3 H) 3.74 (s, 3 H) 3.81 - 3.97 (m, 2 H) 4.17 (d, J=15.5 Hz, 1 H) 4.30 (d, J=15.5 Hz, 1 H) 4.51 - 4.63 (m, 1 H) 4.89 (s, 1 H) 5.00- 5.10 (m, 1 H) 6.64 (s, 1 H) 7.19 (d, J=8.9 Hz, 1 H) 7.38 (s, 1 H) 7.43 - 7.53 (m, 2 H) 7.66 (d, J=9.0 Hz, 1 H) 7.69 - 7.75 (m, 1 H) 8.09 - 8.17 (m, 1 H).

**15-chloro-13,21,25,61,4-pentamethyl-11H,21H,61H-10-oxa-8-thia-4-aza-1(4,1)-indola-2(4,3),6(3,5)-dipyrazola-9(3,1)-naphthalenacyclotridecaphane-12-carboxylic acid (mixture of $S_a$ and $R_a$ atropisomers) (Compound 3)**

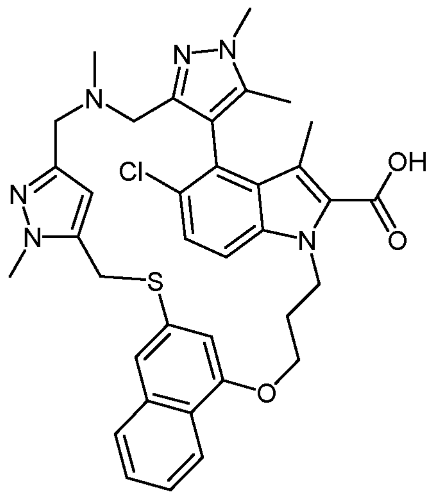

mixture of $S_a$ and $R_a$ atropisomers

A solution of lithium hydroxide (21 mg, 20 eq.) in water (1 mL) was added to a solution of Intermediate 26 (30 mg) in THF (2.5 mL) and MeOH (2.5 mL). The mixture was stirred at 60 °C for 4 h. The reaction mixture was cooled to room temperature and the solution was purified by preparative HPLC (Stationary phase: RP XBridge Prep C18 OBD-

5μm,50×250mm, Mobile phase: 0.25% $NH_4HCO_3$ solution in water, $CH_3CN$), affording Compound 3 (22 mg, 75 %) as a white solid (mixture of $S_a$ and $R_a$ atropisomers).

LC-MS m/z 669 [M + H]+ (LCMS Method Code 1)

$^1$H NMR (400 MHz, DMSO-$d_6$, 27°C) δ ppm 1.76 (s, 3 H), 1.90 (s, 3 H), 1.98 (s, 3 H), 2.35 - 2.48 (m, 2 H), 2.74 (d, J=12.2 Hz, 1 H), 2.77 (d, *J*=13.3 Hz, 1 H), 3.04 (d, *J*=13.3 Hz, 1 H), 3.30 (d, J=12.2 Hz, 1 H), 3.75 (s, 3 H), 3.76 (s, 3 H), 3.77 - 3.83 (m, 1 H), 4.23-4.39 (m, 3 H), 4.43 - 4.54 (m, 1 H), 4.70 (s, 1 H), 5.05 - 5.14 (m, 1 H), 6.86 (d, *J*=1.1 Hz, 1 H), 7.29 (s, 1 H), 7.34 (d, J=9.0 Hz, 1 H), 7.38 - 7.48 (m, 2 H), 7.64 - 7.69 (m, 1 H), 7.78 (d, J=9.0 Hz, 1 H), 7.99 - 8.05 (m, 1 H).

**15-Chloro-13,21,25,61-tetramethyl-11H,21H,61H-10-oxa-8-thia-4-aza-1(4,1)-indola-2(4,3),6(3,5)-dipyrazola-9(3,1)-naphthalenacyclotridecaphane-12-carboxylic acid (mixture of $S_a$ and $R_a$ atropisomers) (Compound 4)**

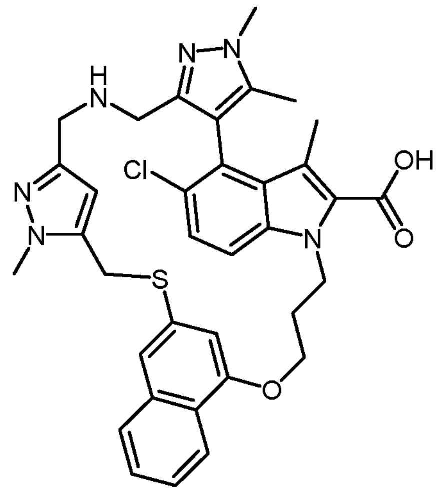

mixture of $S_a$ and $R_a$ atropisomers

A solution of lithium hydroxide (12 mg, 15 eq.) in water (0.5 mL) was added to a solution of Intermediate 25 (22 mg) in THF (0.8 mL) and MeOH (0.8 mL). The mixture was stirred at 60 °C for 4 h. The reaction mixture was cooled to room temperature and the solution was purified by preparative HPLC (Stationary phase: RP XBridge Prep C18 OBD-5μm,50×250mm, Mobile phase: 0.25% $NH_4HCO_3$ solution in water, $CH_3CN$), affording Compound 4 (17 mg, 79 %) as a white solid (mixture of $S_a$ and $R_a$ atropisomers).

LC-MS m/z 655 [M + H]+ (LCMS Method Code 2)

$^1$H NMR (400 MHz, DMSO-*$d_6$*, 27°C) δ ppm 1.92 (s, 3 H), 1.93 (s, 3 H), 2.35 - 2.44 (m, 2 H), 3.22 - 3.30 (m, 2 H), 3.3 3 (d, *J*=13.0 Hz, 1 H), 3.39 (d, *J*=13.0 Hz, 1 H), 3.74 (s, 3 H), 3.77 (s, 3 H), 3.82 - 3.92 (m, 1 H), 3.93 - 4.04 (m, 1 H), 4.26 (d, J=15.7 Hz, 1 H), 4.43 (d, *J*=15.7 Hz, 1 H), 4.47 - 4.56 (m, 1 H), 5.03 (s, 1 H), 5.07 - 5.17 (m, 1 H), 6.79 (d, J=0.8 Hz, 1 H), 7.23 (d, J=8.9 Hz, 1 H), 7.32 (br s, 1 H), 7.38 - 7.49 (m, 2 H), 7.65 - 7.71 (m, 2 H), 8.03 - 8.08 (m, 1 H).

## Compound 5

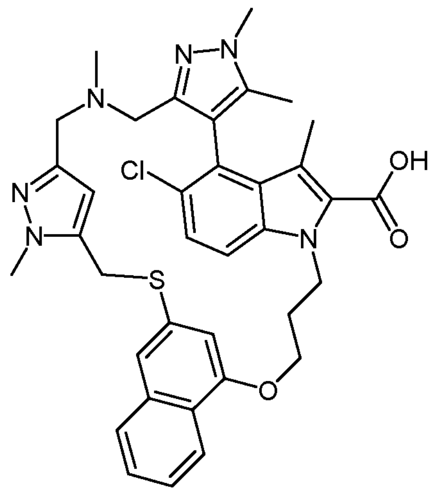

$R_a$ or $S_a$ atropisomer (one atropisomer but absolute stereochemistry undetermined)

Compound 5 was prepared according to an analogous procedure as for Compound 3, starting from Intermediate 27 instead of Intermediate 26

$^1$H NMR (400 MHz, DMSO-d6) δ ppm 1.76 (s, 3 H), 1.88 (s, 3 H), 1.97 (s, 3 H), 2.36 - 2.46 (m, 2 H), 2.70 (d, J=12.2 Hz, 1 H), 2.75 (d, J=13.3 Hz, 1 H), 3.04 (d, J=13.3 Hz, 1 H), 3.32 (d, J=12.2 Hz, 1 H), 3.72 - 3.80 (m, 7 H), 4.25 - 4.34 (m, 2 H), 4.37 (d, J=15.5 Hz, 1 H), 4.41 - 4.52 (m, 1 H), 4.64 (s, 1 H), 5.04 - 5.13 (m, 1 H), 6.86 (d, J=1.6 Hz, 1 H), 7.28 (s, 1 H), 7.34 (d, J=9.0 Hz, 1 H), 7.37 - 7.42 (m, 1 H), 7.42 - 7.47 (m, 1 H), 7.63 - 7.68 (m, 1 H), 7.79 (d, J=9.0 Hz, 1 H), 7.98 - 8.03 (m, 1 H)

## Compound 6

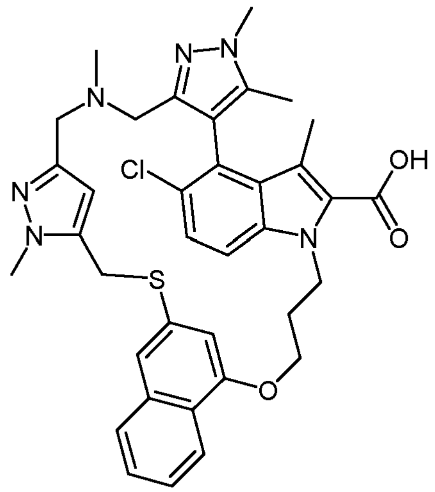

$S_a$ or $R_a$ atropisomer (one atropisomer but absolute stereochemistry undetermined)

Compound 6 was prepared according to an analogous procedure as for Compound 5, starting from Intermediate 28 instead of Intermediate 27.

$^1$H NMR (400 MHz, DMSO-d6) δ ppm 1.76 (s, 3 H), 1.88 (s, 3 H), 1.96 (s, 3 H), 2.35 - 2.46 (m, 2 H), 2.70 (d, J=12.3 Hz, 1 H), 2.75 (d, J=13.3 Hz, 1 H), 3.04 (d, J=13.3 Hz, 1 H), 3.32 (d, J=12.2 Hz, 1 H), 3.71 - 3.80 (m, 7 H), 4.25 - 4.34 (m, 2 H), 4.37 (d, J=15.5 Hz, 1 H), 4.41 - 4.51 (m, 1 H), 4.64 (s, 1 H), 5.09 (dt, J=14.6, 4.5 Hz, 1 H), 6.86 (d, J=1.6 Hz, 1 H), 7.28 (s, 1 H), 7.34 (d, J=8.9 Hz, 1 H), 7.37 - 7.42 (m, 1 H), 7.42 - 7.47 (m, 1 H), 7.64 - 7.68 (m, 1 H), 7.79 (d, J=9.0 Hz, 1 H), 7.98 - 8.03 (m, 1 H)

## Compound 7

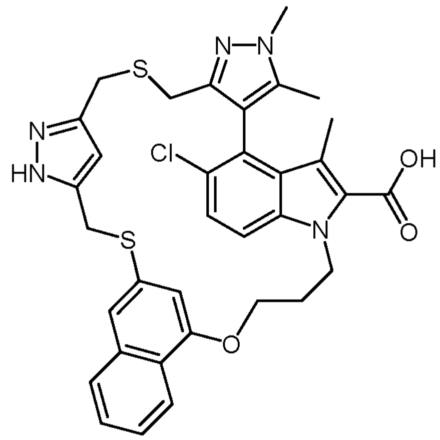

$S_a$ or $R_a$ atropisomer (one atropisomer but absolute stereochemistry undetermined)

LiOH (14 mg, 15 eq.) was added to a solution of Intermediate 37 (27 mg, 0.04 mmol) in a mixture of MeOH (1 mL), THF (1 mL), and water (0.5 mL). The resulting reaction mixture was stirred for 4 h at 60 °C. The reaction mixture was concentrated under reduced pressure to give a white solid. The solid was dissolved in water (5 mL) and acidified with 1 M aqueous HCl to pH 4-5, a white precipitate forming upon acidification. The aqueous layer was extracted with DCM (3 × 20 mL). The combined organic layer was dried over $MgSO_4$ and concentrated under reduced pressure to give a white solid. This crude product was purified by flash column chromatography on silica gel (DCM:MeOH - 1:0 to 9:1) to give a white solid which was triturated with DIPE and filtered to afford Compound 7 (24 mg, yield 86 %) as a white solid.

$^1$H NMR (400 MHz, DMSO-d6) δ ppm 1.90 (s, 3 H), 1.99 (s, 3 H), 2.31 - 2.42 (m, 2 H), 3.02 (d, J=14.3 Hz, 1 H), 3.20 (br dd, J=13.9, 3.7 Hz, 2 H), 3.75 (s, 3 H), 3.79 - 3.93 (m, 3 H), 4.05 - 4.16 (m, 2 H), 4.57 (ddd, J=14.1, 9.1, 4.6 Hz, 1 H), 4.90 (s, 1 H), 4.97 - 5.08 (m, 1 H), 6.66 (d, J=1.6 Hz, 1 H), 7.11 (d, J=8.9 Hz, 1 H), 7.38 (s, 1 H), 7.43 - 7.54 (m, 3 H), 7.56 - 7.65 (m, 2 H), 7.71 - 7.77 (m, 1 H), 8.17 (d, J=8.0 Hz, 1 H)

## Compound 8

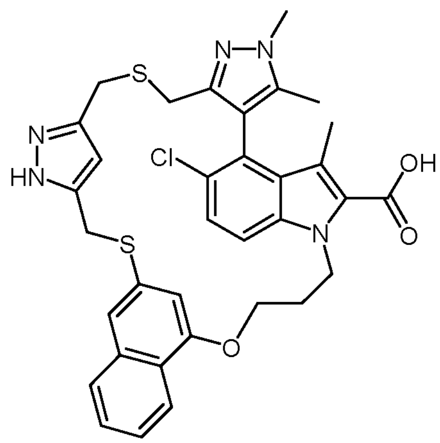

$R_a$ or $S_a$ atropisomer (one atropisomer but absolute stereochemistry undetermined)

Compound 8 was prepared according to an analogous procedure as for Compound 7, starting from Intermediate 38 instead of Intermediate 37.

$^1$H NMR (400 MHz, DMSO-d6) δ ppm 1.90 (s, 3 H), 1.99 (s, 3 H), 2.30 - 2.42 (m, 2 H), 3.02 (d, J=14.3 Hz, 1 H), 3.20 (dd, J=13.9, 3.9 Hz, 3 H), 3.35 (s, 1 H), 3.75 (s, 3 H), 3.86 (dq, J=17.0, 8.6 Hz, 2 H), 4.06 - 4.16 (m, 2 H), 4.57 (ddd, J=14.2, 9.1, 4.5 Hz, 1 H), 4.90 (s, 1 H), 5.03 (dt, J=14.6, 4.8 Hz, 1 H), 6.66 (d, J=1.6 Hz, 1 H), 7.11 (d, J=8.9 Hz, 1 H), 7.38 (s, 1 H), 7.44 - 7.54 (m, 2 H), 7.56 - 7.62 (m, 1 H), 7.71 - 7.78 (m, 1 H), 8.17 (d, J=8.0 Hz, 1 H)

**Compound 9**

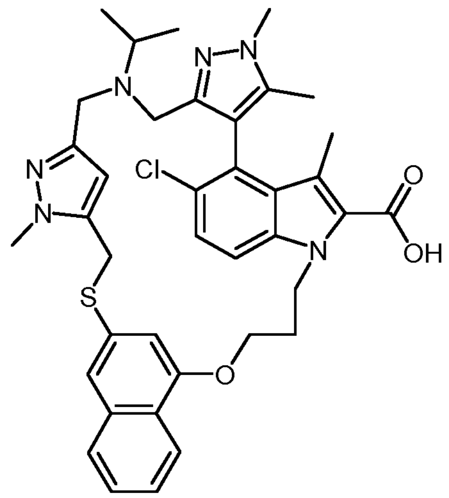

Mixture of $S_a$ and $R_a$ atropisomers

LiOH (1 M in water, 607 µL, 4 eq.) was added to a solution of Intermediate 40 (108 mg, 0.152 mmol) in THF (1.5 mL) and the reaction mixture was stirred at room temperature for 16 h. To push the reaction to completion, additional LiOH (1 M in water, 607 µL, 4 eq.) was added and the reaction mixture was stirred at room temperature for 48 h. The reaction mixture was diluted with DCM (2 mL) and acidified with Amberlite® IR-120 (H+) resin until pH 3. The mixture was filtered to remove the resin and the resin was washed with ACN and MeOH. The solvents were evaporated. The residue was purified by flash column chromatography (RPC18, ACN:MeOH 1:1/25 mM $NH_4HCO_3$, from 41/59 to 83/17). Organic solvents from the collected fractions were evaporated and the resulting aqueous suspension was acidified until pH 3 with a 1 M HCl solution and extracted with EtOAc (x 3). The combined organic layer was dried on $MgSO_4$, filtered, and evaporated to yield Compound 9 (69 mg, yield: 64 %) as a white solid.

$^1$H NMR (400 MHz, 373 K, DMSO-d6) δ ppm: 8.05 (d, J = 7.4 Hz, 1H), 7.68 (d, J = 7.8 Hz, 1H), 7.63 (d, J = 9.0 Hz, 1H), 7.50 - 7.38 (m, 2H), 7.35 (s, 1H), 7.27 (d, J = 8.7 Hz, 1H), 6.64 (s, 1H), 5.28 (s, 1H), 5.15 - 5.02 (m, 1H), 4.68 - 4.55 (m, 1H), 4.30 (d, J = 15.5 Hz, 1H), 4.16 (d, J = 15.5 Hz, 1H), 4.01 - 3.92 (m, 1H), 3.81 (s, 3H), 3.73 (s, 3H), 2.40 - 2.29 (m, 2H), 2.01 (s, 3H), 1.97 (s, 3H), 1.04 - 0.12 (br m, 6H).

**Compound 10**

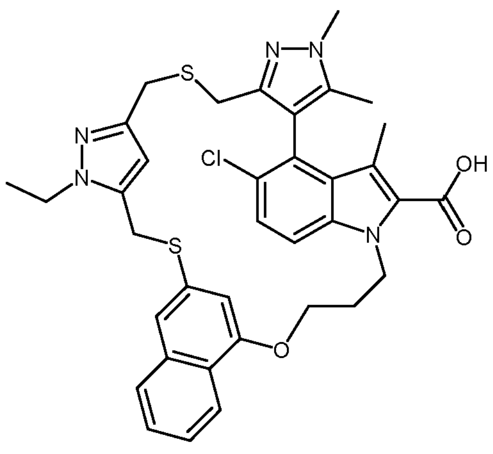

$R_a$ or $S_a$ atropisomer (one atropisomer but absolute stereochemistry undetermined)

LiOH (25 mg, 15 eq.) was added to a solution of Intermediate 41 (49 mg, 0.07 mmol) in a mixture of MeOH (1.6 mL), THF (1.6 mL) and water (0.8 mL). The resulting reaction mixture was stirred for 4 h at 60 °C. The reaction mixture was concentrated under reduced pressure to give a white solid. This solid was dissolved in water (15 mL) and acidified with 1 M aqueous HCl to pH 4-5, a white precipitate forming upon acidification. The aqueous layer was extracted

with DCM (2 × 20 mL), the combined organic layer was dried over $MgSO_4$, and concentrated under reduced pressure. This crude product was purified by flash column chromatography on silica gel (DCM:MeOH - 100:0 to 95:5) to give a solid which was triturated with $Et_2O$ and filtered to afford Compound 10 (34.5 mg, yield: 72 %) as an off-white solid.

$^1H$ NMR (400 MHz, CHLOROFORM-d) δ ppm 1.35 (t, J=7.2 Hz, 3 H), 2.03 (s, 3 H), 2.22 (s, 3H), 2.33 - 2.47 (m, 2H), 2.82 (d, J=14.4 Hz, 1 H), 3.18 (d, J=13.3 Hz, 1 H), 3.27 (d, J=14.6 Hz, 1 H), 3.46- 3.53 (m, 3 H), 3.64- 3.75 (m, 1 H), 3.81- 3.97 (m, 7 H), 4.59 (ddd, J=14.5, 8.2, 3.9 Hz, 1 H) 5.14 - 5.24 (m, 2 H), 6.10 (d, J=1.3 Hz, 1 H), 7.08 (d, J=8.9 Hz, 1 H), 7.33 (d, J=9.1 Hz, 1 H), 7.49- 7.59 (m, 2 H), 7.60 (s, 1 H), 7.69 - 7.77 (m, 1 H), 8.34 (dd, J=8.1, 0.89 Hz, 1 H).

## Compound 11

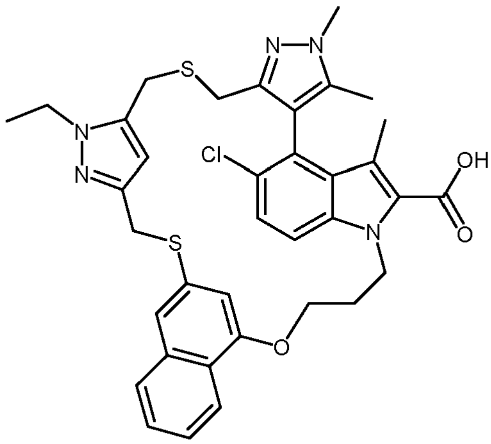

$R_a$ or $S_a$ atropisomer (one atropisomer but absolute stereochemistry undetermined)

Compound 11 was prepared according to an analogous procedure as for Compound 10, starting from Intermediate 42 instead of Intermediate 41.

$^1H$ NMR (400 MHz, CHLOROFORM-d) δ ppm 1.27 (t, J=7.21 Hz, 3 H), 1.99 (s, 3 H), 2.20 (s, 3 H), 2.33 - 2.54 (m, 2 H), 3.04 (d, J=14.74 Hz, 1 H), 3.24 (d, J=14.74 Hz, 1 H), 3.28 (d, J=14.32 Hz, 1 H), 3.45 (d, J=14.32 Hz, 1 H), 3.58 (td, J=9.48, 3.50 Hz, 1 H), 3.82 - 3.98 (m, 7H), 4.09 (d, J=15.15 Hz, 1 H), 4.57 - 4.68 (m, 1H), 5.11 (s, 1 H), 5.16 (dt, J=14.55, 4.27 Hz, 1 H), 6.58 (d, J=1.36 Hz, 1 H), 6.89 (d, J=8.99 Hz, 1 H), 7.24 (d, J=9.09 Hz, 1 H), 7.46 - 7.52 (m, 2 H), 7.53 (s, 1 H), 7.68 - 7.73 (m, 1 H), 8.34 (dd, J=8.31, 1.31 Hz, 1 H).

## Compound 12

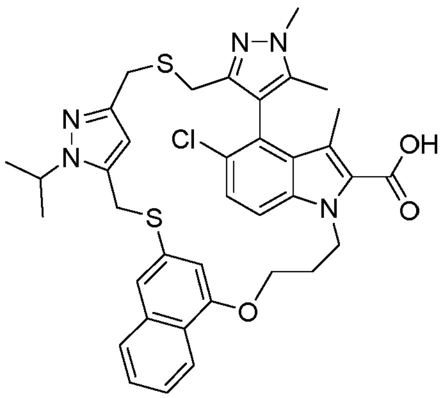

$R_a$ or $S_a$ atropisomer (one atropisomer but absolute stereochemistry undetermined)

LiOH (45 mg, 15 eq.) was added to a solution of Intermediate 43 (90 mg, 0.13 mmol) in a mixture of MeOH (2.8 mL), THF (2.8 mL) and water (1.5 mL). The resulting reaction mixture was stirred for 3 h at 50 °C. The reaction mixture was concentrated under reduced pressure. The residue was purified by flash chromatography on silica gel (DCM:MeOH - 100:0 to 95:5) to afford Compound 12 (66 mg, yield: 75 %) as an off-white solid.

1H NMR (400 MHz, CHLOROFORM-d) δ ppm 1.34 - 1.54 (m, 1 H), 1.42 (d, J=6.60 Hz, 3 H), 1.49 (d, J=6.38

Hz, 2 H), 2.00 (s, 3 H), 2.21 (s, 3 H), 2.37 - 2.49 (m, 2 H), 2.79 (d, J=13.86 Hz, 1 H), 3.20 (d, J=13.64 Hz, 1 H), 3.29 - 3.36 (m, 1 H), 3.38 - 3.45 (m, 1 H), 3.46 - 3.56 (m, 1 H), 3.76 - 3.95 (m, 6 H), 4.48 (spt, J=6.60 Hz, 1 H), 4.54 - 4.64 (m, 1 H), 5.02 (s, 1 H), 5.16 (dt, J=14.58, 4.15 Hz, 1 H), 6.22 (d, J=1.32 Hz, 1 H), 7.00 (d, J=8.80 Hz, 1 H), 7.30 (d, J=9.02 Hz, 1 H), 7.45 - 7.57 (m, 3 H), 7.66 - 7.72 (m, 1 H), 8.28 - 8.36 (m, 1 H).

## Compound 13

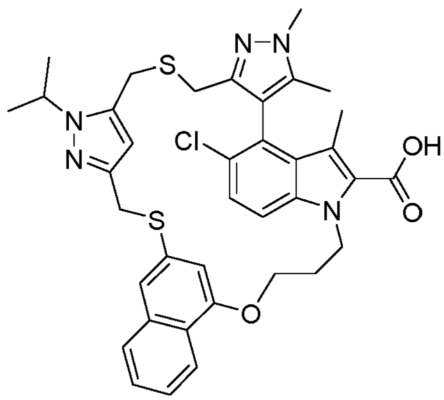

$R_a$ or $S_a$ atropisomer (one atropisomer but absolute stereochemistry undetermined)

LiOH (57 mg, 15 eq.) was added to a solution of Intermediate 44 (114 mg, 0.16 mmol) in a mixture of MeOH (3.6 mL), THF (3.6 mL) and water (1.8 mL). The reaction mixture was stirred for 3 h at 60 °C. The reaction mixture was concentrated under reduced pressure, diluted with water (15 mL), and acidified with 1M aqueous HCl until pH 4-5. The aqueous layer was extracted with DCM (2 × 10 mL) and then with a 1:1 mixture of EtOAc:THF (10 mL). The combined organic layer was dried over $MgSO_4$, filtered, and evaporated. The residue was purified by flash chromatography on silica gel (DCM:MeOH - 100:0 to 95:5) to afford Compound 13 (91 mg, yield: 81 %) as an off-white solid.

$^1$H NMR (400 MHz, CHLOROFORM-d) δ ppm 1.24 (d, J=6.58 Hz, 3 H), 1.31 (d, J=6.58 Hz, 3 H), 1.98 (s, 3 H) 2.17 (s, 3 H), 2.31 - 2.52 (m, 2 H), 3.09 (d, J=14.74 Hz, 1 H), 3.28 (dd, J=14.27, 9.56 Hz, 2 H), 3.43 (d, J=13.90 Hz, 1 H), 3.47 - 3.56 (m, 1 H), 3.83 - 3.92 (m, 5 H), 4.10 (d, J=15.15 Hz, 1 H), 4.29 (spt, J=6.55 Hz, 1 H), 4.55 - 4.69 (m, 1 H), 5.15 (dt, J=14.58, 4.36 Hz, 1 H), 5.21 (s, 1 H), 6.55 (d, J=1.36 Hz, 1 H), 6.82 (d, J=8.99 Hz, 1 H), 7.21 (d, J=9.09 Hz, 1 H), 7.44 - 7.52 (m, 3 H), 7.65 - 7.73 (m, 1 H), 8.29 - 8.36 (m, 1 H).

## Compound 14

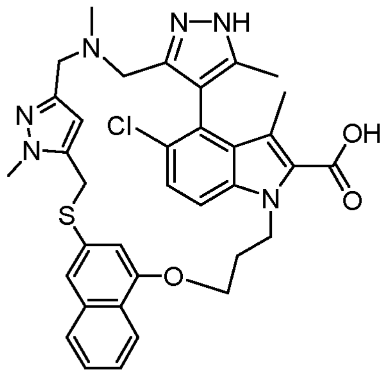

$R_a$ or $S_a$ atropisomer (one atropisomer but absolute stereochemistry undetermined)

A solution of LiOH.$H_2O$ (27 mg, 5 eq.) in water (0.5 mL) was added to a solution of Intermediate 58 (85 mg, 0.127 mmol) in THF (3 mL). The reaction mixture was stirred at 45 °C for 16 h. HCl (0.5 M in water) was added to reach pH 6. The solvent was evaporated and the residue was purified by preparative HPLC (Column: Phenomenex Gemini-NX 150 × 30 mm × 5 um; gradient water (0.05 %HCl)/ACN, from 77/23 to 47/53) to give Compound 14 (35 mg, yield: 41 %) as a white solid.

$^1$H NMR (400 MHz, DMSO-d6) δ ppm 1.89 (br d, J=7.28 Hz, 8 H) 2.39 (br s, 2 H) 3.20 - 3.27 (m, 2 H) 3.40 -

3.48 (m, 3 H) 3.73 (s, 5 H) 4.24 (br s, 2 H) 4.54 (br s, 2 H) 5.00 - 5.13 (m, 1 H) 6.69 (br s, 1 H) 7.28 (br d, J=8.82 Hz, 2 H) 7.35 - 7.48 (m, 2 H) 7.64 (br d, J=8.16 Hz, 1 H) 7.80 (d, J=9.04 Hz, 1 H) 7.98 (br d, J=6.61 Hz, 1 H) 13.22 (br s, 1 H)

## Compound 15

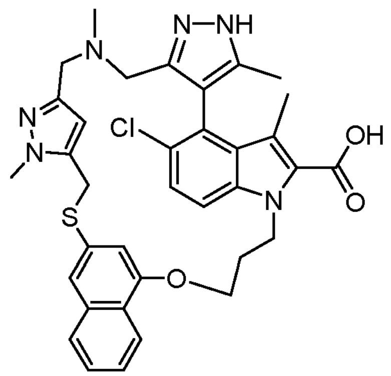

$S_a$ or $R_a$ atropisomer (one atropisomer but absolute stereochemistry undetermined)

Compound 15 was prepared according to an analogous procedure as for Compound 14, starting from Intermediate 59 instead of Intermediate 58.

$^1$H NMR (400 MHz, DMSO-d6) δ ppm 1.83 (s, 5 H) 1.93 (s, 3 H) 2.27 - 2.41 (m, 2 H) 3.08 (br s, 2 H) 3.17 - 3.28 (m, 3 H) 3.73 (s, 4 H) 4.17 - 4.34 (m, 3 H) 4.40 - 4.62 (m, 2 H) 4.99 - 5.10 (m, 1 H) 6.77 (s, 1 H) 7.23 (s, 1 H) 7.30 - 7.46 (m, 3 H) 7.62 (d, J=7.94 Hz, 1 H) 7.79 (d, J=9.04 Hz, 1 H) 7.97 (br d, J=7.94 Hz, 1 H) 12.86 (br s, 1 H)

## Compound 16

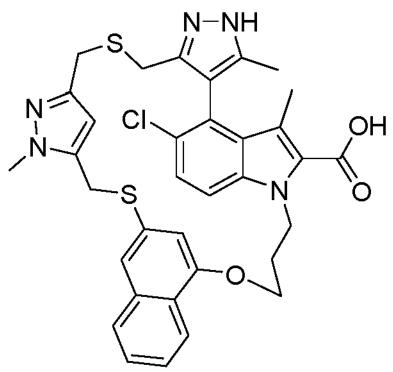

$R_a$ or $S_a$ atropisomer (one atropisomer but absolute stereochemistry undetermined)

Compound 16 was prepared according to an analogous procedure as for Compound 14, starting from Intermediate 64 instead of Intermediate 58.

$^1$H NMR (400 MHz, METHANOL-d4) δ ppm 1.92 (s, 3 H) 2.01 (s, 3 H) 2.45 (br s, 2 H) 2.58 - 2.70 (m, 2 H) 2.84 - 2.92 (m, 1 H) 3.08 (d, J=14.33 Hz, 1 H) 3.76 (s, 3 H) 3.78 - 3.89 (m, 2 H) 3.96 - 4.06 (m, 2 H) 4.61 - 4.69 (m, 2 H) 5.14 - 5.23 (m, 1 H) 6.43 (s, 1 H) 7.06 (d, J=8.82 Hz, 1 H) 7.19 (s, 1 H) 7.41 - 7.49 (m, 2 H) 7.53 (d, J=9.04 Hz, 1 H) 7.63 (br dd, J=6.50, 2.76 Hz, 1 H) 8.09 - 8.16 (m, 1 H)

**Compound 17**

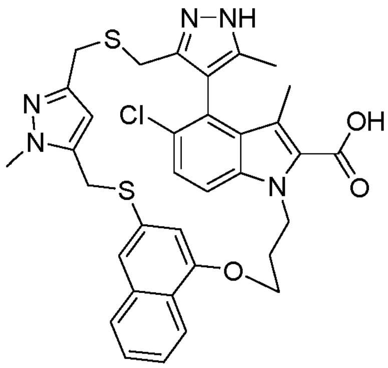

$S_a$ or $R_a$ atropisomer (one atropisomer but absolute stereochemistry undetermined)

Compound 17 was prepared according to an analogous procedure as for Compound 14, starting from Intermediate 65 instead of Intermediate 58.

1H NMR (400 MHz, METHANOL-d4) δ ppm 1.92 (s, 3 H) 2.01 (s, 3 H) 2.44 (br dd, J=9.59, 4.30 Hz, 2 H) 2.57 - 2.69 (m, 2 H) 2.84 - 2.92 (m, 1 H) 3.08 (d, J=14.33 Hz, 1 H) 3.75 (s, 3 H) 3.76 - 3.90 (m, 2 H) 3.94 - 4.03 (m, 2 H) 4.58 - 4.70 (m, 2 H) 5.12 - 5.22 (m, 1 H) 6.42 (s, 1 H) 7.06 (d, J=9.04 Hz, 1 H) 7.18 (s, 1 H) 7.40 - 7.47 (m, 2 H) 7.52 (d, J=9.04 Hz, 1 H) 7.62 (br dd, J=6.62, 2.65 Hz, 1 H) 8.07 - 8.16 (m, 1 H)

**Analytical Analysis**

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rt) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method Codes (Flow expressed in mL/min; column temperature (T) in °C; Run time in minutes)

| Method Code | Instrument | column | mobile phase | gradient | Flow<br>Col T | Run time |
|---|---|---|---|---|---|---|
| 1 | Waters: Acquity® UPLC® - DAD and SQD | BEH C18 column (1.7 µm, 2.1 × 50 mm; Waters Acquity) | A: 10 mM ammonium acetate in $H_2O$/ acetonitrile 95/5; B: acetonitrile | 95 % A and 5 % B to 5 % A and 95 % B in 1.3 minutes and hold for 0.7 minutes | 0.8<br>55 | 2 |
| 2 | Waters: Acquity® UPLC® - DAD and SQD | Waters :BEH C18 (1.8µm, 2.1 * 100mm) | A: 10mM $CH_3COONH_4$ in 95% $H_2O$ + 5% $CH_3CN$<br>B: $CH_3CN$ | From 100% A to 5% A in 2.10min, to 0% A in 0.90min, to 5% A in 0.5min | 0.7<br>55 | 3.5 |

(continued)

| Method Code | Instrument | column | mobile phase | gradient | Flow / Col T | Run time |
|---|---|---|---|---|---|---|
| 3 | Waters: Acquity® UPLC®-DAD, SQD and ELSD | Waters : HSS T3 (1.8μm, 2.1 × 100mm) | A: 10 mM $CH_3COONH_4$ in 95 % $H_2O$ + 5 % $CH_3CN$<br>B: $CH_3CN$ | From 100 % A to 5 % A in 2.10 min, to 0 % A in 0.90 min, to 5 % A in 0.5 min | 0.6<br>55 | 3.5 |
| 4 | Agilent: 1100-DAD and MSD | YMC: Pack ODS-AQ (3μm, 4.6 × 50 mm) | A: HCOOH 0.1 % in water, B: $CH_3CN$ | 95 % A to 5 % A in 4.8 min, held for 1 min, back to 95 % A in 0.2 min. | 2.6 | 6 |
| 5 | Waters: Acquity® UPLC® - DAD and SQD2 | Waters :BEH (1.8 μm, 2.1 × 50 mm) | A: 0.1 % $NH_4HCO_3$ in 95 % $H_2O$ + 5 % $CH_3CN$<br>B: $CH_3CN$ | From 100 % A to 5 % A in 1.3 min, hold 0.7min | 0.8<br>55 | 2,0 |
| 6 | Agilent 1200 equiped with MSD 6110 or equivalent | Waters Xbridge-C18 column (5 μm, 2.0 × 50 mm) | A: water with 0.04 % TFA<br>B: $CH_3CN$ with 0.02 % TFA | 90 % A held for 0.8 min. Then to 20 % A and 80 % B in 3.7 minutes, held for 3 min. Return to 90 % A in 2 min and hold for 0.5 min. | 0.8<br>50 | 10 |
| 7 | Agilent 1200 equiped with MSD 6110 or equivalent | Waters XBridge ShieldRP18 column (5 μm, 2.1 × 50 mm) | A: water with 0.05<br>%$NH_3.H_2O$; B: $CH_3CN$) | 100% A held for 1 min. Then to 40 % A and 60 % B in 4 min Then to 5 % A and 95 % B in 2.5 min. Return to 100% A in 2 min and hold for 0.5 min | 0.8<br>40 | 10 |

LCMS results (RT means retention time)

| Compound number | LCMS results |
|---|---|
| 1 | confirms the MW (RT: 1.79, [M+H]+ 672, LCMS Method 2) |
| 2 | confirms the MW (RT: 1.79, [M+H]+ 672, LCMS Method 2) |
| 3 | confirms the MW (RT: 0.88, [M+H]+ 669, LCMS Method 1) |
| 4 | confirms the MW (RT: 1.65, [M+H]+ 655, LCMS Method 3) |
| 5 | confirms the MW (RT: 1.68, [M+H]+ 669, LCMS Method 3) |
| 6 | confirms the MW (RT: 1.68, [M+H]+ 669, LCMS Method 3) |
| 7 | confirms the MW (RT: 1.03, [M+H]+ 658, LCMS Method 1) |
| 8 | confirms the MW (RT: 1.03, [M+H]+ 658, LCMS Method 1) |
| 9 | confirms the MW (RT: 2.99, [M+H]+ 697, LCMS Method 4) |
| 10 | confirms the MW (RT: 1.92, [M+H]+ 686, LCMS Method 2) |
| 11 | confirms the MW (RT: 1.93, [M+H]+ 686, LCMS Method 2) |
| 12 | confirms the MW (RT: 1.06, [M+H]+ 700, LCMS Method 5) |
| 13 | confirms the MW (RT: 1.05, [M+H]+ 700, LCMS Method 5) |
| 14 | confirms the MW (RT: 3.23, [M+H]+ 655, LCMS Method 6) |
| 15 | confirms the MW (RT: 3.23, [M+H]+ 655, LCMS Method 6) |
| 16 | confirms the MW (RT: 3.63, [M+H]+ 658, LCMS Method 7) |

(continued)

| Compound number | LCMS results |
|---|---|
| 17 | confirms the MW (RT: 3.64, [M+H]+ 658, LCMS Method 7) |

SFC-MS methods

The SFC measurement was performed using an Analytical Supercritical fluid chromatography (SFC) system composed by a binary pump for delivering carbon dioxide (CO2) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Analytical SFC-MS Methods (Flow expressed in mL/min; column temperature (T) in °C; Run time in minutes, Backpressure (BPR) in bars. "i-$PrNH_2$" means isopropylamine, "EtOH" means ethanol, "min" mean minutes, "DEA" means diethylamine.

| SFC Method | Column | mobile phase | gradient | Flow | Run time |
|---|---|---|---|---|---|
| | | | | Col T | BPR |
| Method 1 | Daicel Chiralpak® AD3 column (3.0 μm, 150 × 4.6 mm) | A:$CO_2$ B: EtOH+0.2% i-$PrNH_2$ | 10%-50% B in 6 min, hold 3.5 min | 2.5 | 9.5 |
| | | | | 40 | 130 |
| Method 2 | Daicel Chiralpak® AS3 column (3.0 μm, 150 × 4.6 mm) | A:$CO_2$ B: EtOH+0.2 % i$PrNH_2$ | 5 % B hold 6 min, to 50 % in 1 min hold 2.5 min | 2.5 | 9.5 |
| | | | | 40 | 130 |
| Method 3 | Daicel Chiralpak® IG3 column (3.0 μm, 150 × 4.6 mm) | A:$CO_2$ B: EtOH+0.2 % i$PrNH_2$ | 10 %-50 % B in 6 min, hold 3.5 min | 2.5 | 9.5 |
| | | | | 40 | 130 |
| Method 4 | Brand Chiralcel® OD-3 column (3.0 μm, 100 × 4.6 mm) | A: $CO_2$ B: EtOH+0.05 % DEA | 5 % B to 40% in 4.5 min and 40 % B hold 2.5 min, 5 % B hold 1 min | 2.8 | 8 |
| | | | | 40 | 100 |

Table: Analytical SFC data - $R_t$ means retention time (in minutes), $[M+H]^+$ means the protonated mass of the compound, method refers to the method used for (SFC)MS analysis of enantiomerically pure compounds. No. means number.

| Compound No. | SFC Method | Rt | $[M+H]^+$ |
|---|---|---|---|
| 1 | 1 | 5.02 | 672 |
| 2 | 1 | 5.61 | 672 |
| 7 | 2 | 4.55 | 658 |
| 10 | 3 | 6.16 | 686 |
| 11 | 3 | 6.34 | 686 |
| 14 | 4 | 4.67 | 655 |
| 15 | 4 | 6.03 | 655 |
| 16 | 4 | 5.09 | 658 |
| 17 | 4 | 6.19 | 658 |

NMR

$^1H$ NMR spectra were recorded on Bruker Avance III and Avance NEO spectrometers. $CDCl_3$ was used as solvent, unless otherwise mentioned. The chemical shifts are expressed in ppm relative to tetramethylsilane.

**Pharmacological Analysis**

Biological Example 1

Molp8 multiple myeloma cell line survival assay using Annexin V and 7AAD.

The Annexin V/7AAD principle is based on the location of phosphatidylserine in the plasma membrane and the integrity of the membrane barrier. During early apoptosis, phosphatidylserine loses its normal distribution patterns in the inner leaflet of the plasma membrane and appears on the exterior of the plasma membrane. Annexin proteins are calcium dependent phospholipid binding proteins and can bind phosphatidylserine on the outer membrane during cell death. The viability dye 7AAD is excluded from entering cells with intact plasma membranes but accumulates in cells that have lost membrane integrity. Annexin Fitc is excited by the 488nm laser (max excitation: 490 nm, max emission: 525 nm) as is 7AAD (excitation: 488 nm, emission: 650 nm), which allows this assay to be completed on any multicolor flow cytometer.

The assay conditions were optimized using the following 1x reaction buffer: 5mM HEPES (4-(2-hydroxyethyl)-1-piperazineethanesulfonic acid), 70mM NaCl, 1.25 mM $CaCl_2$, pH 7.4. The reagents / materials used in the assay were as listed in the Table below.

Table: Reagents Used in Assay (Biological Example 1)

| Reagent | MW (Da) | Conc. | Comments |
|---|---|---|---|
| Amexin V-Fitc | | | 300 tests |
| Annexin Buffer | | 10x | diluted in sterile water |
| 7AAD | 1270 | 1 mg/mL | |
| Molp8 Cell Line | | | Mycoplasma negative |
| RPMI 1640 | | | |
| Penicillin / Streptomycin | 334 | 10,000 U/mL | |
| FBS | | | |
| 96 Well U-bottom plate | | | |

The Molp 8 cells were multiple myeloma, human, in suspension (*cells were counted 2x per week and culture density maintained between 0.5 and $1.5\times10^6$ cells per ml).

Assay Procedure

On Day 1, $5\times10^4$ live cells per well were plated in a 96-well U-bottom plate, with the cells plated in 100uL RPMI +10% FBS. An 11-point compound dilution plate was prepared in 3-fold steps (Final concentration range 10μM-0.1nM) and added at a concentration of 1μL per well. The plate was then incubated for 3 days at 37°C and 5 % $CO_2$.

On Day 4, the cells were pelletted and the media discarded. The cells were then resuspended in 50 μL AnnexinV-Fitc (1:40 dilution) and 7AAD (1:100 dilution) in Annexin binding buffer, then incubated for 30 min @ room temperature in the dark. 50 μL binding buffer (100uL total volume) was then added to the cells. There was no wash step required.

Within 2 hours, PeCy5 (7AAD) and Fitc (Annexin) channels were recorded using BD Canto, collecting a minimum per singlet of 10,000 events.

Analysis

The activity of a test compound was calculated based on the live cell percentage.

LC = Median of the low control values
= Low control: cells without treatment (no greater than 100% live)
HC = Median of the high control values
= High control: cells treated with 10uM drug (no less than 0% live)

A best-fit curve was fitted by a minimum sum of squares method to the plot of live cell percent vs. compound concentration. From this an $IC_{50}$ value (inhibitory concentration causing 50 % cytotoxicity) was obtained. An estimate

of the slope of the plot in terms of the Hill coefficient was also obtained.

Representative compounds of Formula (I) of the present invention were tested according to the procedures described in Biological Example 1, with results as listed in the Table below. Wherein a compound was tested more than once, each measurement result is listed individually.

The IC50 values reported in the table below are subject to error margins associated with the assay used and the equipment.

Table: Measured $IC_{50}$ for Representative Compounds of Formula (I)

| Compound | N | MOLP8 Apoptosis $IC_{50}$ (nM) |
|---|---|---|
| 1 | 2 | 54/106 |
| 2 | 2 | 1304/7044 |
| 3 | 2 | 200/256 |
| N=number of independent runs | | |

Biological Example 2

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

Activation of the apoptotic pathway was measured using the CellEvent™ Caspase-3/7 Green ReadyProbes™ Reagent (Thermo Fisher C10423, C10723). This assay produces a green fluorescent stain in cells that enter the apoptosis pathway. CellEvent® Caspase-3/7 Green reagent is a four amino acid peptide (DEVD) conjugated to a nucleic acid-binding dye that is non-fluorescent when not bound to DNA. The CellEvent® Caspase-3/7 Green reagent is intrinsically non-fluorescent, as the DEVD peptide inhibits binding of the dye to DNA. Upon activation of caspase-3/7 in apoptotic cells, the DEVD peptide is cleaved and the free dye can bind DNA, generating a bright green fluorescence. The activation of Caspase-3 and Caspase-7 is downstream of inhibition of MCL-1 or other apoptosis inhibiting proteins in cell lines that are dependent on them.

The live-cell readout on the IncuCyte permits tracking over time of the Caspase activation. The kinetic readout was useful as (a) it reveals differences in time of onset that can be related to differences in the mechanism of apoptosis induction, i.e. this being more direct or indirect; and (b) it allows recognition of artifacts resulting from autofluorescent or precipitating compounds. The IncuCyte readout also allows one to normalize for cell number, as the suspension cells are hard to distribute evenly.

Signals were measured every 2h for a duration of 22h. The ratio of the Caspase mask to the Confluence mask, per image, as raw data, was calculated and the kinetic trace for every well was exported to Genedata Screener for analysis.

In Genedata Screener values for 6h, 12h, and 22h from the kinetic traces were extracted. The values were normalized against negative controls (untreated cells). A standard dose-response analysis was performed on the normalized data.

The following data was reported at each of the following three aforementioned time points: (a) The dose-response curve, (b) The qAC50 and qAC50 Mode, and (c) Max Activity.

Materials used in the assay were as listed in the Table below.

Table - Assay Materials

| ***Reagent*** |
|---|
| MOLP8 cell line (mycoplasma test negative) |
| ViewPlate-384 Black |
| CellEvent™ Caspase-3/7 Green Detection Reagent |
| Breathe-EASIERTM |
| DMSO (dimethyl sulfoxide) |
| RPMI Medium 1640 (1X) without Phenol red and L-Glutamine |
| Heat Inactivated FBS (Fetal bovine serum) |

(continued)

| Reagent |
|---|
| L-Glutamine solution |
| Gentamicin |

Cells were maintained in culture medium containing 10% Heat Inactivated (HI) FBS, 2mM L-Glutamine and 50 μg/mL Gentamycin phenol red free RPMI-1640. Cells were split at 0.4 million /mL twice a week.

On Day 1, plates containing individual wells with test compounds at 10 mM concentration, 150 nL per well. The final concentrations range from 100 μM to 10 pM compound (and no compound control) and compounds were thawed at room temperature for 1 hour. 25 μl of prewarmed medium was added into each well by multidrop (column 1, 3-22, 24), followed by addition of DMSO control (0.6% DMSO) in column 2. The plate was sealed using Breathe-Easy® sealing membrane and shaken for 30 min at room temperature to dissolve the test compound(s) in medium. The plate was then kept in the incubator for 1 hour at 37 °C, 5 % $CO_2$.

MOLP8 cells in medium at 40000/25 μl (20000/50 μl final in assay) were prepared with CellEvent™ Caspase-3/7 Green Detection Reagent at 4 μM (2 μM final in assay). Once prepared, the cells were added to the test compound plate in an amount of 20000 and the plate was immediately placed in the IncuCyte and imaging started using following settings: 10X objective, 2 s exposure time in green channel, interval of 2 h, acquisition stopped after 22 h.

For analysis in IncuCyte, a Basic Analysis protocol was defined to calculate the "confluence" and "caspase" areas from the "Phase" and "green" images, respectively, as follows: (a) Confluence: Segmentation Adjustment 1, Hole Fill 0, Adjust Size -2, No filters (b) Caspase: Top-Hat segmentation, Radius 10, Threshold 0.3 GCU, Edge Split On with sensitivity 0, Hole Fill 0, Adjust Size 1, and filter on a minimum Area of 20 $\mu m^2$. The analyzer is trained on a sufficient number of positive and negative control wells, as well as compound treated wells, verifying that the "confluence" layer detects both live and dead (condensed) cells. The "Caspase Area / Confluence Area" approximates the fraction of cells that are positive for the Caspase3/7 stain, calculated "Per Image".

Assay analysis was completed in Genedata Screener, using a predefined template. More particularly, the assay-specific settings for the experiment analysis were as follows: (a) Plate layout: Negative control wells contain no compound but DMSO, and were defined to be "Neutral Control", (b) Trace Channel: There should be one trace channel, name "Measured Channel", of type "Measured". This was the raw data from the IncuCyte; and (c) Layers: Three layers of the type "Aggregated: Time Series", with the names "Mean 6 h", "Mean 12 h" and "Mean 22 h". They contained the mean of the measured from values from 5.5 to 6.5 hours, from 11.5 to 12.5, and from 21.5 to 22.5 hours, respectively.

Normalization and Correction: Each of the three layers was normalized to Percent-of-Control, with Neutral Control as central reference, and Stimulator Control as Scale Reference. Or, if $\mu_{CR}$ was the mean of the Central Reference, and $\mu_{SC}$ was the mean of the Scale Reference, then the normalized value was calculated as:

$$\%\text{Activation} = 100\% \left( \frac{x_{raw} - \mu_{CR}}{\mu_{SC} - \mu_{CR}} \right)$$

Layer Compound Results: A standard fit model was used as below, with $S_{inf}$, $IC_{50}$ and h as free parameters, and $S_0$ fixed to be 0.

$$\%\text{Activation} = S_0 + \frac{S_{inf} - S_0}{1 + \left( \frac{IC_{50}}{\text{concentration}} \right)^h}$$

The Robust Z' Factor or "RZ' Factor" was calculated in Screener. After excluding outlier kinetic traces in control wells (see below), the RZ' value should be RZ ≥ 0.5 for MOLP8 cells tested at any FBS concentration, and for any of the time points (6 h, 12 h, 22 h).

The "Global SD" was calculated in Screener as the robust standard deviation of the positive or negative controls after normalization (whichever was greater). After excluding outlier kinetic traces in control wells (see below), the Global SD should be Global SD ≤ 10 for MOLP8 cells tested at any FBS concentration, and for any of the time points (6 h, 12 h, 22 h).

Representative compounds of Formula (I) of the present invention were tested according to the procedures described in Biological Example 2, with results as listed in the Table below. The AC50 values reported in the table below

are subject to error margins associated with the assay used and the equipment.

Table: Measured $AC_{50}$ for Representative Compounds of Formula (I)

| Compound | N | MOLP8 Caspase 3/7 $AC_{50}$ at 6 h (nM) | MOLP8 Caspase 3/7 $AC_{50}$ at 12 h (nM) | MOLP8 Caspase 3/7 $AC_{50}$ at 22 h (nM) |
|---|---|---|---|---|
| 1 | 2 | 178/129 | 141/182 | 145/170 |
| 2 | 2 | 4570/4790 | 4470/4680 | 4270/4470 |
| 3 | 3 | 347/178/251 | 363/263/195 | 257/363/162 |
| 4 | 1 | 1620 | 1590 | 1380 |
| N= number of independent runs | | | | |

Biological Example 3

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

The Caspase-Glo® 3/7 Assay is a luminescent assay that measures caspase-3 and -7 activities in purified enzyme preparations or cultures of adherent or suspension cells. The assay provides a proluminescent caspase-3/7 substrate, which contains the tetrapeptide sequence DEVD. This substrate is cleaved to release aminoluciferin, a substrate of luciferase used in the production of light. Addition of the single Caspase-Glo® 3/7 Reagent in an "add-mix-measure" format results in cell lysis, followed by caspase cleavage of the substrate and generation of a "glow-type" luminescent signal.

This assay uses the MOLP-8 human multiple myeloma cell line, which is sensitive to MCL-1 inhibition.

Materials:

- Perkin Elmer Envision
- Multidrop 384 and small volume dispensing cassettes
- Centrifuge
- Countess automated cell counter
- Countess counting chamber slides
- Assay plate: ProxiPlate-384 Plus, White 384-shallow well Microplate
- Sealing tape: Topseal A plus
- T175 culture flask

| **Product** | **Units** | **Storage** |
|---|---|---|
| RPMI1640 (no L-Glutamine, no phenol red) | 500 mL | 4 °C |
| Foetal Bovine Serum (FBS) (Heat inactivated) | 500 mL | 4 °C |
| L-Glutamine (200 mM) | 100 ml | -20 °C |
| Gentamicin (50 mg/mL) | 100 mL | 4 °C |
| Caspase 3/7 Detection kit | 100 mL<br>10 × 10 mL | -20 °C |

Cell culture media:

| MOLP8 | |
|---|---|
| | |
| RPMI-1640 medium | 500 mL |
| 20 % FBS (heat inactivated) | 120 mL |
| 2 mM L-Glutamine | 6.2 mL |
| 50 μg/mL Gentamicin | 620 μL |
| | |
| **Assay media** | |
| | |
| RPMI-1640 medium | 500 mL |
| 10 % FBS (Heat inactivated) | 57 mL |
| 2 mM L-Glutamine | 5.7 mL |
| 50 μg/mL Gentamicin | 570 μL |

Cell culture:

Cell cultures were maintained between 0.2 and 2.0 $\times 10^6$ cells/mL. The cells were harvested by collection in 50 mL conical tubes. The cells were then pelleted at 500 g for 5 mins before removing supernatant and resuspension in fresh pre-warmed culture medium. The cells were counted and diluted as needed.

Caspase-Glo reagent:

The assay reagent was prepared by transferring the buffer solution to the substrate vial and mixing. The solution may be stored for up to 1 week at 4 °C with negligible loss of signal.

Assay procedure:

Compounds were delivered in assay-ready plates (Proxiplate) and stored at -20°C.

Assays always include 1 reference compound plate containing reference compounds. The plates were spotted with 40 nL of compounds (0.5 % DMSO final in cells; serial dilution; 30 μM highest conc. 1/3 dilution, 10 doses, duplicates). The compounds were used at room temperature and 4 μL of pre-warmed media was added to all wells except column 2 and 23. The negative control was prepared by adding 1 % DMSO in media. The positive control was prepared by adding the appropriate positive control compound in final concentration of 60 μM in media. The plate was prepared by adding 4 μL negative control to column 23, 4 μL positive control to column 2 and 4 μL cell suspension to all wells in the plate. The plate with cells was then incubated at 37 °C for 2 hours. The assay signal reagent is the Caspase-Glo solution described above, and 8 μL was added to all wells. The plates were then sealed and measured after 30 minutes.

The activity of a test compound was calculated as percent change in apoptosis induction as follows:

LC = median of the Low Control values

= Central Reference in Screener

= DMSO

=0%

HC = Median of the High Control values

= Scale Reference in Screener
= 30 μM of positive control

= 100 % apoptosis induction

$$\%Effect\ (AC_{50}) = 100 - (sample-LC) / (HC-LC) * 100$$

$$\%Control = (sample / HC) * 100$$

$$\%Control\ min = (sample-LC) / (HC-LC) * 100$$

Table: Measured $AC_{50}$ for Representative Compounds of Formula (I). Averaged values are reported over all runs on all batches of a particular compound.

| Compound | MOLP8 Caspase-Glo $AC_{50}$ (μM) |
|---|---|
| 1 | 0.05 |
| 2 | 10.3 |
| 3 | |
| 4 | 0.92 |
| 5 | 14.5 |
| 6 | |
| 7 | 0.07 |
| 8 | 0.09 |
| 9 | 0.16 |
| 10 | 0.12 |
| 11 | >30 |
| 12 | 0.51 |
| 13 | 1.25 |
| 14 | 0.92 |
| 15 | 15.3 |
| 16 | 0.54 |
| 17 | 21.3 |

## Claims

1. A compound of Formula (I)

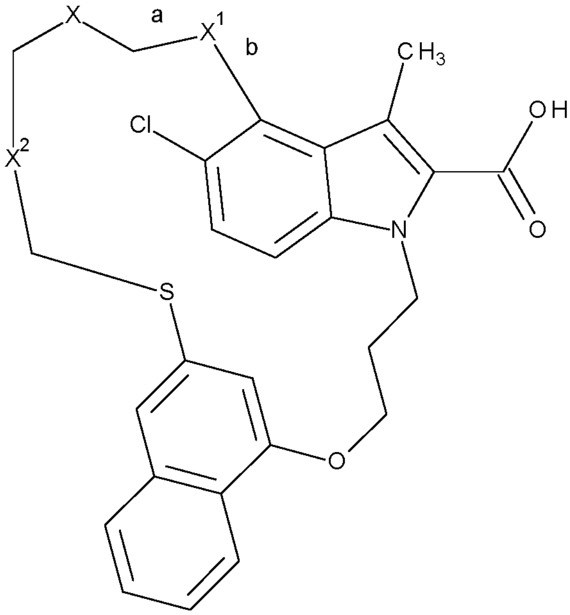

(I)

or a tautomer or a stereoisomeric form thereof, wherein
$X^1$ represents

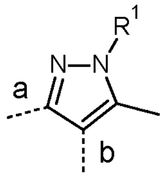

or

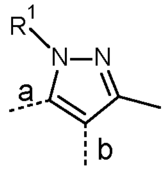

,

wherein 'a' and 'b' indicate how variable $X^1$ is attached to the remainder of the molecule;
$R^1$ represents hydrogen or $C_{1-6}$alkyl;
$X^2$ represents

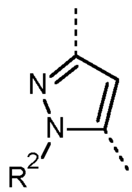

which can be attached to the remainder of the molecule in both directions;
$R^2$ represents hydrogen or $C_{1-6}$alkyl;
X represents -S- or -N($R^x$)-;
$R^x$ represents hydrogen, methyl, $C_{2-6}$alkyl, -C(=O)-$C_{1-6}$alkyl, -S(=O)$_2$-$C_{1-6}$alkyl, $C_{3-6}$cycloalkyl, -C(=O)-$C_{3-6}$cycloalkyl, or -S(=O)$_2$-$C_{3-6}$cycloalkyl; wherein $C_{2-6}$alkyl, -C(=O)-$C_{1-6}$alkyl, -S(=O)$_2$-$C_{1-6}$alkyl, $C_{3-6}$cycloalkyl, -C(=O)-$C_{3-6}$cycloalkyl, and -S(=O)$_2$-$C_{3-6}$cycloalkyl are optionally substituted with one, two or three substituents selected from the group consisting of halo, $C_{1-4}$alkyl and $C_{1-4}$alkyl substituted with one, two or three halo atoms;
or a pharmaceutically acceptable salt, or a solvate thereof.

**2.** The compound according to claim 1, wherein $X^1$ represents

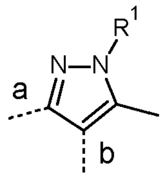

,

wherein 'a' and 'b' indicate how variable $X^1$ is attached to the remainder of the molecule;
$R^1$ represents $C_{1-6}$alkyl;
$R^2$ represents $C_{1-6}$alkyl;
$R^x$ represents hydrogen or methyl.

**3.** The compound according to claim 1 or 2, wherein X represent-N($R^x$)-.

**4.** The compound according to claim 1, wherein $X^1$ represents

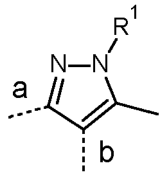

.

**5.** A pharmaceutical composition comprising a compound as claimed in any one of claims 1 to 4 and a pharmaceutically acceptable carrier or diluent.

**6.** A process for preparing a pharmaceutical composition as defined in claim 5 comprising mixing a pharmaceutically acceptable carrier with a therapeutically effective amount of a compound according to any one of claims 1 to 4.

**7.** A compound as claimed in any one of claims 1 to 4 or a pharmaceutical composition as claimed in claim 5 for use as a medicament.

**8.** A compound as claimed in any one of claims 1 to 4 or a pharmaceutical composition as claimed in claim 5 for use in the prevention or treatment of cancer.

**9.** The compound or a pharmaceutical composition for use according to claim 8, wherein cancer is selected from prostate, lung, pancreatic, breast, ovarian, cervical, melanoma, B-cell chronic lymphocytic leukemia (CLL), acute myeloid leukemia (AML), and acute lymphoblastic leukemia (ALL).

## Patentansprüche

**1.** Verbindung von Formel (I)

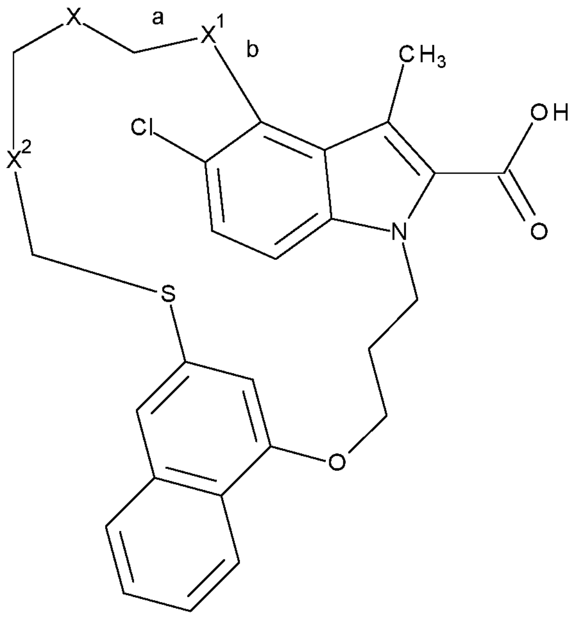

(I)

oder ein Tautomer oder eine stereoisomere Form davon, wobei
$X^1$

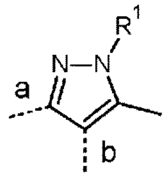 oder 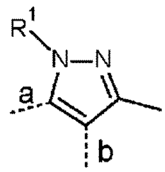

darstellt,
wobei "a" und "b" angeben, wie variabel $X^1$ an den Rest des Moleküls angelagert ist;
$R^1$ Wasserstoff oder $C_{1-6}$-Alkyl darstellt;
$X^2$

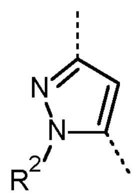

darstellt, das an den Rest des Moleküls in beiden Richtungen angelagert werden kann;
$R^2$ Wasserstoff oder $C_{1-6}$-Alkyl darstellt;
X -S- oder -N($R^x$)- darstellt;
$R^x$ Wasserstoff, Methyl, $C_{2-6}$-Alkyl, -C(=O)-$C_{1-6}$-Alkyl, -S(=O)$_2$-$C_{1-6}$-Alkyl, $C_{3-6}$-Cycloalkyl, -C(=O)-$C_{3-6}$-Cycloalkyl oder -S(=O)$_2$-$C_{3-6}$-Cycloalkyl darstellt; wobei $C_{2-6}$-Alkyl, -C(=O)-$C_{1-6}$-Alkyl, -S(=O)$_2$-$C_{1-6}$-Alkyl, $C_{3-6}$-Cycloalkyl, -C(=O)-$C_{3-6}$-Cycloalkyl und -S(=O)$_2$-$C_{3-6}$-Cycloalkyl optional mit einem, zwei oder drei Substituenten substituiert sind, die aus der Gruppe ausgewählt sind, bestehend aus Halogen, $C_{1-4}$-Alkyl und $C_{1-4}$-Alkyl, substituiert mit einem, zwei oder drei Halogenatomen;
oder ein pharmazeutisch verträgliches Salz oder ein Solvat davon.

**2.** Verbindung nach Anspruch 1, wobei

$X^1$

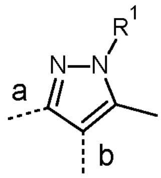

darstellt,
wobei "a" und "b" angeben, wie variabel $X^1$ an den Rest des Moleküls angelagert ist;
$R^1$ $C_{1-6}$-Alkyl darstellt;
$R^2$ $C_{1-6}$-Alkyl darstellt;
$R^x$ Wasserstoff oder Methyl darstellt.

**3.** Verbindung nach Anspruch 1 oder 2, wobei X -N($R^x$)- darstellt.

**4.** Verbindung nach Anspruch 1, wobei $X^1$

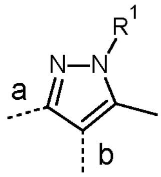

darstellt.

**5.** Pharmazeutische Zusammensetzung, umfassend eine Verbindung nach einem der Ansprüche 1 bis 4 und einen pharmazeutisch verträglichen Träger oder ein pharmazeutisch verträgliches Verdünnungsmittel.

**6.** Verfahren zum Herstellen einer pharmazeutischen Zusammensetzung wie in Anspruch 5 definiert, umfassend ein Mischen eines pharmazeutisch verträglichen Trägers mit einer therapeutisch wirksamen Menge einer Verbindung nach einem der Ansprüche 1 bis 4.

**7.** Verbindung nach einem der Ansprüche 1 bis 4 oder eine pharmazeutische Zusammensetzung nach Anspruch 5 zur Verwendung als ein Medikament.

**8.** Verbindung nach einem der Ansprüche 1 bis 4 oder eine pharmazeutische Zusammensetzung nach Anspruch 5 zur Verwendung bei der Vorbeugung oder Behandlung von Krebs.

**9.** Verbindung oder pharmazeutische Zusammensetzung zur Verwendung nach Anspruch 8, wobei Krebs aus Prostata, Lunge, Bauchspeicheldrüse, Brust, Eierstock, Gebärmutterhals, Melanom, chronischer lymphozytischer Leukämie (CLL) der B-Zellen, akuter myeloischer Leukämie (AML) und akuter lymphoblastischer Leukämie (ALL) ausgewählt ist.

## Revendications

**1.** Composé de formule (I)

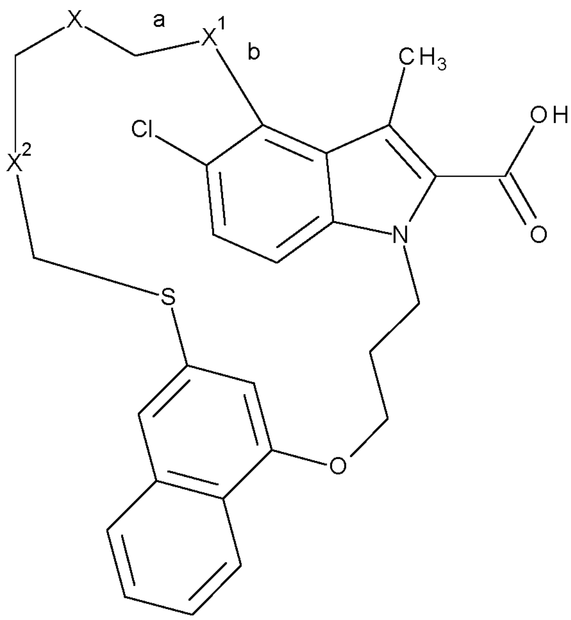

(I)

ou tautomère ou forme stéréoisomère de celui-ci, où
$X^1$ représente

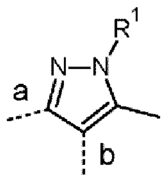 ou 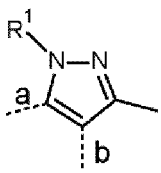 ,

où 'a' et 'b' indiquent comment une variable $X^1$ est fixée au reste de la molécule ;
$R^1$ représente hydrogène ou $C_{1-6}$alkyle ;
$X^2$ représente

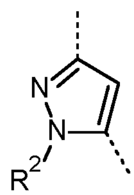

qui peut être fixé au reste de la molécule dans les deux sens ;
$R^2$ représente hydrogène ou $C_{1-6}$alkyle ;
X représente -S- ou $-N(R^x)-$ ;
$R^x$ représente hydrogène, méthyle, $C_{2-6}$alkyle, $-C(=O)-C_{1-6}$alkyle, $-S(=O)_2-C_{1-6}$alkyle, $C_{3-6}$cycloalkyle, $-C(=O)-C_{3-6}$cycloalkyle, ou $-S(=O)_2-C_{3-6}$cycloalkyle ; où $C_{2-6}$alkyle, $-C(=O)-C_{1-6}$alkyle, $-S(=O)_2-C_{1-6}$alkyle, $C_{3-6}$cycloalkyle, $-C(=O)-C_{3-6}$cycloalkyle, et $-S(=O)_2-C_{3-6}$cycloalkyle sont éventuellement substitués par un, deux ou trois substituants choisis dans le groupe constitué de halo, $C_{1-4}$alkyle et $C_{1-4}$alkyle substitué par un, deux ou trois atomes d'halo ;
ou un sel pharmaceutiquement acceptable, ou un solvate de celui-ci.

**2.** Composé selon la revendication 1, dans lequel

$X^1$ représente

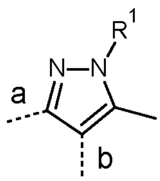

,

où 'a' et 'b' indiquent comment une variable $X^1$ est fixée au reste de la molécule ;
$R^1$ représente $C_{1-6}$alkyle ;
$R^2$ représente $C_{1-6}$alkyle ;
$R^x$ représente hydrogène ou méthyle.

**3.** Composé selon la revendication 1 ou 2, dans lequel X représente -N($R^x$)-.

**4.** Composé selon la revendication 1, dans lequel $X^1$ représente

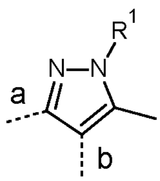

.

**5.** Composition pharmaceutique comprenant un composé selon l'une quelconque des revendications 1 à 4 et un support ou diluant pharmaceutiquement acceptable.

**6.** Procédé permettant de préparer une composition pharmaceutique telle que définie dans la revendication 5 comprenant le mélange d'un support pharmaceutiquement acceptable avec une quantité efficace sur le plan thérapeutique d'un composé selon l'une quelconque des revendications 1 à 4.

**7.** Composé selon l'une quelconque des revendications 1 à 4 ou composition pharmaceutique selon la revendication 5 pour utilisation en tant que médicament.

**8.** Composé selon l'une quelconque des revendications 1 à 4 ou composition pharmaceutique selon la revendication 5 pour utilisation dans la prévention ou le traitement d'un cancer.

**9.** Composé ou composition pharmaceutique pour utilisation selon la revendication 8, dans lequel le cancer est choisi parmi les cancers de prostate, poumon, pancréas, sein, ovaire, col de l'utérus, mélanome, leucémie lymphoïde chronique à lymphocytes B (LLC), leucémie myéloïde aiguë (LMA) et leucémie lymphoblastique aiguë (LLA).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018178226 A
- WO 2017182625 A
- WO 2018178227 A
- WO 2020063792 A
- CN 110845520
- WO 2020103864 A
- CN 20191114551


### Non-patent literature cited in the description


- **CHENG et al.** *eLife,* 2016, vol. 5, e17755
- **JULIAN et al.** *Cell Death and Differentiation,* 2017, vol. 24, 1380-1389
- **HANAHAN ; WEINBERG.** *Cell,* 2011, 1-44
- **BEROUKHIM et al.** *Nature,* 2010, vol. 463 (7283), 899-905
- **YECIES et al.** *Blood,* 2010, vol. 115 (16), 3304-3313
- **ROBERTS et al.** *NEJM,* 2016, vol. 374, 311-322
- **KOTSCHY et al.** *Nature,* 2016, vol. 538, 477-486
- **MERINO et al.** *Sci. Transl. Med,* 2017, (9
- **CHEN et al.** *JCI,* 2018, vol. 128 (1), 500-516
- **WANG et al.** *Genes and Dev,* 2013, vol. 27, 1351-1364
- **STEIMER et al.** *Blood,* 2009, (113), 2805-2815
- **ELIEL, E.L. ; WILEN, S. H.** Stereochemistry of Organic Compounds. John Wiley and Sons, Inc, 1994
- **CHARRON, CARLIE L. et al.** *Tetrahedron Lett.,* 2016, vol. 57 (37), 4119-4127
- **AUSTIN R. et al.** *Cancer Letters,* 2016
- **T. W. GREENE ; P. G. M. WUTS.** Protective Groups in Organic Synthesis. Wiley, 2007
- Part 8 : Pharmaceutical preparations and their Manufacture. **GENNARO et al.** Remington's Pharmaceutical Sciences. Mack Publishing Company, 1990